(19) Europäisches Patentamt
European Patent Office
Office européen des brevets

(11) EP 1 590 665 B1

(12) **EUROPEAN PATENT SPECIFICATION**

(45) Date of publication and mention of the grant of the patent:
**07.06.2006 Bulletin 2006/23**

(21) Application number: **04705527.2**

(22) Date of filing: **27.01.2004**

(51) Int Cl.:
***G01N 33/483*** *(2006.01)* ***G01N 33/487*** *(2006.01)*
***G01N 33/50*** *(2006.01)*

(86) International application number:
**PCT/JP2004/000723**

(87) International publication number:
**WO 2004/068137 (12.08.2004 Gazette 2004/33)**

(54) **METHODS AND DEVICE FOR IN VITRO DETECTION AND CHARACTERIZATION OF PSYCHOACTIVES USING ANALYSIS OF REPETITIVE ELECTRICAL ACTIVITY IN A NEURONAL SAMPLE**

VERFAHREN UND VORRICHTUNGEN FÜR DIE IN VITRO DETEKTION UND CHARAKTERISIERUNG PSYCHOAKTIVER SUBSTANZEN MITTELS ANALYSE REPETITIVER ELEKTROPHYSIOLOGISCHER AKTIVITÄTEN IN EINER NEURONALEN PROBE

METHODES ET DISPOSITIF POUR UNE DETECTION IN VITRO ET UNE CARACTERISATION DE SUBSTANCES PSYCHOACTIVES AU MOYEN D'UNE ANALYSE D'ACTIVITE ELECTRIQUE REPETITIVE DANS UN ECHANTILLON NEURONAL

(84) Designated Contracting States:
**CH DE FR GB LI**

(30) Priority: **27.01.2003 US 352629**

(43) Date of publication of application:
**02.11.2005 Bulletin 2005/44**

(73) Proprietors:
- **MATSUSHITA ELECTRIC INDUSTRIAL CO., LTD.**
  **Kadoma-shi, Osaka 571-8501 (JP)**
- **The Regents of the University of California**
  **Oakland,**
  **California 94607-5200 (US)**

(72) Inventors:
- **LYNCH, Gary**
  **Irvine, CA 92612 (US)**
- **SHIMONO, Ken**
  **Hirakata-shi,**
  **Osaka 573-1146 (JP)**
- **TAKETANI, Makoto**
  **Irvine, CA 92612 (US)**
- **SUGIHARA, Hirokazu**
  **Katano-shi, Osaka 5760054 (JP)**
- **COLGIN, Laura, L.**
  **Irvine, CA 92612 (US)**
- **JIA, Yousheng**
  **Irvine, CA 92612 (US)**

(74) Representative: **Vossius & Partner**
**Siebertstrasse 4**
**81675 München (DE)**

(56) References cited:
**WO-A-00/79273** **US-A- 5 772 983**

- **FISAHN A ET AL: "CHOLINERGIC INDUCTION OF NETWORK OSCILLATIONS AT 40 HZ IN THE HIPPOCAMPUS IN VITRO" NATURE, MACMILLAN JOURNALS LTD. LONDON, GB, vol. 394, 1998, pages 186-189, XP001002058 ISSN: 0028-0836**
- **MALOUF A T ET AL: "COMPARISON OF THE ACTIONS OF PHENCYCLIDINE AND SIGMA LIGANDS ON CAI HIPPOCAMPAL PYRAMIDAL NEURONS IN THE RAT" NEUROPHARMACOLOGY, PERGAMON PRESS, OXFORD, GB, vol. 27, no. 11, 1988, pages 1161-1170, XP002916483 ISSN: 0028-3908**
- **OKA HIROAKI ET AL: "A new planar multielectrode array for extracellular recording: Application to hippocampal acute slice" JOURNAL OF NEUROSCIENCE METHODS, vol. 93, no. 1, 30 October 1999 (1999-10-30), pages 61-67, XP002289548 ISSN: 0165-0270**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **DICKSON CLAYTON T ET AL: "Muscarinic induction of synchronous population activity in the entorhinal cortex" JOURNAL OF NEUROSCIENCE, vol. 17, no. 17, 1997, pages 6729-6744, XP002289549 ISSN: 0270-6474**

## Description

FIELD OF THE INVENTION

This invention relates to methods for the detection of psychoactive compounds in an *in vitro* neuronal tissue sample, preferably by detecting oscillations of extracellular voltage before and after the introduction of a candidate sample onto an *in vitro* neuronal tissue sample and to devices for practicing those processes. Analysis of the extracellular voltage parameters leads to indication of the presence of psychoactive material in the candidate sample and information as to its pharmacological activity and composition. Further, the invention includes a process of initiating and maintaining the presence of repetitive neuronal activity within the *in vitro* sample. Additionally, this invention includes a method for the stimulation of or initiation of repetitive neuronal activity, e.g., EEG, in an *in vitro* neuronal tissue sample by introducing a stimulating composition comprising compounds that facilitate or mimic the actions of acetylcholine, serotonin, or catecholamines, such as carbachol.

BACKGROUND OF THE INVENTION

Rhythmic activity in the hippocampus of small mammals is described as falling into three frequency bands: 4-10 Hz (theta), 10-30 Hz (beta), and above 30 Hz (gamma) (Traub et al., 1998; 1999 for a recent discussion). Theta is by far the best studied of these and is related to, among other things, locomotor activity (Vanderwolf, 1969) and memory encoding (Landfield et al., 1972; Vertes and Kocsis, 1997). In accord with the latter idea is the close relationship between theta and long-term potentiation (Larson and Lynch, 1986; Larson et al., 1986; 1993), a probable substrate of certain forms of memory. The functional correlates of the higher frequency rhythms have recently become the subjects of considerable interest. Gamma activity was first analyzed in the olfactory system (Freeman, 1975 for an early review) with the conclusion that it allows coherence to develop between bulb, piriform cortex, and entorhinal cortex prior to the arrival of an odor (Kay and Freeman, 1998). Activity falling in the gamma range also appears in the visual cortex during cue presentation (Gray and Singer, 1989) where it is proposed to transiently synchronize cells with disparate receptive fields. Synchronization, according to this hypothesis, allows multiple features of a cue to be assembled into a coherent representation (Singer, 1998 for a review). Beta rhythms have not typically been discriminated from the gamma wave in discussions of high frequency hippocampal activity although they have been selectively induced in hippocampal slices (Boddeke et al, 1997). In any event, the growing evidence that high frequency synchronization is essential to coherent operations in the cortical telencephalon has emphasized the importance of identifying the pathways and neurotransmitter systems responsible for the beta and gamma oscillations.

Ascending cholinergic projections promote endogenous oscillations including those in the beta and gamma ranges. Although early work (Stumpf, 1965) found cholinergic blockers or septal lesions to be without obvious effect, subsequent studies showed that fast waves in freely moving rats are enhanced by the cholinesterase inhibitor physostigmine and substantially reduced by antagonists (Leung, 1985). Cholinergic stimulation of hippocampal or entorhinal slices is usually described as inducing seconds-long episodes of theta-like activity (Konopacki et al., 1987; MacVicar and Tse, 1989; Dickson and Alonso, 1997; Williams and Kauer, 1997) but recent experiments show that it can also trigger higher frequency rhythms (20-40 Hz) in cortical and hippocampal slices (Boddeke et al., 1997; Fisahn et al., 1998). Cholinergic septohippocampal fibers innervate discrete regions of the hippocampal system (Lewis and Shute, 1967; Mosko et al., 1973; Frotscher and Leranth, 1985; Matthews et al., 1987) where they contact subpopulations of interneurons and select dendritic zones of principal cells (Mosko et al., 1973; Lynch et al., 1978; Matthews et al., 1987). Stimulation of muscarinic receptors depolarizes pyramidal cells, depresses release from some interneurons, and increases the excitability of others (Pitler and Alger, 1992; Behrends and Bruggencate, 1993). The combination of *in vivo* and *in vitro* results suggests that acetylcholine plays an important role in generating high frequency activity in the cortical telencephalon.

How cholinergically driven high frequency rhythms affect cortical operations depends on whether they are regionally specialized and how they are produced. Carbachol-elicited oscillations have been reported to originate in restricted loci in entorhinal cortex (Dickson and Alonso, 1997) and hippocampus (Fisahn et al., 1998). As for regional specificity, the answer requires systematic mapping over broad expanses of the entorhinohippocampal system.

None of the documents discussed above utilize analysis of the high frequency oscillations found in the *in vitro* samples for indication of the presence of, characterization of the pharmacological activity of, or the composition of the psychoactive material. Additionally, none of the documents apply such an analysis under free-running oscillatory conditions, i.e., oscillations occurring without the continued presence of an oscillatory-triggering agent.

SUMMARY OF THE INVENTION

The inventive methods here include methods for detection of pharmacological activity, characterization of the pharmacological activity, and for determining the composition of psychoactive compounds in an *in vitro* neuronal tissue sample. An allied inventive method is a method for the stimulation of the initiation of, and/or stimulation of, repetitive neuronal activity variously by the independent steps of contacting the neuronal tissue with an agent that stimulates those oscillations of extracellular voltage, and/or utilizing a co-implanted bit of neuronal tissue, and/or stimulating the neuronal sample using an electrical stimulation pattern. The method includes removing such stimulating compositions, materials, tissues, or electrical stimulation patterns, but conserving the oscillatory behaviors induced by them. Such techniques may be followed by examination of the effect of psychoactive compounds on the induced oscillatory behavior.

In one variation, the method for detection of psychoactive compounds in an *in vitro* neuronal tissue sample includes the steps of inducing and then detecting the presence of oscillations in the tissue sample, removing the inducing agent while conserving the triggered oscillatory activity, and providing a baseline value of those oscillations. After that detection, the *in vitro* sample is brought into contact with a candidate sample of a psychoactive compound or compounds. Coincidentally with (or subsequent to) introduction of the candidate sample, the oscillations, e.g., EEG waves, are then measured. The two sets of oscillation data are then rendered to produce two so-called "calculated values." Comparing the two calculated values will then allow detection of pharmacological activity, characterization of the pharmacological activity, and (if desired) determination of the composition of psychoactive compounds in the sample should one or more be present.

The various oscillations are typically those found in extracellular voltage. For instance, they may be a theta, beta, or gamma EEG waves.

It is desirable to use a standard electrophysiological system. It is also desirable to use a multi-electrode dish ("MED") so that a number of different active or less active sites on the neuronal sample may be simultaneously or sequentially sampled. Use of the MED permits measurement and calculation of spatial relationships; both measured and calculated, amongst the values of the neural oscillations. The multi-electrode nature of the MED also enables the determination and characterization of region-specific effects within the given *in vitro* neuronal sample.

Appropriate mathematical analysis of the oscillations of extracellular voltage may include a Fast Fourier Transform (FFT) of oscillations measured at a single spatial point to enhance differences in amplitude and frequency of the before-and-after single-site measurements.

Similarly, the sequence of oscillations of extra-cellular voltage obtained in an array as a function of time may be subjected to Current Source Density (CSD) analysis to produce and depict current flow patterns within the *in vitro* neuronal tissue sample.

A further portion of the invention includes the use of chemical or anatomical compositions and electrical stimulation patterns that tend to stimulate or induce repetitive neuronal activity in *in vitro* neuronal tissue samples. These compositions may be compounds that mimic or facilitate the actions of acetylcholine, serotonin, or catecholamines. Such compositions may or may not be removed from the tissue samples before, during, or after electrophysiological analysis of the samples and/or test compounds using our described procedures. They may be co-deposited neuronal tissue. They may be cholinomimetic compounds. A highly desirable chemical compound is carbachol. Electrical stimulations may also be used.

In another variation, the process for the detection and characterization of psychoactive compounds in a candidate sample composition in a neuronal tissue sample comprises the steps of :

a) inducing a set of baseline oscillations by contacting the neuronal tissue sample with an inducing agent and maintaining the set of baseline oscillations after washout of the inducing agent.
b) measuring a combination of parameters from the set of baseline oscillations and from a set of resulting oscillations generated after contact of the neuronal tissue sample with the candidate sample composition;
c) comparing said combination of parameters from the set of baseline oscillations and resulting oscillations; and
d) characterizing said candidate sample composition based upon the differences between said combination of parameters from the set of baseline oscillations and resulting oscillations.

The combination of parameters typically includes two parameters, but the combination may not need be limited to two, and may include any number of parameters for comparison necessary for the detection and characterization of a psychoactive compound. Furthermore, the inducing agent for producing the set of baseline oscillations is removed. The inducing agent may be a chemical compound, co-deposited neuronal tissue, or an electrical stimulation.

Parameters useful for comparing to detect and to characterize a psychoactive compound in the candidate sample composition include the power and frequency of the rhythmic oscillations, however, any combination of parameters may be used. In a preferred variation, the method used to detect and characterize the psychoactive compound in the candidate sample composition generally involves forming a 2-dimensional array of frequency and power to characterize the psychoactive as belonging to a particular psychoactive compound class. In another preferred variation, the 2-dimensional array of frequency and power also generally characterizes psychoactive compounds by distinguishing them from other members within their class.

Generally, the procedures of the invention make use of electrophysiological techniques for methods wherein: 1) oscillations are induced in brain tissue with an inducing agent, and 2) the oscillations continue after the inducing agent has been removed. The methods include the application of agonists and/or enzyme inhibitors and tissue preparation techniques yielding spontaneously occurring oscillations remaining without continued application of the inducing compound. These "agent-free" oscillations may provide an improved assay for detecting and characterizing compounds, and a drug discovery and development method where the influences of inducing agents are absent from characterization and/or detection steps performed during such agent-free oscillations. Such a method has many advantages including, but not limited to, the fact that the inducing compound tends not to obscure test article effects - as may prior art methods that require the continual infusion of such inducing compounds. The methods of the invention may find particular use in elucidating the mechanism of action of various drugs, e.g., cholinergic therapeutics.

The invention includes various methods of detection, characterization of the pharmacological activity, and determination of various psychoactive test compound(s) or psychoactive test composition(s). A number of methods for identifying those test compounds are within the methods of the invention.

BRIEF DESCRIPTION OF THE DRAWINGS

The accompanying drawings illustrate and explain the principles of the invention. They are not intended to limit the scope of the invention in any way.

Figure 1 depicts the induction of fast oscillations in field CA3 of hippocampal slices and shows examples of the power spectra of control and extracellular oscillations induced by CCh. Calibration: 50 ms, 100 μV.

Figure 2 shows the lasting increases and spikes and bursts in CA3 pyramidal cell activity levels after transient CCh application. Calibration: 10 ms, 100 μV.

Figure 3 depicts the effects of pharmacological manipulations on long lasting rhythms. For Figure 3A, calibration: 5 ms, 400 μV. For Figure 3C, calibration: 5 ms, 500 μV.

Figure 4 demonstrates the effects of atropine and 5-HT on spike and burst frequency. For Figure 4A, calibration: 20 ms, 100 μV. For Figure 4D, calibration: 20 ms, 100 μV.

Figure 5 shows the effects of selective muscarinic antagonists on cholinergic plasticity. Calibration: 5 ms, 500 μV.

Figure 6 illustrates beta rhythmic activity induced in hippocampal slices by various acetylcholinesterase inhibitors.

Figure 7 depicts the phenomenon of "aliasing" in depicting measured images at low resolution.

Figure 8 depicts the effects of anti-aliasing.

Figure 9A and 9B show respectively the placement of a hippocampal slice on an 8x8 detector array and an elicited post-synaptic response at one of the electrodes.

Figure 10 shows a continuous two-dimensional current source density analysis of evoked response computed from the placement of the slice in Figs. 9A and 9B.

Figures 11A and 11B show the alignment of computed physiological phenomena with a known anatomical structure.

Figures 12A-12E show a micrograph of a hippocampal slice and the distribution of carbachol-induced beta waves within the hippocampus.

Figures 13A-13C show carbachol-induced beta waves in a hippocampus measured with a dense microelectrode array.

Figure 14 shows a current source density analysis of carbachol-induced activity for the slice shown in Fig. 13A.

Figures 15A and 15B show the relationship of recurring carbachol-induced oscillations in apical and basal dendrites.

Figure 16 shows a computed evolution of current source density over a specific time.

Figure 17 shows two distinct carbachol-induced rhythms in hippocampus and cortex.

Figure 18 shows a two-dimensional current source density analysis of beta-like activity elicited by orthodromic activation of field CA3.

Figure 19 shows the effect of benzodiazepines on carbachol-induced beta waves.

Figure 20A-C show the effect of diazepam on carbachol-induced beta waves.

Figures 21A-C show the effect of flurazepam on carbachol-induced beta waves.

Figures 22A-C show the effect of ampakine (CX614) on carbachol-induced beta waves.

Figures 23A-C show the effect of ampakine (CX691) on carbachol-induced beta waves.

Figure 24 shows the effect of CX546 on CCh-induced rhythmic activity in hippocampal slices. Inter-electrode spacing is 300 μm.

Figure 25 depicts sample changes in frequency and power of rhythmic activity plotted over time with CX546.

Figure 26 shows the percent changes in frequency and power between benzodiazepines, cyclothiazide, and ampakines.

Figure 27 is a two-dimensional plot of changes in frequency and power between benzodiazepines, cyclothiazide, and ampakines.

Figure 28 demonstrates that incubation of tissue with 5 mM CCh induces beta oscillations as well as lower frequency bursting activity (0.1-0.2 Hz).

Figure 29 shows that 5-HT and fluoxetine decrease the frequency of CCh-induced network bursting activity.

Figure 30 demonstrates that 5-HT decreases the power of CCh-induced β oscillations.

Figure 31 shows the relative differences in spontaneous rhythmic activities caused by the addition of physostigmine, a cholinesterase inhibitor, to septohippocampal co-cultures grown directly on the MED probe.

Figure 32 shows the effect of a cholinesterase inhibitor (physostigmine) on the number of spikes in septo-hippocampus co-culture.

Figure 33 is a two-dimensional plot of changes in frequency and power between the control and SSRI (fluoxetine) treated tissue in raphe-hippocampal co-culture.

Figures 34A-C show the effect of bicuculline on carbachol-induced beta waves.

Figures 35A-C show the effect of picrotoxin on carbachol-induced beta waves.

Figures 36A-C show the effect of CNQX on carbachol-induced beta waves.

Figures 37A-C show the effect of DNQX on carbachol-induced beta waves.

Figures 38A-C show the effect of NBQX on carbachol-induced beta waves.

Figure 39 broadly depicts the predictive results of comparing the oscillatory rhythms before and after the addition of certain classes of psychoactives to neural tissue according to the methods of the invention.

## DETAILED DESCRIPTION OF THE INVENTION

This invention includes a process for the detection and/or characterization of psychoactive activity or compositions preferably using rhythmic oscillations of extracellular voltage (or potential) in *in vitro* neuronal tissue samples. Additionally, the inventive process comprises the stimulation or inducement of those oscillations using certain agents, e.g., certain chemical compositions or electrical stimulation techniques that are discussed in more detail below.

The measurement of extracellular potentials in *in vitro* neuronal tissue either with standard methods or over the spatial array of a neuronal sample is known. See, for instance, the various descriptions of such devices found in Dunwiddie et al. *J Physiol* 276:353-367 (1978), and the like, and U.S. Pat. Nos. 5,563,067 and 5,810,725, each to Sugihara et al..

### Definitions

For the purposes of this description, we use the following terms as defined in this section, unless the context of the word indicates a different meaning.

As used herein, the term "sink" refers to current being absorbed from the extracellular medium into a neuronal element.

As used herein, the term "source" refers to current being introduced into the extracellular medium from within a neuronal element.

As used herein, the term "hippocampus" refers to a region of the telencephalon which is located behind the temporal lobes and has been implicated in memory formation and retrieval in humans and other animals.

As used herein, the term "hippocampal slice" refers to a physical slice of hippocampal tissue generally approximately 100-500 micrometers in thickness that can be used on the electrophysiological recording devices described herein.

As used herein, the term "CA1", "CA2", "CA3", and "CA4" refer to one of four regions of hippocampus.

As used herein, the term "dendrites" refers to the highly branched structure emanating from the cell body of the nerve cells.

### Measurement Apparatus

The cell potential measuring apparatus preferably used with this inventive process includes "standard" stimulating and recording techniques and variations that would be appreciated by one skilled in the art (see Dunwiddie T and Lynch G (1978) J Physiol 276: 353-367 for one such apparatus). Also preferable is an apparatus which employs plural microelectrodes on an insulating substrate, a conductive pattern for connecting the microelectrodes to some region out of the microelectrode area, electric contacts connected to the end of the conductive pattern, an insulating film covering the surface of the conductive pattern, and a wall enclosing the region including the microelectrodes on the surface of the insulating film. The reference electrodes may have comparatively lower impedance than the impedance of the measuring microelectrodes. They may be placed at plural positions in the region enclosed by the wall and often at a specific distance from the microelectrodes. The electrical contacts are further usually connected between the conductive pattern for wiring of each reference electrode and the end of the conductive pattern. The surface of the conductive pattern for wiring of the reference electrodes is typically covered with an insulating film.

Typically, for the multi-electrode methods, the microelectrodes are situated in a matrix arrangement in a rectangle having sides of, e.g., 0.8 to 2.2 mm (in the case of 300 μm microelectrode pitch) or 0.8 to 3.3 mm (in the case of 450 μm microelectrode pitch). Four reference electrodes are situated at four corners of a rectangle of 5 to 15 mm on one side. More preferably, 64 microelectrodes are situated in eight rows and eight columns at central pitches of about 100 to 450 μm, preferably 100 to 300 μm.

Preferably, the microelectrodes and the reference electrodes are formed of layers of nickel plating, gold plating, and platinum black on an indium-tin oxide (ITO) film.

The insulating substrate (for example, a glass substrate) may be nearly square. Plural electric contacts may be connected to the end of the conductive pattern and preferably are placed on the four sides of the insulating substrate. As a result, layout of wiring patterns of multiple microelectrodes and reference electrodes is easy. Because the pitches of electric contacts may be made to be relatively large, electric connection through the electric contacts with external units is also easy.

The microelectrode region is usually very small. When observing the sample through a microscope, it is hard to distinguish position and both vertical and lateral directions. It is desirable to place indexing micro-marks near the microelectrode region to allow visual recognition through the microscope variously of direction, axes, and position.

It is even more preferable to perform the following sequence of events to determine electrode positions versus the anatomical correlates of the *in vitro* neuronal samples. Placing a control *in vitro* neuronal sample on the array in order that the array can cover the important area of the sample, taking a picture of the control sample on the array, recording the response from the control sample, placing a test sample on the array in the same relative position as the control sample as accurately as possible, taking a picture of the test sample on the array, recording the response from the test sample, and comparing the control picture and the test picture, and comparing the control response and the test response.

An alternative method, which would be appreciated by one skilled in the art, is to use an object recognition algorithm where the object is the gross anatomical structure of the *in vitro* neuronal samples, and comparing object recognition algorithm data, and comparing the control response and the test response.

The most preferred cell potential measuring apparatus is made up of a cell placement device having cell potential measuring electrodes, contact sites for contacting with an electric contact, and an electrode holder for fixing the insulating substrate by sandwiching from above and beneath. The cell potential measuring electrodes may be connected electrically to the cell placement assembly device to allow processing of the voltage or potential signals generated by the sample and measured between each such microelectrode and the reference electrodes. The cell potential measuring assembly may include a region enclosed by a wall for cultivating sample neuronal cells or tissues. It also preferably includes an optical device for magnifying and observing optically the cells or tissues cultivated in the region enclosed by the wall. This cell potential measuring apparatus preferably further comprises an image memory device for storing the magnified image obtained by the optical device.

Typically a personal computer having installed measurement software is included to accept the measured cell potentials. The computer and cell placement device are typically connected through an I/O board for measurement. The I/O board includes an A/D converter and a D/A converter. The A/D converter is usually for measuring and converting the resulting potentials; the D/A converter is for stimulus signals to the sample, when needed.

The measurement software installed in the computer may include software for setting conditions for giving a stimulus signal, forming the stimulus signal, and for recording the obtained detection signal. By using such measurement software, the computer may comprise means for giving a stimulus signal to the cells and means for processing the signal detected from the cells. The computer may also control any optical observation devices (SIT camera and image memory device) and the cell culture system.

Desirably, the extracellular potential detected from the cells may be displayed in real time. In addition, the recorded spontaneous activity, potential, or induced potential desirably is displayed by overlaying on the microscope image of the cell. It is an alternative that the software also include image processing, e.g., feature recognition, edge detection, or edge enhancement, algorithmic capabilities. When measuring the potential, the entire recorded waveform is usually displayed visually correlated to the position of the waveform in the neuronal sample.

Concerning data analysis or processing, Fast Fourier Transform (FFT) analysis, coherence analysis, and correlation analysis are also desirable. In the variation discussed below, Current Source Density Analysis (CSD) is also highly desirable. Other useable functions may include single spike separation function using waveform discrimination, temporal profile display function, and topography display function. These analysis results may be displayed by overlaying on the displayed images of the neuronal sample stored in the image memory device.

When a stimulus signal is issued from the computer, this stimulus signal is sent to the cell placement device through a D/A converter and an isolator. The cell placement device includes a cell potential measuring electrode that may be formed, e.g., of 64 microelectrodes on a glass substrate in a matrix form and having an enclosing wall for maintaining the neuronal sample (e.g., segments of cells or tissues) in contact with the microelectrodes and their culture fluid. Preferably, the stimulus signal sent to the cell placement device is applied to arbitrary electrodes out of the 64 microelectrodes and then to the sample or samples.

The induced, evoked, or spontaneous potential occurring between each microelectrode and reference potential (which is at the potential of the culture fluid) is passed through a 64-channel high sensitivity amplifier and an A/D converter into the computer. The amplification factor of the amplifier may be, e.g., about 80-100 dB, for example, in a frequency band of about 0.1 to 10 kHz, or to 20Hz. However, when measuring the potential induced by a stimulus signal, by using a low-cut filter, the frequency band is preferably 100Hz to 40kHz or more.

The desired apparatus may include a cell culture system having a temperature controller, a culture fluid circulation device, and a feeder for supplying, e.g., a mixed gas of air and carbon dioxide. The cell culture system may be made up of a commercial microincubator, a temperature controller, and $CO_2$ cylinder. The microincubator can be used to control in a temperature range of 0 to 50°C by means of a Peltier element and is applicable to the liquid feed rate of 3.0 ml/min or less and gas flow rate of 1.0 liter/min or less. Or, alternatively, a microincubator incorporating a temperature controller may be used.

Data Measurement and Analysis

In general, the procedures described herein utilize simultaneous measurement and preferably recording of extracellular voltage or potential values. Such procedures can occur at single sites within the neuronal tissue or can occur both spatially and temporally at multiple measurement sites. It is further preferable to observe the frequency and amplitude of the signals at each of the measurement sites in the spatial array. It is even more preferable that the placement of the neuronal sample and the inherent physical boundaries in the sample be observable either using optical devices or electronic sensing devices and that those margins be correlated to the position of the sensors, all within the chosen computer.

For multi-electrode methods, the neuronal sample is placed upon said *in vitro* cell potential measuring electrode array and procedures that would be known to one skilled in the art are used for maintaining its viability during the testing. The neuronal sample may be cultured, if desired. Typical procedures are discussed below with respect to the Examples. Each of the microelectrodes is monitored, both as a function of time and as a function of frequency, for rhythmic oscillations of extracellular voltages or potentials. This produces an array of frequency and amplitude signals as a function of time. It is preferable to measure the oscillations from a region of near DC (e.g., at 2 Hz) to a region above 35 Hz. This permits measurement retention of the typical three frequency bands found in neuronal rhythmic activity: 4 to 20 Hz (theta EEG), 15 to 25 Hz (beta EEG), and above 30 Hz (gamma EEG). The higher frequency band of 10 to 50 Hz, is significantly instructive.

*Analysis*

One procedure providing significant information as to the presence of or characterization of psychoactive compounds or psychoactive activity is the use of Fast Fourier Transforms (FFT). FFT are used in a variety of disparate areas and are commonly used in a device known as a spectrum analyzer. The application of FFT to specific measurement in the measurement array and comparing that result to a specific measurement at that same location prior to the introduction of the candidate composition will be instructive as to the presence, characterization, or pharmacological activity of a psychoactive compound. Specifically, if the candidate is psychoactive in the region of the neuronal sample that is analyzed, a comparison of the so-analyzed signals may show a shift in peak frequencies, amplitudes, or a combination of the two.

Another type of analysis, which is also instructive, is Current Source Density (CSD) analysis, further described in Examples 4 and 5. A discussion of this analytical procedure is also found, e.g., in Nicholson et al., "Theory of Current Source Density Analysis and Determination of Conductivity Tensor for Anuran Cerebellum," Journal of Neurophysiology, 1975, March, 38(2):356-68. This analytical procedure is used to convert the potentials or voltages measured by the devices described above, and convert them into a similar configured array of current flows and, more importantly, current magnitudes. By correlating the magnitude and direction of the currents as a function of time, current "sinks" and "sources" may be observed. The locations of such "sinks" and "sources" are instructive in determining the presence, characterization, or pharmacological activity of psychoactive drugs added to the *in vitro* neuronal sample.

*Stimulating Oscillations*

We have found it desirable in performing certain procedures to stimulate (induce) oscillations of extracellular voltage or potential variously by pharmacological/chemical, physiological, or anatomical methods. Pharmacological compositions, e.g., one or more compounds that facilitate or mimic the actions of acetylcholine, serotonin, or catecholamines in neuronal tissue may be used, however, other similar stimulations are also acceptable. The chemical stimulating compositions may include one or more cholinomimetic compounds such as carbachol (CCh, carbyl choline chloride). We have found that removing the stimulating compositions after the oscillations have been initiated, while conserving the induced oscillatory behavior, so that, e.g., the results from testing candidate psychoactive compositions are not confounded is desirable.

As shown in Figures 1 and 2A-B, and further described in Example 4, the addition of carbachol, a cholinergic agonist, to neuronal tissue results in rhythmic activity that persists after carbachol removal (washout). Carbachol was infused for a 20-minute period (indicated by black bar) following at least 10 minutes of baseline recording.

In addition to compositions containing cholinergic agonists, pharmacological/chemical compositions that may be used for stimulating oscillations in neuronal tissue samples include, but are not limited to, cholinesterase inhibitors (AchEI) and sympathetic agonists.

Examples of cholinergic agonists include acetylcholine, arechol, carbachol, methacholine, bethanechol, muscarine, and pilocarpine.

Examples of cholinesterase inhibitors include ambenonium, demecarium, diidopropyl-fluorophosphate, echothiophate, edrophonium, huperzine and huperzine analogs, neostigmine, physotigmine, and pyridostigmine.

Examples of sympathetic agonists include dopamine and norepinephrine.

In general, conservation of oscillatory activity is derived from modulating cholinergic interactions. Figures 3 and 6, and the description in Example 5 support this notion. For instance, in Figures 3B and 6A-6E, it is shown that cholinesterase inhibitors such as physostigmine and huperzine analogs, induce rhythmic activity in neuronal tissue similar to that seen with carbachol. Furthermore, CCh-induced activity is blocked by atropine (Figure 3C), a cholinergic antagonist, but not APV (Figure 3A), a NMDA antagonist. Specifically, the rhythmic activity may be modulated by muscarinic interactions since atropine is a cholinergic muscarinic antagonist. More specifically, the rhythmic activity may be modulated by interactions with the M1 muscarinic receptor. As shown in Figures 5A-5B, and further described in Example 6, the M1 selective muscarinic antagonist, pirenzepine (PRZ), has a blocking effect on CCh-induced oscillations, whereas the M2 selective muscarinic antagonist, methoctramine (MTC), has minimal to no effect.

Once the inducing or stimulating composition has been added to the *in vitro* neuronal tissue sample, a set of baseline values of the oscillations is then taken.

*Candidate Sample Compositions*

A candidate sample composition that may or may not contain a psychoactive compound is contacted with the neuronal tissue sample, preferably after a set of baseline oscillations (array of extracellular voltages or potentials), e.g., stimulated by carbachol, have been measured. We have found that a comparison of a combination of parameters measured from these oscillatory extracellular voltages, which typically involves a comparison of their frequency and power, before and after the introduction of a psychoactive compound(s), provides information on the presence of and/or characterization of psychoactive compositions. Although the combination for comparison typically includes two parameters, any number of combinations may be used.

In one variation, the oscillatory behavior of psychoactive compositions is characterized by plotting the change in power of the oscillations as a function of the change in frequency of the oscillations. Figures 27 and 39 are illustrations of such a two-dimensional plot, which depict, in a qualitative fashion, the relative differences in evoked potentials caused by the addition of various psychoactives to neuronal tissue. Observation of, or quantification of these relative differences allows detection of, and characterization of psychoactive compounds by identifying the various general psychoactive functional groupings or compound classes (e.g., benzodiazepines vs. ampakines) they may belong to, as well as distinguishing between specific members of those groups (e.g., diazepam vs. triazolam). For example, as shown in Figure 39 and described further in Examples 13-16, in comparison to the control (carbachol-induced oscillations), benzodiazepines effected an increase in power and no significant change in frequency of the oscillations; ampakines effected an increase in frequency and a decrease in power of oscillations; GABA antagonists effected a decrease in power and frequency of oscillations; and AMPA antagonists demonstrated a decrease in power but no significant change in frequency of the oscillations. As further described in Example 13, comparing the change in frequency and/or power of oscillations is also useful in discriminating psychoactive compounds within a drug class.

Baseline oscillations may also be stimulated or induced by co-deposited neuronal tissue. As further described in Example 15, in comparison to the control (oscillations induced by a septal-hippocampal co-culture for the acetylcholinesterase inhibitor (AchEI) and by a raphe-hippocampal co-culture for the SSRI), the AchEI increased the power without significantly affecting the frequency of the oscillations (Figure 31), and the SSRI decreased the power and slightly decreased the frequency of the oscillations (Figure 33).

Psychoactives that may be detected and characterized by the methods of this invention include, but are not limited to the following psychoactive compound classes:

1. GABA antagonists such as bicuculline, β-Hydrastine, picrotoxin, and SR 95531 (Gabazine);

2. AMPA antagonists such as CNQX, DNQX, GYKI 52466HCl, Joro spider toxin, 1-Naphthyl-acetyl spermine, NS257, and NBQX;

3. Benzodiazepines (generally, any of a group of chemically similar psychotropic drugs with potent hypnotic and sedative action; used predominantly as antianxiety and sleep-inducing drugs) such as alprazolam, clorazepate, temazepam, flurazepam, cholrdiazepoxide, diazepam, oxazepam, medazepam, lorazepam, flutoprazepam, fludiazepam, alprazolam, oxazolam, clotiazepam, etizolam, flurazepam, hloxazolam, estazolam, nitrazepam, nimetazepam, flunitrazepam, trizolam, rimazafone, flumazenil, bromazepam, and the like;

4. AMPA receptor modulators such as ampakines (benzoylpiperidine drugs (BDP)), e.g., CX516 (BDP-12), CX554 (BDP-20), CX614, CX691; and piracetam-like nootropics such as aniracetam, nefiracetam, and the like;

5. Antipsychotic drugs, generally such as phenothiazine derivatives, thioxanthene derivatives, butyrophenone derivatives (dopamine receptor antagonists), iminodibenzyl derivatives, dibenzotthiazepine derivatives, benzamide derivatives, theipine derivatives, benzisoxazole derivatives (serotonin and dopamine antagonists), and specifically such as: carbamazepine, chlorpromazine, chlorprothixene, clozapine, divalproex sodium, fluphenazine, haloperidol, lithium carbonate, lithium carbonate, lithium citrate, loxapine, mesoridazine, molindone, olanzapine, perphenazine, pimozide, quetiapine, risperidone, thioridazine, thiothixene, trifluoperazine, and triflupromazine;

6. Antidepressant drugs, generically such as tricyclic and tetracyclic antidepressants, selective serotonin reuptake inhibitors (SSIRs), serotonin and norepinephrine uptake inhibitors, and monoamine oxidize inhibitors and specifically such as aminoketone, amitriptyline, amoxapine, bupropion, doxepin, desipramine, clomipramine , fluoxetine, fluvoxamine, *Hypericum, Hypericum perforatum,* imipramine, isocarboxazid, maprotiline, mirtazapine, nefazodone, nortriptyline, nortriptyline, paroxetine, phenylpiperazine, phenelzine, protriptyline, sertraline, tranylcypromine, trazodone, triazolopyridine, trimipramine, and venlafaxine;

7. Central nervous system stimulants, generically such as xanthines and amphetamines, and specifically such as dextroamphetamine, *Ephedra, Ephedra sinica,* metamphetamine, methylphenidate, methylphenidate, and pemoline;

8. Anticonvulsants generically such as barbiturates, oxazolidinediones derivatives, carbonic anhydrase inhibitors, benzodiazepines, and GABA transaminase inhibitors; specifically such as carbamazepine, clobazam, dezinamide, divalproex sodium, felbamate, flunarizine, fosphenytoin, gabapentin, lamotrigine, levetiracetam, midazolam, milacemide, MK-801, oxcarbazepine, phenobarbital, primidone, progabide, stiripentol, tiagabine, topiramate, valproic acid, vigabatrin, and zonisamide;

9. Antianxiety drugs, generically such as antihistamines, barbiturates, benzodiazepines, beta-blockers, buspirone, propanediols, selective serotonin reuptake inhibitors (SSIR's), serotonin receptor agonists, and tricyclic antidepressants; specifically such as alprazolam, buspirone, chlordiazepoxide, clomipramine, clorazepate, diazepam, fluoxetine, fluvoxamine, halazepam, lorazepam, meprobamate, oxazepam, phenobarbital, *Piper, Piper methysticum,* prazepam, and propranolol;

10. Hypnotics (generally, hyptonics are drugs that are pertinent to sleep or hypnosis or are agents that cause an insensitivity to pain by inhibiting afferent impulses or by inhibiting the reception of sensory impressions in the cortical centers of the brain, thus causing partial or complete unconsciousness). Hypnotics generally include sedatives, analgesics, anesthetics, and intoxicants, and are sometimes called somnifacients and soporifics when used to induce sleep. Specific hypnotics include aliphatic alcohols, barbiturates, benzodiazepines, *Hypericum, Hypericum perforatum,* melatonin, certain non-barbiturates, *Piper, Piper methysticum, Valeriana, and Valeriana officinalis;*

11. Narcotic analgesics such as opioid receptor agonists and specifically such as heroin and morphine; and

12. Dopaminergic agents such as amantadine, benztropine, carbidopa/levodopa, and trihexyphenidyl.

EXAMPLES

The following Examples utilized both standard stimulating and recording devices and/or multi-electrode dish (MED) probes for simultaneously recording from 64 sites to address the origins and regional variations in cholinergically induced high frequency rhythms in hippocampal cortex. Additional pharmacological and physiological studies relating to the oscillations were then conducted.

Drugs/chemicals were purchased from Research Biochemicals International (diazepam, bicuculline, CNQX, 2-hydroxysaclofen) or Sigma (all other compounds). CX614 and CX691 were obtained from Cortex Pharmaceuticals Inc. (Irvine, CA). All drugs were bath applied at known concentrations and were prepared daily from frozen aliquots. Drugs/chemicals were added to the infusion line using an injection pump with the exception of AP5, which was bath applied. Solutions were freshly prepared on the day of the experiment. Statistical and measurement techniques included the use of paired, two-tailed t-tests and two-way ANOVAs with repeated measures. These tests were employed to assess statistical significance. Data are expressed as means $\pm$ S.E.M. Power spectra were estimated from 400 ms samples using the Fast Fourier Transformation function in MATLAB (MathWorks, Natick, MA). Power was normalized to the last 5 minutes of agonist infusion.

EXAMPLE 1

Preparation of Multi-Electrode Array (MED probe)

The general procedures for the preparation of the Multi-Electrode Dish (Matsushita Electric Industrial Co. Ltd., Osaka, Japan: "the MED probe") have been described above. Procedures for MED probe preparation have also described by Oka et al. (1999). The device has an array of 64 planar microelectrodes, each having a size of 50 x 50 μm, arranged in an 8 by 8 pattern. Probes come with two types of interpolar distance, 150μm (Panasonic: MED-P515AP) and 450μm (Panasonic: MED-P545AP).

To provide sufficient adhesion of the neuronal sample slice to the MED surface, the surface of the MED probe was treated with 0.1% polyethylenimine (Sigma: P-3143) in 25 mM borate buffer, pH 8.4, for 8 hours at room temperature. The probe surface was rinsed 3 times with sterile distilled water. The probe (chamber) was then filled with DMEM/F-12 mixed medium, containing 10% fetal bovine serum (GIBCO: 16141-079) and 10% horse serum (GIBCO: 16050-122), for at least 1 hour at 37° C. DMEM/F-12 mixed medium is a 1:1 mixture of Dulbecco's Modified Eagle's Medium and Ham's F-12 (GIBCO: D/F-12 medium, 12400-024), supplemented with N2 supplement (GIBCO: 17502-014) and hydrocortisone (20 nM, Sigma, H0888).

EXAMPLE 2

Preparation of Hippocampal Slices

Three to four week old male Sprague-Dawley rats were sacrificed by decapitation following halothane anesthesia under an accredited animal protocol from the University of California Institutional Animal Care and Use Committee with guidelines from the National Institute of Health. Brain tissue was removed, and for standard electrophysiological recording techniques, 350 μm hippocampal slices were cut using a vibrating tissue slicer (Leica VT1000; Bannockburn, IL). Artificial cerebrospinal fluid (ACSF) was of the following composition (in mM) for dissection: NaCl 124, KCl 3, $KH_2PO_4$ 1.25, $MgSO_4$ 5, $CaCl_2$ 3.4, D-glucose 10, $NaHCO_3$ 26.

Hippocampal slices were then placed on an interface recording chamber, and infused at a rate of 60 ml/hour with oxygenated recording ACSF of the same composition as described above except that the $CaCl_2$ and $MgSO_4$ concentrations were lowered to 3 mM and 1 mM, respectively. Humidified 95% $O_2$/5% $CO_2$ was additionally blown into the chamber throughout the recovery and recording periods. Slices were allowed to recover for a period of at least one hour.

For multi-electrode experiments, 17-24 day old Sprague-Dawley rats were sacrificed by decapitation after anesthesia using halothane (2-Bromo-2chloro-1,1,1-trifluoroethane, Sigma: B4388), and whole brains removed carefully. The brains were immediately soaked in ice-cold, oxygenated preparation buffer of the following composition (in mM): 124 NaCl, 26 $NaHCO_3$, 10 glucose, 3 KCl, 1.25 $NaH_2PO_4$, 2 $CaCl_2$, 2 $MgSO_4$, for approximately 2 minutes. Appropriate portions of the brain were trimmed and placed on the ice-cold stage of a vibrating tissue slicer (Leica: VT-1000S). The stage was immediately filled with both oxygenated and frozen preparation buffers. The thickness of each tissue slice was 350 μm. Each slice was gently taken off the blade by a painting brush, trimmed, and immediately soaked in the oxygenated preparation buffer for 1 hour at room temperature. A slice was then placed on the center of the MED probe. The slice was positioned to cover the 8x8 array. After positioning the slice, the MED probe was immediately placed in a box filled with 95% $O_2$ and 5% $CO_2$ and allowed to recover at 32° C for 1 hour.

EXAMPLE 3

Electrophysiological Recording

For standard electrophysiological experiments, field potentials were recorded using extracellular recording electrodes filled with 2M NaCl (1-5 MΩ). For recording of spontaneous and rhythmic activity, electrodes were placed in the stratum pyramidale of field CA3. For monitoring the excitatory postsynaptic potential (EPSP) evoked by Schaffer collateral/commissural stimulation, a recording electrode was placed in CA1 stratum radiatum. Stimulation was delivered through bipolar stimulating electrodes (twisted 64 μm insulated nichrome wires), and stimulation current intensity was set to evoke a response that was <50% of the maximum monophasic response (biphasic pulses of 0.1-0.3 ms duration, 10-50 μA). Samples of 800 ms were recorded every 30 seconds; in the cases involving stimulation, stimulation pulses were delivered every 30 seconds. Stable baseline conditions were monitored for at least 10 minutes prior to infusion of drug solutions. Data were recorded using a differential AC amplifier (A-M Systems Model 1700; Carlsborg, WA) and digitized at 5 kHz. Experiments were conducted at physiological temperatures (32 ± 2°C).

Spike and burst counts were computed by finding positive peaks in the second derivative of smoothed time series recordings, within 400 ms time windows, again using MATLAB. Spikes occurring at intervals ≤ 5 ms constituted a "burst". Spike and burst counts were normalized to the last five minutes of cholinergic agonist infusion, unless otherwise noted.

For multi-electrode experiments, the slices on the MED probe were placed in a small $CO_2$ incubator (Asahi Lifescience: model 4020) at 32° C. After recovery of the slice on the MED probe, the medium was replaced with DMEM/F-12 mixed medium without serum. The slices were on the interface, and contacted with a moisturized 95% $O_2$ and 5% $CO_2$ gas mixture. Under this condition, the responses were recorded for more than 2 hours.

Spontaneous and evoked field potentials at all 64 sites were recorded simultaneously with the multi-channel recording system (Matsushita Electric Industrial Co. Ltd., the "MED64 system") at a 20 kHz sampling rate. In the case of the evoked response, one of the planar microelectrodes out of the 64 available was used for cathode of stimulating. Bipolar constant current pulses (10 to 50 μA, 0.1 msec) were produced by the data acquisition software through the isolator. The stimulating microelectrode was selected by the 64 switch-box. The measured field potentials were then subjected to analysis using the CSD analysis (Nicholson et al.) discussed below.

EXAMPLE 4

Long Lasting Rhythms Induced By Carbachol

In this Example, the ability of carbachol to induce long lasting rhythmic activity in neuronal tissue is demonstrated. Figure 1 shows the long lasting rhythmic activity induced or stimulated by a cholinergic agonist in field CA3 of hippocampal slices, and conservation of this rhythmic activity after washout of the cholinergic agonist. Figure 1A shows the power spectra of the control (dotted line) and extracellular oscillations induced by 20 μM CCh (solid line). Figure 1B shows example traces of extracellular recordings prior to (above) and following (below) infusion of 20 μM CCh. Figure 1C then illustrates the persistence of oscillations after CCh washout. Each point represents the average normalized power (mean ± SEM) within the beta or gamma frequency band. CCh (20 μM) was infused for a 20-minute period (indicated by black bar) following at least 10 minutes of baseline recording.

Infusion of CCh (20 μM) into field CA3 caused the rapid appearance of fast rhythmic activity with a dominant frequency in the beta band (27.4 ± 0.2 Hz, at 10-20 min of infusion, n = 28) and a secondary frequency in the gamma range (48.2 ± 0.2 Hz, Fig.1A). Power in the beta range increased over thirty fold during infusion ($p < 0.0001$, $n = 28$). The waveform of the CA3 rhythm is illustrated in Fig. 1B. Each cycle begins with a large spike or burst of spikes, followed by a steep positive-going potential, which gradually declines until the cycle begins again. The spiking activity was largely absent from CA3 under baseline conditions. The increase in rhythmic activity was associated with a reliable decrease in the amplitude of the field EPSP recorded in the apical dendrites of field CA1 in response to stimulation of Schaffer collateral/commissural projections.

The depression of the fEPSP produced by CCh reversed quickly upon washout. Although recovery was never quite complete, this provided a measure of the time course for CCh wash-in and washout. In contrast to reversible effects observed with the evoked response, the rhythmic activity continued at a high level even after hours of washout. Fig. 1C shows normalized power in the beta and gamma frequency ranges during washout for a group of 14 slices. Power in the beta band after 50-60 minutes of washout was over twenty-fold greater than that recorded in the pre-CCh period ($p < 0.0001$, $n = 14$) and gave no evidence of decreasing with time. In three cases, recordings were continued for longer than three hours of washout, at which time high levels of rhythmic activity were still present. Similar effects were obtained in the gamma frequency range.

Figure 2 also shows the lasting increases in CA3 pyramidal cell activity levels after CCh washout. As seen in Figure 2A, representative traces show examples of spikes and bursts, indicated by '*' and '**' respectively. Figure 2B demonstrates spike rates (above) and burst rates (below) of CA3 pyramidal neurons plotted across time to demonstrate the sustained increases that result from transient CCh application. CCh was infused for 20 minutes. Each point (mean ± SEM) represents the spike or burst count within a 400-ms time window, normalized to the last five minutes of CCh infusion.

The induction of persistent rhythms was accompanied by long lasting changes in firing patterns of CA3 pyramidal neurons. Spiking and bursting spontaneously recorded in CA3 s. pyramidale, relatively infrequent under baseline conditions, increased following transient CCh application (Figure 2A), following the same time course as ongoing rhythms. In Figure 2B the spike rate (top) has been normalized to the mean rate during the last five minutes of CCh infusion. As can be seen in these group data, spiking increased about three-fold with infusion and remained close to that level throughout 60 minutes of washout ($p < 0.0001$ pre-CCh vs. 60 minutes after, n =14). The increased number of short, high frequency bursts of spikes produced by CCh also persisted without evident change throughout the washout period (Figure 2B, bottom). In all, the rhythms induced by cholinergic stimulation, as well as the spiking activity associated with them, remained in place for hours after the stimulation was removed.

EXAMPLE 5

<u>Changes in the Cholinergic Projections are Responsible for Long Lasting Rhythms</u>

Example 5 demonstrates that the induction or stimulation of oscillations in neuronal tissue is most likely derived from modulating cholinergic interactions. For example, Figure 3A illustrates the NMDA-independence of long-term increases in oscillatory activity. Specifically, Figure 3A shows five cases in which slices were bath-applied with 100 μM APV (D,L-2-amino-5-phosphopentanoic acid, NMDA antagonist) for 15 minutes prior to the start of CCh infusion, 20 minutes during which 20 μM CCh was injected into the infusion line, and 25 minutes following CCh washout. Representative EPSPs from CA1 s. radiatum are shown (inset) at the end of the APV infusion and 30 minutes post-APV washout. The mean beta power after 50-60 minutes of CCh washout was 76.0 ± 1.6% of the peak effect for the CCh alone group and 109.0 ± 4.7% for the CCh plus APV group ($p < 0.0001$). Thus, neither induction nor expression of stable rhythmic activity was shown to be blocked by NMDA receptor antagonists.

In contrast, Figure 3B shows long-term increases in rhythmic activity caused by elevation of endogenous acetylcholine levels. The acetylcholinesterase inhibitor, physostigmine, was applied for 20 minutes at a moderate concentration (5 μM). As shown in Figure 3B, physostigmine caused an increase in rhythmic activity that, other than being smaller in scale, was not detectably different from that produced by CCh. The peak effect was again in the beta range, with a secondary increase in the gamma band, and the resultant waves had the same spike-on-trough pattern seen with the agonist. One notable difference between CCh and physostigmine is that the latter had no evident effect on the fEPSP. Most importantly, the increased rhythmic activity persisted without evident change for the entire duration of the washout period; power in the beta and gamma ranges was approximately three times baseline power at the end of the recording session ($p < 0.0001$, n = 5).

Figure 3C specifically demonstrates the necessity of cholinergic muscarinic receptors for maintenance of beta and gamma rhythms. The muscarinic antagonist atropine (ATR) was infused at a concentration of 10 μM for a period of 20 minutes beginning 45 minutes after CCh washout, allowing rhythms time to stabilize. Representative EPSPs from CA1 s. radiatum are shown (inset) during atropine wash-in and washout. As can be seen in Figure 3C, atropine application decreases CCh-induced oscillatory activity, which is not revived after atropine washout.

The above data establish that cholinergic synapses underlie a use-dependent, long-lasting change in a naturally occurring aspect of hippocampal physiology. The question then arises as to whether the same synapses also express the change or trigger a reaction in a non-cholinergic system that drives rhythms. If the former hypothesis were true, then blocking cholinergic transmission after washout of CCh (or physostigmine) would be expected to have a profound effect on the induced rhythms. Figure 3C summarizes results confirming this prediction. The muscarinic antagonist, atropine, was infused starting 45 minutes after CCh washout, allowing long-term rhythms to stabilize and, as is evident, completely abolished the oscillations. Normalized power of the beta and gamma activity was reduced to near baseline levels by the last 10 minutes of atropine infusion ($p < 0.0001$ for beta and $p < 0.0001$ for gamma, n = 5). EPSPs were recorded in CA1 s. radiatum to monitor the health of the slice and appeared to remain strong and healthy during atropine wash-in and washout, the same period during which oscillations were lost (Figure 3C, inset).

The effect on oscillatory activity demonstrated by cholinergic agonists may also be similarly potentiated by acetylcholinesterase inhibitors. As seen in Figure 6, acetylcholinesterase inhibitors were infused for 30 minutes following a 10 minute baseline. Power was averaged across the 20-40 Hz frequency band and normalized to baseline activity. In Figure 6A, the carbamate derivative, physostigmine, elicited stable cholinergic rhythms at a relatively low concentration (2.5 μM) and increased beta power approximately three-fold. In Figure 6B, infusion of the huperzine analog, huperzine X (HUP-X) at a concentration of 2.5 μM produced rhythms of a power similar to those generated by physostigmine. As seen in Figure 6C, example traces demonstrate that the waveform and frequency of rhythms generated by acetylcholinesterase inhibitors were nearly identical to carbachol-driven rhythms. Note that little to no rhythmic activity was present under baseline conditions. Figure 6D shows that the rhythms produced by the huperzine analog, dimethylhuperzine A ((-)-DMHA), were concentration dependent. Rhythms were of high magnitude and stably maintained for the higher concentration (10 μM), but the lower concentration (2.5 μM) produced weak rhythms that appeared to lose power over time. Again, Figure 6E shows two concentrations of a huperzine analog, huperzine A ((-)-HA) which were tested with regard to their ability to produce lasting cholinergic rhythms. The 10 μM infusion produced stable, long-lasting increases in beta power, while rhythms following the 2.5 μM infusion were of low power and appeared to gradually decline.

EXAMPLE 6

<u>Reversal of Long Lasting Increases in Rhythmic Activity</u>

Example 6 further analyzes the unexpected finding of tonic rhythms being unaffected by washout of atropine. Loss of rhythmic activity in the atropine cases was not accompanied by reductions in the size of EPSPs during antagonist infusion and washout. Serotonergic activation has been reported to desynchronize hippocampal EEG (Assaf, S. Y. & Miller, J. J. *Neuroscience* 3: 539-550 (1978)) and thus, was used to determine if induced rhythms recovered after a transient suppression unrelated to cholinergic receptors.

Figure 3D shows temporary depression of rhythmic activity brought on by 5-HT infusion. 5-HT (30 μM) was infused for 20 minutes after 45 minutes of CCh washout. As shown in Fig. 3D, serotonin (5-HT, 10μM) caused an immediate reversal of rhythmic activity but, in marked contrast to the atropine result, the rhythms returned essentially unchanged upon washout of the agonist.

Figure 4 compares the effects of atropine and 5-HT on spike and burst frequency. Spike and burst rates are normalized to the average rates during the last five minutes of CCh infusion. Figure 4A provides examples of spontaneous activity recorded in CA3 s. pyramidale following induction of long-term rhythms immediately prior to, during, and 30 minutes after atropine infusion. Figure 4B shows the reversible effects of 5-HT on spike frequency contrasted with the irreversible effects of atropine. Figure 4C demonstrates the recovery and lack of recovery of lasting increases in burst frequency following 5-HT application and atropine application, respectively. Figure 4D shows representative samples of spontaneous activity recorded in the CA3 pyramidal cell layer immediately prior to, during, and 30 minutes after 5-HT infusion.

Reversal and subsequent recovery of long lasting rhythms following 5-HT application were accompanied by corresponding changes in CA3 pyramidal cell activity levels (Figs. 4B-D). In contrast, the complete elimination of rhythms due to atropine was associated with a permanent reversal of CCh-induced increases in pyramidal cell activity (Figs. 4A-C). These data suggest that the loss of rhythmic activity with the muscarinic antagonist is due to an effect unique to cholinergic transmission; i.e., intense activation of cholinergic receptors induces stable rhythms while blockade of the receptors eliminates them.

Figure 5 shows the effects of selective muscarinic antagonists on cholinergic plasticity. As seen in Figure 5A, the M2 selective muscarinic antagonist, methoctramine (MTC), does not eliminate long-term rhythms. MTC (10 μM) was infused for 20 minutes beginning at 45 minutes post carbachol washout. MTC application resulted in a slight decrease in power and subsequent recovery. In Figure 5B, infusion of the M1 selective muscarinic antagonist, pirenzepine (PRZ), reproduced the elimination of rhythms seen with atropine. A 20-minute pirenzepine (10 μM) infusion was initiated 45 minutes after carbachol washout. Long-term rhythms did not recover upon washout of the antagonist. Representative traces are shown (inset) to demonstrate that loss of rhythms was not associated with run-down or signs of fatigue in the CA1 evoked response to Schaffer collateral stimulation.

The results indicate that transient periods of cholinergic stimulation induce long-term rhythms, associated with lasting increases in cell spiking and burst rates. In that they involve naturally occurring physiology, are triggered by relatively brief episodes of intense activity, and remain long after the triggers are removed (three hours at least), the tonic rhythms resemble long-term potentiation. A plausible explanation for lasting increases in rhythmic activity would be that an induction of LTP at glutamatergic synapses, resulting from burst patterns induced by CCh, maintained the rhythmic activity via strengthened collateral connections. However, NMDA receptor antagonists at concentrations that completely suppress LTP did not interfere with the formation of stable rhythms. Subsequent experiments showed that facilitation of endogenous cholinergic activity resulted in the formation of stable rhythms while antagonism of endogenous cholinergic transmission eliminated already developed rhythms. In all, the long-term rhythms appear to be both induced and expressed by endogenous synapses, probably within field CA3. This observation leads to the further conclusion that the cholinergic projections to hippocampus are plastic and in particular undergo use-dependent changes that bear some resemblance to those found in LTP.

Because the long-term rhythms are not notably different than the acute oscillations in the presence of CCh or physostigmine, it is likely that they are due to the same end points produced by the drugs, namely stimulation of above normal numbers of cholinergic receptors.

One of the more surprising features of the rhythms was their erasure by transient exposure to a muscarinic antagonist. That is, not only were the rhythms blocked by the antagonist, as expected if the oscillations reflect ongoing cholinergic activity, but they also failed to return upon washout of the antagonist. This was not due to suppression of the rhythms per se because non-cholinergic agents blocked the oscillations only while present in the tissue. These results suggest that periods of subnormal cholinergic activity reset the lasting changes induced by periods of intense activity. This observation should be of help in identifying the changes that express the long lasting rhythms. Beyond this, reversibility has important implications for the possible functional significance of cholinergic plasticity. Possibly, the strength of the connections between the ascending cholinergic projections and their forebrain targets undergoes continuous adjustment, with the most recent period of (high/low) activity serving to set up activity for the events that follow.

In all, the data herein provide evidence that long lasting, use-dependent plasticity is not restricted to glutamatergic synapses. Additionally, these data link cholinergic fast rhythms to lasting changes in neuronal activity, consistent with a primary role for fast rhythms, and the cholinergic system in general, in mnemonic processing.

EXAMPLE 7

Current Source Density (CSD) Analysis

The well-studied methods of current source density analysis use the Laplacian transform ($\nabla 2$) on measured field potentials ($\phi$) to attempt to identify the locations and relative magnitudes of current sources and sinks (Im) (Howland et al., 1955; Mitzdorf, 1985 for review):

$$Im = -(\sigma x\ \nabla 2x\ \phi + \sigma y\ \nabla 2y\ \phi + \sigma z\ \nabla 2z\ \phi)$$

where $\sigma$ is the conductivity in each of the three orthogonal dimensions. The method is rarely used in its full three-dimensional form for electrophysiological measures (Nicholson, 1973; Nicholson & Freeman, 1975; Nicholson & Llinas, 1975) but rather a reduced form in one dimension is typically applied (Haberly & Shepherd, 1973; Ketchum & Haberly, 1993; Kolta et al, 1996). One-dimensional current source density analyses are, however, conducted in material of two or more dimensions (e.g., a brain slice), with the consequence that any currents occurring orthogonally to the axis of measure are undetected, and the resulting one-dimensional results may therefore be misleading. Care is thus taken in one-dimensional analyses to ascertain that there are minimal currents occurring laterally to the orientation in which samples are measured. Preferably, when the *in vitro* neuronal sample is a slice of the hippocampal region of the brain, alignment of the linear series of measures is made so as to be parallel to the direction of apical dendritic growth (as it has been demonstrated that currents lateral to apical dendrites are very small relative to currents occurring along the apical-proximal axis). The technique used in this Example is a two-dimensional method in which simultaneous samples are recorded from multiple electrodes in an equidistant array, enabling the continuous sensing of current flows in any direction within the plane of the slice, regardless of the relative orientation of the rows and columns of the array and the dendritic processes present in the slice. The array is made up of 64 planar electrodes, each with a size of 50 x 50 $\mu$m, arranged in an 8 x 8 pattern with interpolar distances of 150 $\mu$m or 450 $\mu$m (Oka et al., 1999).

After low pass filtering at 100 Hz, the data is spatially smoothed by a 3x3 weighted average kernel (0 1/8 0, 1/8 1/2 1/8, 0 1/8 0) and the result convolved with a 3x3 Laplacian kernel (0 1 0,1 -4 1, 0 1 0) to produce a discrete approximation of the second spatial derivative. Preferably, the medium is considered ohmic with a homogeneous conductance. The full correlation matrix is computed for all channels (64) in the time window considered (0-3 sec). Current vs. time plots for single points in the slice is obtained by computing the 8x8 current source density for each time step, and calculating the value at the desired location via bilinear interpolation. Well-recognized limitations on the resolution affordable by current source density analysis arise from the relationship between the interelectrode distance (sampling resolution) and the radii of current sources or sinks occurring in the slice, and would be appreciated by one skilled in the art. The phenomenon of 'aliasing,' well known in the realm of computer graphics interfaces, refers to the occurrence of spurious data "ghosts" (aliases) when the distance between sampling points is larger than the size of the smallest phenomena to be represented. In computer screen graphics this occurs when the screen resolution is insufficient for the image being displayed (Figure 7 left panel - A high-resolution image with smooth edges, Figure 7 middle panel -- The same image as sampled with relatively low-resolution inter-pixel distance appears to have new features with high spatial frequency (the jagged stairsteps)). The appearance of spurious images on the screen is typically treated by 'anti-aliasing' that incorporates low-pass filtering of the image with introduction of partially shaded pixels at the image's edges (Figure 7 right). Anti-aliasing includes a low-pass filter that removes the spurious alias features from the image, and therefore may also (as in the present instance) eliminate some high spatial frequency detail from the original image. The corresponding potential introduction of artifacts into array-sampled physiological data can be removed by the same anti-aliasing method (see Figure 8 below), and will correspondingly eliminate some fine (high spatial frequency) detail from the data. Preferably, the analysis uses low-pass filtering without full anti-aliasing. Alternatively, if the data contain phenomena with spatial frequencies too high to be resolved by the inter-electrode sampling distances, the result is low-pass filtered to remove spurious aliases while also removing high frequency data (Figure 8).

Specifically, Figure 8 depicts the effects of anti-aliasing. The top panel shows an instance of inter-sensor spacing sufficient for the resolution of the sampled data. Aliases appear as very high frequency features; low-pass filtering at one-half the inter-sensor spatial frequency (double the inter-sensor distance) eliminates all aliases without reducing resolution of the sampled data. On the other hand, the bottom left panel in Figure 8 shows the instance in which the inter-sensor spacing was insufficient for the resolution of the sampled data. Spatial frequency of aliases overlap the highest frequencies present in the data. The left panel shows that low pass filtering at one-half inter-sensor spatial frequency will fail to eliminate all the aliases, and yet will eliminate some of the finest detail of the data. The right hand panel in Figure 8 shows that low pass filtering at a lower spatial frequency successfully eliminates all spurious aliases, and further reduces the resolution of the sampled data, eliminating much of the high-frequency fine detail that may be present in the measured data. Only lower frequency or larger events remain.

EXAMPLE 8

Continuous, Two-Dimensional, Current Source-Density Analysis

Two-dimensional current source density analyses were conducted in the context of stimulation of the Schaffer-commissural afferents to field CA1 in a hippocampal slice preparation. Figures 9A and 9B illustrate a typical experiment. Figure 9A shows the placement of the slice on an 8x8 MED electrode array, with interelectrode spacing of 150μm, centered in the apical dendritic field of CA1 in a hippocampal slice. The electrodes cover the basal dendrites of CA1, apical dendrites of CA1, and the upper blade of the dentate gyrus granule cell field on the electrode array. To prevent the GABAA- and GABAB- mediated inhibitory components, this experiment was carried out with 50 μM picrotoxin and 100 μM 2-hydroxysaclofen. Figure 9B shows a typical postsynaptic current elicited (EPSC) by a single stimulation pulse to the electrode indicated in dark gray (10) and measured from the electrode in light gray (12). The time scale is typical for such evoked responses. Vertical dotted lines mark the time points for which measures will be taken across all 64 electrodes and shown in Figure 10 below.

Figure 9B illustrates the time course and magnitude of the postsynaptic current at the indicated electrode, which is in the proximal portion of the apical dendrites of CA1. The direction of the current sink and source are as indicated; after the initial fiber volley (lasting approximately 1 msec) a current sink increased over a period of about 5 msec and decreased over the subsequent five milliseconds before returning to baseline at roughly 13 msec. It became a current source and lasted for approximately another 15-20 msec before returning to baseline at about 35-40 msec. The relative magnitudes and time courses of the sink and source are typical for such evoked responses.

Two-dimensional current source density analysis was performed to obtain comparable measures of current sources and sinks of the same response. Figure 10 illustrates the continuous two-dimensional current source density analysis of the evoked response shown in Figure 9B. Each panel in Figure 10 illustrates the computed instantaneous sources and sinks in the slice, at selected times indicated by vertical dashed lines in Figure 9B. The array was positioned on the slice as shown in Figure 9A; the cell body layer of hippocampal field CA1 defines a roughly horizontal curve about one quarter of the way from the top of each panel. The dotted lines indicate the cell body layer of CA1 (top) and the most distal extent of the apical dendritic field (bottom). As seen in the calibration bar at bottom, sinks are black and sources are white, against a current-neutral background of gray.

It can be seen that after the initial fiber volley (lasting roughly 1 msec), a current sink in the apical dendrites of CA1 rapidly rose over a period of approximately 5 msec (see panel marked '6 ms') and fell over the subsequent five milliseconds before disappearing at about the 11 ms panel with the singularity appearing at the site of stimulation. After an interim of about two milliseconds during which currents were indistinguishable from neutral background, a source appeared in the apical dendrites ('15 ms') and lasted for approximately another 15-20 milliseconds before disappearing at roughly 35 ms. The time courses and magnitudes of the waveform from a single electrode (Figure 9B) can be seen to correspond closely to the computed current source density sink-source series in Figure 10.

Revealed by the two-dimensional current source density method are spatial aspects of the current sources and sinks that are difficult to discern by other means. The excitatory postsynaptic current sink spread across the apical field of CA1, in the region of Schaffer commissural fibers presumably stimulated by the initiating current pulse (panels from 3 ms to 9 ms); the ensuing current source occupied approximately the same zone (panels 15 ms to 27 ms). Both the sink and subsequent source that occurred in the apical dendrites were accompanied by currents of reversed polarity in the basal dendritic field of CA1 (near the top of each panel). Thus the predominant evoked response can be characterized as a current sink-source dipole that occurred from 3 to 9 msec and reversed to form a current source-sink dipole from 15 to 27 msec. Other, smaller currents were present in the slice but are not discussed here.

After a brief pause, a current source appears in these dendrites, with its center slightly more distal than that of the current sink. The apical source intensifies, expands, dissipates and disappears by about time 50 msec, making the 'period' of the evoked response about 50 msec. Both the apical sink and ensuing apical source are accompanied by a field of reversed polarity appearing in the basal dendrites of CA1 (top of each panel) that grows and dissipates with approximately the same time course as the apical events.

As described, the anti-aliasing performed in these analyses to prevent the introduction of artifacts due to aliasing will cause a loss of data with sufficiently high spatial frequencies. The filtering passes only those phenomena with spatial frequencies at most one-half that of the sampling frequency; in the present Example the inter-electrode distance was 150μm, preventing the measurement of current sources or sinks smaller than about 300μm across (150μm radius). This could be a shortcoming in a structure the size of hippocampal field CA1, and it is expected that fine spatial detail is lost in the method. It is instructive, therefore, to examine the spatial resolution of the CSDs measured by this method.

Figures 11A and 11B depict the boundaries of the apical dendrites of field CA1 superimposed on the region of measurement.

Figure 11A shows the slice and the position of the array (from Figures 9A and 9B); on the right is the instantaneous CSD in the region of the electrode array (from Figure 10), at 6 msec after stimulation at the indicated electrode. The top of the array coincides with the basal dendrites of CA1; the upper-central portion of the array overlays the field of apical dendrites in CA1, and the bottom third of the array overlays the upper blade of the dentate gyrus. The dotted lines indicate the cell body layer of CA1 (top) and the most distal extent of the apical dendritic field (bottom).

As seen in Figure 11B, The primary measured current sink (black) occupies only the region where apical dendrites occur, and the reciprocal current source (white, top) occurred only in the region of basal dendrites. (An absence of current appeared in the computed image at the location corresponding to the stimulating electrode; no recordings were taken from that electrode and the resulting CSD processing left a gap.) The physiological response computed by continuous two-dimensional current source density analysis 6 milliseconds after the stimulation of a single electrode (indicated in dark gray); the image is a close-up of the 6 millisecond frame from Figure 10B. Indicated are the limits of the apical dendritic field of CA1 by dotted lines. It can be seen that the extent of the evoked current sink closely corresponds to the limits of the apical dendrites. An apparent hole in the current sink occurs at the site of the stimulating electrode, where no recording is performed and no current source density is computed. There is little current in the cell body layer itself, and current source appears in the basal dendrites (top of panel). There is little current at the apical tips of the CA1 dendrites (middle of panel) and some less intense current sources occurring in the apical dendrites of the granule cells of dentate gyrus.

It can be appreciated that the borders of the computed sink do not coincide precisely with the location of the anatomical limits of the dendritic field, and inaccuracies that may result from the resolution limits the method.

EXAMPLE 9

Distribution and Current Sources for Carbachol-Induced Beta Waves Within the Hippocampus

Figures 12A-12E depict the distribution of carbachol-induced beta waves within a hippocampal slice. Figure 12A contains a micrograph of a hippocampal slice and an underlying 64 electrode array with 450 μm between recording positions (the 'broad array'). Subfields of hippocampus and overlying cortex may also be seen. Figure 12B shows spectra of carbachol-induced spontaneous activity at 20 electrode sites that contact part of hippocampus in the slice. Each x-axis is on a logarithmic scale from 1 to 100 Hz. Activity is seen in the 10-30 Hz frequency range, especially in apical dendrites of fields CA1 and CA3, with lower levels of activity elsewhere. The calibration bar is $5x10^{-11}$ $V^2$. As is seen in Figure 12C, the baseline activity was of low voltage and generally devoid of activity and there were no reliable differences between subfields.

Infusion of carbachol was followed by a gradually developing rhythmic activity in fields CA1 and CA3 that was close to or within the beta band (10-30 Hz) in 41 of 55 slices tested (Fig. 12D for an example). Thirty-four out of the forty one slices had beta band oscillations in field both CA1 and CA3. Low voltage activity at the upper end of the theta range was observed in field either CA1 or CA3 in the remaining seven cases. The relative prominence in CA1 vs. CA3 was not consistent across slices. High frequency activity was also found in the dentate gyrus, much more so in the internal than external wing of the structure (Figs. 12B, 12D). Figure 12E shows a close-up of carbachol-induced activity in the upper left electrodes in the array, indicating the reversal of polarity across the cell body layer of field CA1. In Figure 12E, the calibration bars are 0.1 mV; 250 msec. As may be seen in the higher gain record of Figure 12E, in the pyramidal cell fields, the waves were larger in the apical than in the basal dendrites and typically had phase reversals between the two loci.

Fast Fourier transforms were used for quantitative analysis of the frequency and distribution of the carbachol-induced rhythms. For the example illustrated in Figure 12B, most of the power in the spectrum was found between 10 and 30 Hz with a definite peak at 20 Hz. The group mean for the dominant CA1 frequency for all slices was 18.8 ± 5.7 Hz (mean ± s.d.) and 19.2 ± 4.8 Hz for the beta instances alone. The equivalent values for CA3 were 16.4 ± 5.7 and 17.3 ± 4.2 Hz. The differences between CA3 and CA1 did not reach statistical significance. The recording sites with the greatest power in the 10-30 Hz band were located in the apical dendrites of field CA3 and CA1, typically in the more distal fields.

As may be seen in Figure 13A, arrays with 150 μm spacing (the 'dense array') provided finer spatial resolution of the rhythmic activity. Recording centered on the CA3/CA1 border revealed a surprisingly steep gradient of absolute potentials in the distal to proximal dimension with the largest potentials occupying a discrete region corresponding to stratum moleculare of fields CA3a and CA3b (Figure 13B). It is noteworthy that potentials reversed across the cell layer boundary (see, e.g., upper left quadrant of Figure 13B). Lesser though still substantial voltage bands are also present in CA1. The frequency spectra for all channels are shown in Figure 13C.

Continuous two-dimensional current source density analyses were conducted to further define the sources of these cholinergically induced oscillations. Figure 14 illustrates the instantaneous sources (white) and sinks (black; against a neutral background of gray), at 4-millisecond intervals from an arbitrarily chosen starting time of 0 during the same spontaneous activity a dense array shown in Figure 13. The outlines of the granule and pyramidal cell fields of CA3 and CA1 and the extents of their apical dendrites are superimposed to illustrate the locations of events occurring during the time period. The array includes part of the upper blade of the dentate gyrus. Each frame shows the instantaneous computed current source density in the region of the electrode array. From an arbitrarily chosen starting point, a sink appears in the apical dendrites of the border between fields CA3 and CA1, with an associated source across the cell boundary layer in the basal dendrites. A large sink in apical CA1 is accompanied by a source in the basal dendrites of CA1. The sink and its accompanying basal source intensified and expanded over the course of roughly 12 milliseconds, and both subsided to approximate baseline levels by roughly 16 msec after initial appearance. After a brief interim during which activity is not distinguished from background, a source appears in the apical dendrites at about 20 msec, with a corresponding sink in the basal dendrites. These expand and intensify before dissipating by roughly 20 msec later (40 msec), after which an apical sink reappears to re-initiate the cycle. The source (and its accompanying dipole) lasted for roughly 20 msec before dissipating. It is noteworthy that the apical source appeared to have its center more distally located than that of the more focal apical sink which preceded it, to within the resolving power of the interelectrode spacing of 150 μm. The sink-source dipole recurred and gave rise to the frequency plot shown in Figure 13C; the time course from peak to peak of this recurring apical sink-source dipole was approximately 40 msec, consistent with a frequency of ~25 Hz.

Figures 15A and 15B show a typical record of the repetitive oscillations induced by carbachol in a slice. The source-sink and sink-source dipoles, and their sharply defined boundaries between sources and sinks along the pyramidal cell body layer of the *in vitro* neuronal sample, were recurring features across many experiments, as was the latency from one apical sink through an apical source to the next apical sink

Figure 15A shows a hippocampal slice on a broad (450μm interelectrode spacing) array. The electrodes used to measure basal dendritic responses of pyramidal cell fields are indicated in light gray; those used to measure apical dendritic responses are in dark gray. Figure 15B shows averaged responses from apical (black) and basal (light gray) dendritic fields are shown over 500 msec (0.5 second) of elapsed time for the slice in Fig.15A. Carbachol-induced (20 μM) oscillations were sustained across all sampled periods, as in this typical response. Average apical and basal responses are reversed in polarity; i.e., are 180 degrees out of phase. The sustained occurrence of alternating current source-sink dipoles across the cell body layers of CA3 and CA1, within the range of 10-30 Hz (beta), were robustly observed in the majority of slices and time periods sampled.

Shifts between sinks and sources over larger areas were assessed with continuous two-dimensional current source density analyses using an electrode array with interelectrode spacing of 450μm, as opposed to previous current source density examples, which used arrays of 150μm distances. The resulting aliases and antialiasing (see Figure 8) necessarily entailed a further loss of resolution; for interelectrode spacing of 450μm and concomitant antialiasing, the smallest events that can reliably be imaged are those with a radius of 450μm (diameter of 900μm) or larger.

Figure 16 shows the computed evolution of current source density over 42 msec. Each frame shows the computed current source density at a particular time (indicated in milliseconds in the upper left corner of each frame) during recording of spontaneous activity with a broad (450 μm spacing) array. Black indicates maximum magnitude of sinks; white indicates maximum magnitude of sources. The positions of the pyramidal and granule cell fields are indicated.

Beginning at an arbitrarily chosen time of 0 msec, a weak current source in the apical dendrites of field CA3 is surrounded by diffuse sources centered on the pyramidal cell layers. An intense focal sink in the apical dendrites or proximal s. radiatum of field CA3 begins at about 6 msec, accompanied by sources in the cell and basal dendrite layers of CA3. The sink grew and a fully developed sink-source relationship centered on CA3b was evident at the 9 msec time point. The sink continues through roughly 12 msec before beginning to expand toward field CA1. The sink in apical CA3 dissipates at roughly 18 msec, followed about three msec later by the apical sink in CA1 and was accompanied by the appearance of a pronounced cell body / basal dendrite source at 15 msec. Note that by this time point the sink-source relationship in CA3 had begun to collapse. The basal sources dissipate at roughly the same time as their associated apical sinks. An apical source begins in CA3 at about 24 msec, accompanied within a few milliseconds by a mild basal source in CA3; the apical source then expands towards CA1, with an accompanying basal sink in CA1. These source-sink pairs then dissipate after about 15-20 msec. Such cycles recur irregularly in this slice. The wave involved an intense apical dendritic sink lasting about 10 msec followed by an apical source lasting for about twice that period. These events began in CA3 and were seen in CA1 within 3-5 msec. In summary, cholinergically induced beta range rhythms have current sources that are located in the apical dendrites and that are usually better defined than the more proximally situated current sinks.

These observations showed that relatively brief apical sinks are interposed between the longer lasting apical sources. Examination of averaged currents demonstrated that distribution of sources and sinks across the pyramidal cell subdivisions of hippocampus varied between slices. Between-slice variability was also present in serial current source density analysis with major differences in the degree to which events were centered in CA3 vs. CA1. However, the brief apical sink - longer apical source sequence for the apical dendrites was prominent in all slices exhibiting oscillations in the beta range.

EXAMPLE 10

Carbachol Induces a 40 Hz Rhythm in Retrohippocampal Cortex

The question of whether the rhythms elicited by carbachol are regionally differentiated was addressed by close examination of those slices in which the electrode array was positioned underneath a significant portion of the retrohippocampal cortex field along with the pyramidal cell fields of hippocampus itself. Figure 17A shows a micrograph of a cortico-hippocampal on a broad array. Two sites of interest -- one in the CA1 (100) and one in the deep layer of the entorhinal cortex (102) are shown. Carbachol (50μM)-induced fast waves from the indicated positions in field CA1 and entorhinal cortex are compared in Figure 17B. Power values are given in the drawing as times $10^{-11}$. As may be seen in Figure 17B, the entorhinal oscillations have a higher frequency than those from the hippocampus. Fast Fourier transforms indicated that the dominant frequency in the cortex is about twice that in field CA1. Rhythmic activity with a peak near 20 Hz was found through the apical dendrites of the hippocampal pyramidal cell fields while the 40 Hz activity was predominantly associated with the entorhinal sites. It was also observed that the 40 Hz oscillations were centered in the deep layers of the medial entorhinal cortex (right top spectrum in Figure 17B). Regions lying between the hippocampus and medial entorhinal cortex exhibited both peaks. Indeed, it appears the relative balance of 40 vs. 20 Hz increases in an orderly manner across the series of steps included in the retrohippocampal cortex. Forty Hz activity was recorded in the deep layers of the entorhinal cortex in each of the 15 slices which had appropriately positioned electrodes; it thus appears to be a characteristic response of this region to cholinergic stimulation. Results for all recording sites are summarized in Figures 11C (distribution of representative activity in the slice (calibrations bar: 0.1mV, 500msec.)) and Figure 17D (distribution of low-pass (0-100 Hz) filtered power spectra in the slice (Calibration bar: 2 x $10^{-11}$ $V^2$)).

EXAMPLE 11

Transmitter Systems Involved in Carbachol-Induced Beta Waves

Carbachol-induced high frequency rhythms were completely eliminated by 20 μM atropine and greatly reduced by the AMPA receptor antagonist CNQX (20 μM). In the presence of bicuculline (10 μM), muscarinic stimulation produced high frequency spiking in the stratum pyramidale and in some instances epileptiform discharges, but rhythmic activity was absent. Under these conditions, carbachol initiated repetitive bursting behavior and occasional seizures. The interval between the epileptiform bursts sometimes approximated the period of the theta wave (data not shown). These seizures were similar to the activities reported by Williams and Kauer (1997).

EXAMPLE 12

Orthodromic Activation of Pyramidal Cells Triggers Beta-Like Activity in the Apical Dendrites

How the GABAergic cells responsible for apical sources are activated is clearly of importance for understanding the origins of beta oscillations. If pyramidal cell collaterals are involved, then orthodromic stimulation sufficient to cause repetitive spiking should result in the appearance of beta like activity in the areas in which the carbachol-elicited rhythms are found. Single pulse stimulation of the Schaffer-commissural fibers does not typically cause repetitive spiking because (i) the potent feed-forward dendritic inhibition in the stratum radiatum shunts the excitatory current and (ii) perisomatic inhibition prevents repetitive discharges. Concentrations of bicuculline that partially block the GABAa receptor pool were used to reduce these inhibitory responses and thereby allow the pyramidal cells to emit 4-5 spikes in response to orthodromic stimulation.

Figures 18A-18D show a two-dimensional current source density analysis of beta-like activity elicited by orthodromic activation of field CA3. Figure 18A shows the position of the array (150μm interelectrode spacing) with respect to a hippocampal slice, centered in field CA3. Stimulation was induced at the electrode indicated by solid color (106) and was recorded at the circled electrode (108). As noted below, Figure 18B shows a typical response to orthodromic stimulation of field CA3. The response exhibits a small burst of spikes followed by a positive-going wave lasting approximately 30-40 msec. Arrows indicate the peak sink (53 msec) and peak source (83 msec). Fig. 18C shows the distribution of power spectra in the slice: the largest responses are in apical dendrites of CA1 and apical and proximal dendrites of CA3. Fourier transforms showed that the frequency spectrum for the response had peaks at 15-20 Hz and at 100-200 Hz (Fig 18C); note that the log scale used extends from 1 to 1000. Fig 18D shows a two-dimensional instantaneous current source density analyses at two time points (53 msec and 83 msec after stimulation, indicated by arrows). The 15-20 Hz component was pronounced in the distal apical dendrites and had a distribution similar to that for carbachol-induced beta waves. At 53 msec, the evoked current sink is at its peak, and can be seen to occur predominantly in the apical dendrites of CA3, with a corresponding source in the basal dendrites. At 83 msec, the rebound source peaks; it can be seen also to occur in the apical dendrites of CA3, with a corresponding sink in the basal dendrites.

Responses of the type described in Figure 18B were sensitive to bicuculline concentrations and were blocked by it at 20 μM. Current source density analyses of the late evoked response showed a source that ran in the stratum radiatum about 200-400 μm above the s. pyramidale from the stimulation site near the hilus towards CA1; this was paralleled by a dense sink in the cell bodies and basal dendrites (Figure 18D).

EXAMPLE 13

Benzodiazepine Identification and Characterization

Figures 19A-19C show the effect of benzodiazepines on carbachol-induced beta waves. Benzodiazepines markedly enhanced the amplitude of beta oscillations as can be seen in a comparison of Figures 19A (20 μM carbachol alone) and 19B (same slice with carbachol plus 3μM diazepam). A Fourier transform indicated that the increased amplitude was not accompanied by a significant change in the frequency of the waves. Rhythmic activity is greatly enhanced and spreads to regions that were relatively inactive. (Calibration bars for Figures 19A and 19B: 0.2 mV, 500 msec).

Figure 19C summarizes the frequency spectra for recording loci within the hippocampus and shows the superposition of power spectra in the absence (black) and presence (gray) of diazepam. This showed that diazepam (gray spectra) caused a nearly threefold increase in power within the 20 Hz band over carbachol alone (black spectra), without much shift in the frequency. In addition, a peak is added at a higher (approximately 40 Hz gamma) frequency by the addition of diazepam. (Calibration bar: 5 μV).

Further, Table 1 summarizes the results for 8 experiments in which carbachol-induced high frequency rhythms were present prior to the infusion of diazepam. The increase in power at peak frequency was 321 ± 170% for CA3 and 217 ± 137% for CA1. Within-slice effects of diazepam were correlated (r = 0.93) and the increase in CA3 was statistically greater than that in CA1 ($p<0.05$, paired t-test, 2 tails). The frequency of the oscillations after diazepam was correlated across slices with that recorded under carbachol alone (CA3: r = 0.85; CA1: r = 0.96).

Table 1. Summary of eight experiments in which carbachol-induced high frequency rhythms were present in a hippocampal slice prior to infusion of diazepam. Shown are the maximal percent increases in power at the peak frequency in fields CA3 and CA1.

| Slice # | CA3(%) | CA1(%) |
|---|---|---|
| 1 | 639 | 521 |
| 2 | 344 | 264 |
| 3 | 414 | 211 |
| 4 | 197 | 119 |
| 5 | 431 | 200 |
| 6 | 177 | 213 |
| 7 | 132 | 117 |

Table continued

| Slice # | CA3(%) | CA1(%) |
|---|---|---|
| 8 | 235 | 90 |
| mean ±sd | 321 ± 170 | 217 ± 137 |

As is seen, the application of a member of the benzodiazepine class of compounds markedly enhanced the amplitude of beta oscillations. For the results shown in Figs. 20A-C and 21A-C, the potentials were measured for the respective samples with carbachol alone and with carbachol plus two type benzodizepines: one example being 3 μM diazepam (Figures 20A-C), and another being 20 μM flurazepam (Figures 21A-C).

Figure 20A is a photomicrograph of a hippocampal slice placed atop the MED array. Similarly, Figure 21A is a photomicrograph of the hippocampal slice atop the MED array used there. The inter-electrode distance was 450 μm. The results of the Fast Fourier Transform on both sets of data are shown (with the significant anatomical boundaries in the background in light gray respectively in Figures 20B and 21B). The so-analyzed data indicate that the increased amplitude of the benzodiazepine-infused data is not accompanied by a significant change in the frequency of the waves. As is seen in Figures 20C and 21C, both benzodiazepines caused a nearly threefold increase in the power within the 20 Hz band. The increase in power at peak frequency was about 300%.

EXAMPLE 14

Ampakine Identification and Characterization

In this Example, a wide spaced MED was used with a hippocampus sample (shown with the underlying microelectrodes respectively in Figs. 22A and 23A). The potential was again measured for the sample with carbachol alone and subsequently with 10μM Ampakine (CX614 in Figs. 22A-C and CX691 in Figs. 23A-C). The results of the Fast Fourier Transform on both sets of data are shown respectively in Figs. 22B and 23B (with the significant anatomical boundaries in the background in light gray and thence correlated to the data). The resulting data show the increased frequency of the Ampakine-infused data was also accompanied by a significant change in the amplitude of the waves. Specifically, Figs. 22C and 23C summarize the frequency spectrum for the specifically chosen recording loci. This shows that the Ampakines not only caused a significant increase in the power within the 20 Hz band (arrow) but also resulted in a 10 Hz shift in the peak.

Ampakines caused a marked reduction in synchrony and a secondary peak of slightly greater than 10 Hz. The resulting data show the decreased amplitude of the Ampakine-infused data is accompanied by a slightly lower shift in the frequency of the waves. Both of the structurally dissimilar Ampakines not only caused a significant decrease in the power within the 20 Hz band but also resulted in a 10 Hz shift in the peak. As shown, the secondary peak appeared to be centered in the dentate gyrus and field CA3 as opposed to the CA1-subicular localization for pre-Ampakine beta rhythm.

EXAMPLE 15

GABA Antagonist Identification and Characterization

Antagonists of GABAergic receptors caused a marked reduction, and desynchronized activity punctuated with complex spikes. Figures 34A-C and 35A-C respectively show the effect of bicuculline and picrotoxin on carbachol-induced beta waves.

Figure 34A is a photomicrograph of a hippocampal slice placed atop the MED array. Similarly, Figure 35A is a photomicrograph of the hippocampal slice atop the MED array used there. The inter-electrode distance was 450 μm. The potentials were measured for the sample with carbachol alone and subsequently with the two listed type GABAergic antagonists: 10 μM bicuculline (Figs. 34A-C) and 50 μM picrotoxin (Figs. 35A-C).

The results of the Fast Fourier Transform on both sets of data are shown respectively in Figs. 34C and 35C (with the significant anatomical boundaries in the background in light gray and thence correlated to the data). The resulting data showed that the amplitude of the antagonist-infused data markedly decreased after administration of either of the GABAergic antagonists. Said antagonists not only caused a significant decrease in the power within the 20 Hz band but also resulted in a slow component, which consisted of complex spikes. These spikes appeared to be centered in the CA1, subiculum and cortex. Figs. 34B and 35B show the selected data from Figs. 34C and 35C.

EXAMPLE 16

AMPA Antagonist Identification and Characterization

Structurally different antagonists of AMPA-type glutamate receptors caused a very rapid loss of synchronized activity in the *in vitro* neuronal sample previously treated with carbachol. The potentials were measured for the sample with carbachol alone and subsequently with three types glutamate antagonists. One example is 20 μM CNQX (Figs. 36A-C), one is 20μM DNQX (Figs. 37A-C) and the other is 10 μM NBQX (Figs. 38A-C). The results of the Fast Fourier Transform on each respective set of data are shown in Figs. 36C, 37C, and 38C (with the significant anatomical boundaries in the background in light gray and thence correlated to the data). The resulting data show the amplitude data of the antagonist-infused samples was significantly diminished and/or eliminated. All three antagonists caused a significant decrease in the power. Figs. 36B, 37B, and 38B show the selected data from Figs. 36C, 37C, and 38C.

EXAMPLE 17

Ampakines vs. Benzodiazepines: Differentiation Using Beta Oscillation in a Rat Hippocampus Slice

Methods

Preparation of multielectrode arrays. The multielectrode arrays were prepared as described in Example 1.

Preparation of hippocampal slices. The hippocampal slices were prepared as described in Example 2, except that the preparation buffer contained 0.75 mM MgSO4 and the tissue slices were 375 μm.

The slices were incubated in the presence of 5 μM carbachol (CCh), a muscarinic receptor agonist, for 20 min and then transferred into the center of the MED probe. After positioning the slice into the desired orientation and taking a picture of the slice on the MED probe, the MED probe was placed on the MED connector in an incubator at 32 ºC. The slices were continuously superfused with ACSF containing 5 μM CCH at 1 ml/min.
Psychoactives. Triazolam (TZL), chlordiazepoxide (CDP), and flurazepam (FZP) were purchased from Research Biochemicals International (RBI).
Cyclothiazide(CTZ) was purchased from Sigma. CX516 and CX546 were obtained from Cortex Pharmaceuticals. Each drug was dissolved in ACSF containing 5 μM CCh on the day of the experiment. The concentration of each psychoactive was high enough so that at least the effect in power was significant.
Electrophysiological recording. During electrophysiological recording, the slices on the MED probe were placed in the incubator at 32 ºC. The slices were kept in the interface, and a moisturized 95% O2 and 5% CO2 gas mixture was blown from above. Under these conditions, electrophysiological responses were recorded for >2 hr. Spontaneous field potentials at all 64 sites were recorded simultaneously with the MED64 system (Panasonic) at a 5 kHz sampling rate. Samples of 1 second duration were recorded every 30 seconds. Stable baseline conditions were monitored for at least 20 minutes prior to infusion of the chemical solutions.
Fig. 24 shows the effect of CX546 on CCh-induced rhythmic activity in hippocampal slices. Figure 24A is a micrograph of a hippocampal slice on the MED probe. Figure 24 B shows samples of baseline and drug wash-in activity recorded at an electrode located in apical dendrites of field CA3 (shown by a square), where the largest activities are obtained.

Statistics and Measures

Power spectra of the rhythmic activity were calculated using the Fast Fourier Transformation function in MED64 Performer 1.5 (Panasonic).
In Fig. 25, samples of frequency and power of the rhythmic activity were plotted over time. Statistical significance of the effects of the drugs were assessed by comparing 10 responses each for the last five minutes of baseline and drug infusion across six (or seven) experiments (slices) using "Two-factor ANOVA with replication" in Microsoft Excel.

Results

Data were obtained from 37 stable extracellular recordings in which the baseline power was within the range of 50-400 (167 ± 14.3)μV2. The main component of the baseline rhythmic activity was in the beta range (24.74 ± 0.56 Hz). After recording the baseline for at least 20 min, the drug solutions were infused continuously for 20 min at the same flow rate (1 ml/min). The concentrations of drugs were 10 μM for benzodiazepines, 100 μM for cyclothiazide and 250

μM for ampakines (CX516 and CX546).

Figure 26 shows percent changes in frequency and power between the last five minutes (10 samples) of baseline and each drug infusion. Changes in power were similar within the class but opposite between the classes of drugs. Benzodiazepines, namely FZP, CDP and TZL increased beta range power by 55 ±18 % (mean ± s.e.m.; n=6), 55 ± 13 %(n=6), and 64 ± 17% (n=6), respectively. On the other hand, AMPA receptor modulators, namely CX516, CX546, and CTZ reduced power by 36 ± 7 % (n=7), 65 ± 7 % (n=6), and 67 ± 4 % (n=6), respectively. All these changes in power were statistically significant ($p<0.01$, Two-factor ANOVA with replication).

The changes in frequency were different within benzodiazepines. FZP increased frequency by 5.4 ± 2.5 % ($p<0.01$, ANOVA, n=6) but CDP decreased it by 5.0 ± 2.4 % ($p<0.01$, ANOVA, n=6). TZL did not change frequency ($p>0.05$, ANOVA, n=6). On the other hand, AMPA receptor modulators increased frequency. CX516, CXC546 and CTZ increased frequency by 4.0 ± 2.3 % ($p<0.05$, ANOVA, n=7), 11 ± 2.5 % ($p<0.01$, ANOVA, n=6) and 13 ± 1.8 % ($p<0.01$, ANOVA, n=6), respectively.

Fig. 26 demonstrates the percent changes in frequency and power between the last five minutes (10 samples) of baseline and each drug infusion. Since both power and frequency are useful to differentiate drugs between and within classes, these changes were plotted in a two dimensional space (power and frequency axes) in Figure 27. In this plot, benzodiazepines (grey) are away from AMPA receptor modulators (black). Further, two of the benzodiazepines (CDP and TZL) are similar to each other compared to FZP (opposite directions in frequency change). Two AMPA receptor modulators (CX546 and CTZ) are also similar to each other compared to CX516; however, these differences might be due to the degree of occupancy of the receptors. Dose response experiments are necessary to further assess these differences.

Fig. 27 is a two dimensional plot of changes in frequency and power between the last five minutes (10 samples) of baseline and each drug infusion.

EXAMPLE 18

<u>5-HT1A Receptor Agonist Assay Using Low Frequency Bursting Activity and Beta Oscillation in Rat Hippocampus Slice</u>

Figure 28 is an example trace of extracellular field recordings from stratum pyramidale of the CA3 area which demonstrates CCh (5 mM) induced beta oscillations (28A) as well as lower frequency bursting activity (0.1-0.2 Hz, expanded trace in Figure 28B).

This low frequency bursting activity was reduced reversibly by infusing 5-HT (3 and 10 mM) and fluoxetine (10 mM) (Figure 29). This effect was found to be mediated by 5-HT1A receptor activation, since the selective 5-HT1A receptor antagonist N-(2-(4-(2-methoxyphenyl)-1-piperazinyl) ethyl)-N-(2-pyridinyl)cyclohexane carboxamide (WAY 100635, 1mM) reversed the suppression of bursting activity by 5-HT(10 mM) (data not shown).

In Figure 29 it is shown that 5-HT and fluoxetine decrease the frequency of CCh-induced network bursting activity. The upper trace in Figure 29A shows the effects of 5-HT; the lower trace shows the effects of fluoxetine. The bar graph in Figure 29B, illustrates pooled data.

Figure 30 demonstrates that beta oscillations induced by CCh are also sensitive to 5-HT. 5-HT decreases the power of CCh-induced β oscillations. In Figure 30A, example traces of extracellular field recordings from stratum pyramidale of the CA3 area illustrate the effects of 5-HT. The bar graph in Figure 30B illustrates pooled data.

EXAMPLE 19

<u>SSRI and Cholinesterase Inhibitor Assay Using Co-Culture Sample</u>

Drugs affecting modulatory projections such as cholinergic and serotonergic projections are difficult to measure. Utilizing co-cultures of hippocampus with raphe nucleus or septum attached provides naturalistic connections between them and enables testing compounds acting on cholinesterase inhibitor systems and selective serotonin reuptake systems.

<u>Method</u>

<u>Preparation of the MED probes.</u> Before use, the MED probe (Panasonic; MED-P545AP, each electrode: 50 x 50 μm, interpolar distance: 450 μm) was soaked in 70% ethanol for 15 min, dried up in a clean bench and sterilized with UV radiation for 15 min. The surface of the probe was treated with 0.1% polyethylenimine and 25 mM borate buffer, pH 8.4, overnight at room temperature. The probe surface was dried and rinsed 3 times with sterile distilled water. Finally, the probe was filled with culture medium and stored in a CO2 incubator until use (for at least one hour). The culture medium was a 2:1 mixture of Basal Medium Eagle (Sigma; B9638) and Earle Balanced Salts Solution (Sigma; E7510), supplemented with the following compounds (in mM): NaCl (20), NaHCO3 (5), CaCl2 (0.2), MgSO4 (1.7), glucose (48), HEPES (26.7); 5% horse serum (GIBCO; 26050) and 10 ml/l penicillin-streptomycin (GIBCO;10378) were added and the pH adjusted to 7.2.

Dissection of brain slice. All procedures for culture preparation were carried out under a sterilized bench. Sprague-Dawley rats were sterilized with 70% ethanol, sacrificed by decapitation following anesthesia and the whole brain removed. The brains were immediately soaked in sterile ice-cold MEM (pH 7.2; GIBCO; 61100), supplemented with HEPES (25 mM), Tris-base (10 mM), glucose (10 mM) and $MgCl_2$ (3 mM). Appropriate portions of the brain were trimmed by hand and the remaining brain block was placed on the ice-cold stage of a vibrating tissue slicer (Leica; VT1000S). Septal and raphe portions were taken from five or six day-old rats and the hippocampal portion was taken from eleven day-old rats. The thickness of the slices was set at 200 μm. The slices were gently taken off the blade with a pipette. Each slice was trimmed appropriately.

Culture on membrane. One hippocampal slice and one septal or raphe slice were placed on the center of a porous membrane (Millipore, Bedford, MA), which was previously coated as mentioned above. After positioning the section on the membrane, the cutting solution was removed and culture medium was added underneath the membrane (approximately 1 ml). The slices on the membrane were stored in a CO2 incubator at 34° C. The medium was exchanged with all volume every other day.

For electrophysiological recordings, the slices were picked up by cutting membrane, filliped on MED probes and positioned to cover the 8 x 8 microelectrode array. Culture medium was then added to an interface level (approximately 250 ml), and the slices on MED probes were stored in $CO_2$ incubator at 34 °C for 1 hour. After incubation, the slices on the probes were removed from the incubator and placed in a smaller $CO_2$ incubator at 34 °C and connected to the recording component of MED-64. The medium was replaced with sterile artificial cerebrospinal fluid (ACSF) of the following composition (in mM): NaCl (124), NaHCO3 (26), glucose (10), KCl (3), NaH2PO4 (1.25), CaCl2 (2), MgSO4 (1), and HEPES (10). Spontaneous field potentials at all 64 sites were recorded simultaneously with the multi-channel recording system (Panasonic; MED64 system) at a 20 kHz sampling rate.

Culture on MED probe. One hippocampal slice and one septal or raphe slice were placed on the center of the MED probe, which was previously coated as mentioned above, and positioned next each other on the 8x8 microelectrode array. After positioning the section on the MED probe, the cutting solution was removed and culture medium was added to the slice up to an interface level (approximately 250 μl). Sterile distilled water was added around the probe to increase humidity and prevent over-drying of the culture medium in the MED probe. The slices on the MED probes were stored in a $CO_2$ incubator at 34° C. The medium was exchanged with half volume every day.

For electrophysiological recordings, the MED probes containing the slices were removed from the incubator and placed in a smaller $CO_2$ incubator at 34 °C and connected to the stimulation/recording component of MED-64. The medium was replaced with sterile artificial cerebrospinal fluid (ACSF) of the following composition (in mM): NaCl (124), NaHCO3 (26), glucose (10), KCl (3), NaH2PO4 (1.25), CaCl2 (2), MgSO4 (1), and HEPES (10). Spontaneous field potentials at all 64 sites were recorded simultaneously with the multi-channel recording system (Panasonic; MED64 system) at a 20 kHz sampling rate.

Results

Figure 31 is a graphic representation of the results from the addition of a cholinesterase inhibitor to a septo-hippocampal co-culture grown directly on the MED probe. Specifically, Figure 31 is a two-dimensional plot of changes in frequency and power between the control and cholinesterase inhibitor (physostigmine, 10 μm) in septo-hippocampus co-culture. The amplitude and frequency were the results of the Fast Fourier Transform on each respective set of data. As seen from the figure, physostigmine increased the power of spontaneous rhythmic activity but did not affect the frequency.

Figure 32 shows the effect of physostigmine (10 μm) in number of spikes in the same septo-hippocampus co-culture. Atropine (1 μM) completely reversed the effect of physotigmine.

The effect of SSRIs on raphe-hippocampal preparations are shown in Figure 33 (decrease in power, slight decrease in frequency). Figure 33 is a two-dimensional plot of changes in frequency and power between the control and SSRI (fluoxetine) in a raphe-hippocampal co-culture. The figure shows the relative differences in spontaneous rhythmic activities caused by fluoxetine (2μM) to raphe-hippocampal co-culture grown on the membrane, transferred and measured on MED probe. The amplitude and frequency were the results of the Fast Fourier Transform on each respective set of data.

REFERENCES

Albuquerque, E. X., Pereira, E. F., Alkondon, M., Schrattenholz, A. & Maelicke, A. (1997) Nicotinic acetylcholine receptors on hippocampal neurons: distribution on the neuronal surface and modulation of receptor activity. J Recept Signal Transduct Res 17,243-266.

Apostol, G. & Creutzfeldt, O. D. (1974) Crosscorrelation between the activity of septal units and hippocampal EEG during arousal. Brain Res 67, 65-75.

Arai, A., Silberg, J. & Lynch, G. (1995) Differences in the refractory properties of two distinct inhibitory circuitries in field CA1 of the hippocampus. Brain Res 704, 298-306.

Assaf, S. Y. & Miller, J. J. (1978) The role of a raphe serotonin system in the control of septal unit activity and hippocampal desynchronization. Neuroscience 3, 539-550. Auerbach, J. M. & Segal, M. (1994) A novel cholinergic induction of long-term potentiation in rat hippocampus. J Neurophysiol 72, 2034-2040.

Behrends JC, Bruggencate G (1993) Cholinergic modulation of synaptic inhibition in the guinea pig hippocampus *in vitro:* excitation of GABAergic interneurons and inhibition of GABA-release. J Neurophysiol 69:626-629.

Benson DM, Blitzer RD, Landau EM (1988) An analysis of the depolarization produced in guinea pig hippocampus by cholinergic receptor stimulation. J Physiol (London) 404:479-496.

Bliss, T. V. & Lomo, T. (1973) Long-lasting potentiation of synaptic transmission in the dentate area of the anaesthetized rabbit following stimulation of the perforant path. J Physiol 232, 331-356.

Blitzer, R. D., Gil, O. & Landau, E. M. (1990) Cholinergic stimulation enhances long-term potentiation in the CA1 region of rat hippocampus. Neurosci Lett 119, 207-210.

Boddeke HWGM, Best R, Boeijinga PH (1997) Synchronous 20Hz rhythmic activity in hippocampal networks induced by activation of metabotropic glutamate receptors *in vitro.* Neuroscience 76:653-658.

Bressler, S. L., Coppola, R. & Nakamura, R. (1993) Episodic multiregional cortical coherence at multiple frequencies during visual task performance. Nature 366, 153-156.

Brussaard, A. B., Kits, K. S., Baker, R. E., Willems, W. P., Leyting-Vermeulen, J. W., Voom, P., Smit, A. B., Bicknell, R. J. & Herbison, A. E. (1997) Plasticity in fast synaptic inhibition of adult oxytocin neurons caused by switch in GABA(A) receptor subunit expression. Neuron 19,1103-1114.

Buzsaki G. (1986). Hippocampal sharp waves: origin and significance. Brain Res 398: 242-252.

Charpak S, Parae D, Llinas R (1995). The entorhinal cortex entrains fast CA1 monosynaptic component of the perforant path. Eur J Neurosci 7: 1548-1557. Chrobak JJ, Buzsaki G (1998). Gamma oscillations in the entorhinal cortex of the freely behaving rat. J Neurosci, 18: 388-398.

Dickson CT, Alonso A (1997) Muscarinic induction of synchronous population activity in the entorhinal cortex J Neurosci 17:6729-6744.

Fell, J., Klaver, P., Lehnertz, K., Grunwald, T., Schaller, C., Elger, C. E. & Fernandez, G. (2001) Human memory formation is accompanied by rhinalhippocampal coupling and decoupling. Nat Neurosci 4, 1259-1264.

Fellous, J. M. & Sejnowski, T. J. (2000) Cholinergic induction of oscillations in the hippocampal slice in the slow (0.5-2 Hz), theta (5-12 Hz), and gamma (35-70 Hz) bands. Hippocampus 10, 187-197.

Fisahn A, Pike FG, Buhl EH, Paulsen O (1998) Cholinergic induction of network oscillations at 40Hz in the hippocampus *in vitro.* Nature 394:186-189

Freeman WJ (1975) Mass action in the nervous system. New York: Academic Press. Frotscher M, Leranth C (1985) Cholinergic innervation of the rat hippocampus as revealed by choline acetyltransferase immunocytochemistry: a combined light and electron microscopic study. J Comp Neurol 239:237-246

Funahashi M, Stewart M (1998) Properties of gamma-frequency oscillations initiated by propagating population bursts in retrohippocampal region of rat brain slices. J Physiol (Lond) 510:191-208.

Gogolak, G., Stumpf, C., Petsche, H. & Sterc, J. (1968) The firing pattern of septal neurons and the form of the hippocampal theta wave. Brain Res 7, 201-207.

Gray, C. M., Konig, P., Engel, A. K. & Singer, W. (1989) Oscillatory responses in cat visual cortex exhibit intercolumnar synchronization which reflects global stimulus properties. Nature 338, 334-337.

Gray CM, Singer W (1989) Stimulus-specific neuronal oscillation columns of cat visual cortex. Proc Natl Acad Sci USA 56:1698-1702

Haberly L, Shepherd G (1973). Current density analysis of summed evoked potentials in opossum prepyriform cortex. J Neurophysiol, 36: 789-803.

Hajos N, Papp ECS, Acsady L, Levy A, Freund TF (1998) Distinct interneuron types express M2 muscarinic receptor immunoreactivity on their dendrites or axon terminals in the hippocampus. Neuroscience 82:355-376

Howland B, Lettvin J, McCulloch W, Pitts W, Wall P (1955). Reflex inhibition by dorsal root interaction. J Neurophysiol, 18: 1-17.

Horowitz JM, Freeman WJ, Stoll PJ (1973) A neural network with a background level of excitation in the cat hippocampus. Int J Neurosci 5:113-123.

Huerta Pt, Lisman JE. (1993) Heightened synaptic plasticity of hippocampal CA1 neurons during cholinergically induced rhythmic state. Nature 364:723-725.

Iijima, T., Witter, M. P., Ichikawa, M., Tominaga, T., Kajiwara, R. & Matsumoto, G. (1996) Entorhinal-hippocampal interactions revealed by real-time imaging. Science 272:1176-1179.

Kay LM, Freeman WJ (1998) Bidirectional processing in the olfactory-limbic axis during olfactory behavior. Behav Neurosci 112:514-553

Ketchum KL, Haberly LB (1993) Membrane currents evoked by afferent fiber stimulation in rat piriform cortex. I. Current source density analysis. J. Neurophysiol 69: 248-260.

Kolta A, Ambros-Ingerson J, Lynch G (1996) Early and late components of AMPA-receptor mediated field potentials in hippocampal slices. Brain Res. 737: 133-145. Komatsu, Y. (1994) Age-dependent long-term potentiation of inhibitory synaptic transmission in rat visual cortex. J Neurosci 14, 6488-6499.

Konopacki J, MacIver MB, Bland BH, Roth SH (1987) Carbachol-induced EEG 'theta' activity in hippocampal brain slice. Brain Res 405:196-198.

Korn, H., Oda, Y. & Faber, D. S. (1992) Long-term potentiation of inhibitory circuits and synapses in the central nervous system. Proc Natl Acad Sci U S A 89, 440-443. Landfield PW, McGaugh JL, Tusa RJ (1972) Theta rhythm: a temporal correlate of memory storage processes in the rat. Science 175:87-89.

Landfield PW, McGaugh JL., Lynch G. (1978) Impaired synaptic potentiation processes in the hippocampus of aged, memory-deficient rats. Brain Res 150, 85-101.

Larson J, Wong D, Lynch G. (1986). Patterned stimulation at the theta frequency is optimal for the induction of hippocampal long-term potentiation. Brain Res., 368: 347-350.

Larson J, Lynch G. (1986). Induction of synaptic potentiation in hippocampus by patterned stimulation involves two events. Science 232: 985-988.

Larson J, Xiao P, Lynch G (1993) Reversal of LTP by theta frequency stimulation. Brain Res 600:97-102

Leung LS (1982) Nonlinear feedback model of neuronal populations in hippocampal CA1 region. J Neruophysiol 47:845-868.

Leung LS (1985) Spectral analysis of hippocampal EEG in the freely moving rat: effects of centrally active drugs and relations to evoked potentials. Electroencephalogr Clin Neurophysiol 60:65-77.

Levey AI, Edmunds SM, Koliatsos V, Wiley RG, Helman CJ (1995) Expression of m1-m4 muscarinic acetylcholine receptor proteins in rat hippocampus and regulation by cholinergic innervation. J Neurosci 15:4077-4092.

Lewis PR, Shute CCD (1967) The cholinergic limbic system: projections to hippocampal formation, medial cortex, nuclei of the ascending cholinergic reticular system, and the subfornical organ and supra-optic crest. Brain 90:521-40.

Lynch G, Rose G, Gall C (1978) Anatomical and functional aspects of the septo-hippocampal projections. In: Functions of the septo-hippocampal system (Ciba foundation symposium 58), pp5-24. Amsterdam: Elsevier.

Macadar, O., Roig, J. A., Monti, J. M. & Budelli, R. (1970) The functional relationship between septal and hippocampal unit activity and hippocampal theta rhythm. Physiol Behav 5,1443-1449.

MacVicar BA, Tse FWY (1989) Local neuronal circuitry underlying cholinergic rhythmical slow activity in CA3 area of rat hippocampus. J Physiol (London) 417:197-212

Madison DV, Lancaster B, Nicoll RA (1987) Voltage clamp analysis of cholinergic action in the hippocampus. J Neurosci 7:733-741

Matthews DA, Salvaterra PM, Crawford GD, Houser CR, Vaughn JE (1987) An immunocytochemical study of choline acethltransferase-containing neurons and axon terminals in normal and partially deafferented hippocampal formation. Brain Res 402:30-43

Mitzdorf U (1985). Current source-density method and application in cat cerebral cortex: Investigation of evoked potentials and EEG phenomena. Physiol Rev, 65: 37-100.

Morales, F. R., Roig, J. A., Monti, J. M., Macadar, O. & Budelli, R. (1971) Septal unit activity and hippocampal EEG during the sleep-wakefulness cycle of the rat. Physiol Behav 6, 563-567.

Mosko S, Lynch G, Cotman CW (1973) Distribution of the septal projections to the hippocampus of the rat. J Comp Neurol 152:163-174

Mott DD, Lewis DV. (1991). Facilitation of the induction of long-term potentiation by GABAB
receptors. Science, 252: 1718-1720.

Nakajima Y, Nakajima S, Leonard RJ, Yamaguchi K (1986) Acetylcholine raises excitability by inhibiting the fast transient potassium current in cultured hippocampal neurons. Proc Natl Acad Sci USA 83:3022-3026

Nicholson C (1973). Theoretical analysis of field potentials in anisotropic ensembles of neuronal elements. IEEE Trans Biomed Eng, 20: 278-288.

Nicholson C, Freeman J (1975). Theory of current source-density analysis and determination of conductivity tensor for anuran cerebellum. J Neurophysiol, 38: 356-368.

Nicholson C, Llinas R (1975). Real time current source density analysis using multi-electrode array in cat cerebellum. Brain Res, 100: 418-424.

Nusser, Z., Hajos, N., Somogyi, P. & Mody, I. (1998) Increased number of synaptic GABA(A) receptors underlies potentiation at hippocampal inhibitory synapses. Nature 395, 172-177.

Oka H, Shimono K, Ogawa R, Sugihara H, Taketani M (1999) A new planar multielectrode array for extracellular recording: application to hippocampal acute slice. J Neurosci Methods 93:61-67.
Otis, T. S., De Koninck, Y. & Mody, I. (1994) Lasting potentiation of inhibition is associated with an increased number of gamma-aminobutyric acid type A receptors activated during miniature inhibitory postsynaptic currents. Proc Natl Acad Sci U S A 91, 7698-7702.
Petsche, H., Stumpf, C. & Gogolak, G. (1962) The significance of the rabbit's septum as a relay station between the midbrain and the hippocampus. I. The control of hippocampal arousal activity by the septum cells. Electroencephalogr Clin Neurophysiol 14,202-211.
Pitler TA, Alger BE (1992) Cholinergic excitation of GABAergic interneurons in the rat hippocampal slice. J Physiol (Lond). 450:127-142
Racine R, Livingston K, Joaquin A(1975) Effects of procaine hydrochloride, diazepam, and diphenylhydantoin on seizure development n cortical and subcortical structures in rats. Electroenceph Clin Neurophysiol 38:355-365
Sarter M; Bruno JP. (1998). Age-related changes in rodent cortical acetylcholine and cognition: main effects of age versus age as an intervening variable. Brain Res., 27:143-156.
Shimono, K., Brucher, F., Granger, R., Lynch, G. & Taketani, M. (2000) Origins and distribution of cholinergically induced beta rhythms in hippocampal slices. J Neurosci 20, 8462-8473.
Singer W (1998) Consciousness and structure of neuronal representations. Philos Trans R Soc Lond B Biol Sci 353:1829-1840.
Squire, L. R. & Zola-Morgan, S. (1991) The medial temporal lobe memory system. Science 253, 1380-1386.
Staubli, U. & Lynch, G. (1987) Stable hippocampal long-term potentiation elicited by 'theta' pattern stimulation. Brain Res 435, 227-234.
Stumpf C (1965) Drug action on the electrical activity of the hippocampus. Int Rev Neurosci 8:77-138.
Traub RD, Spruston N, Soltesz I, Konnerth A. (1998). Gamma-frequency oscillations: A neuronal population phenomenon, regulated by synaptic and intrinsic cellular processes, and inducing synaptic plasticity. Prog. Neurobiol., 55: 563-575. Traub RD, Whittington MA, Buhl EH, Jefferys JGR, Faulkner HJ. (1999). On the mechanism of the gamma -> beta frequency shift in neuronal oscillations induced in rat hippocampal slices by tetanic stimulation. J. Neurosci., 19: 1088-1105.
Traub RD, Whittington MA, Colling SB, Buzsaki GB, Jefferys GR (1995) Analysis of the gamma rhythm in the rat hippocampus *in vitro* and in vivo. J Physiol (London) 493:471-484
Vanderwolf CH (1969) Hippocampal electrical activity and voluntary movement in the rat. Electroenceph Clin Neurophysiol 26:407-418.
Van der Zee, E. A., Strosberg, A. D., Bohus, B. & Luiten, P. G. (1993) Colocalization of muscarinic acetylcholine receptors and protein kinase C gamma in rat parietal cortex. Brain Res Mol Brain Res 18,152-162.
Vertes RP, Kocsis B (1997) Brainstem-dienchephalo-septhippocampal systems controlling the theta rhythm of the hippocampus. Neuroscience 81:893-926.
Williams JH, Kauer JA (1997) Properties of carbachol-induced oscillatory activity in rat hippocampus. J Neurophysiol 78:2631-2640.
Winson, J. (1978) Loss of hippocampal theta rhythm results in spatial memory deficit in the rat. Science 201,160-163.
Witter, M. P., Wouterlood, F. G., Naber, P. A. & Van Haeften, T. (2000) Anatomical organization of the parahippocampal-hippocampal network. Ann N Y Acad Sci 911, 1-24.
Woolf NJ, Eckenstein F, Butcher LL. (1984) Cholinergic Systems in the rat brain: I. projections to the limibic telencephalon. Brain Res Bull 13, 751-784.

## Claims

1. A process for the characterization of psychoactive compounds in a candidate sample composition in a neuronal tissue sample comprising:

   a) inducing a set of baseline oscillations by contacting the neuronal tissue sample with an inducing agent and maintaining the set of baseline oscillations after washout of the inducing agent.
   b) measuring a combination of parameters from the set of baseline oscillations and from a set of resulting oscillations generated after contact of the neuronal tissue sample with the candidate sample composition;
   c) comparing said combination of parameters from the set of baseline oscillations and resulting oscillations; and
   d) characterizing said candidate sample composition based upon the differences between said combination of parameters from the set of baseline oscillations and resulting oscillations.

2. The process of claim 1 wherein the step of characterizing identifies a compound class of which said psychoactive compound is a member.

**3.** The process of claim 2 further comprising the step of comparing relative differences between said identified compound class and one or more different psychoactive compound classes.

**4.** The process of claim 2 further comprising the step of distinguishing said psychoactive compound from other members of the compound class.

**5.** The process of claim 1 wherein said combination of parameters comprises frequency and power.

**6.** The process of claim 1 wherein said psychoactive compound is selected from the group consisting of AMPA antagonists, AMPA receptor modulators, antianxiety drugs, anticonvulsants, antidepressant drugs, antipsychotic drugs, benzodiazepines, central nervous system stimulants, dopaminergic agents, GABA antagonists, hypnotic drugs, and narcotic analgesics.

**7.** The process of claim 6 wherein said psychoactive compound comprises an AMPA antagonist.

**8.** The process of claim 7 wherein said AMPA antagonist is selected from the group consisting of CNQX, DNQX, GYKI 52466HCl, Joro spider toxin, 1-Naphthyl-acetyl spermine, NS257, and NBQX.

**9.** The process of claim 6 wherein said psychoactive compound comprises an AMPA receptor modulator.

**10.** The process of claim 9 wherein said AMPA receptor modulator comprises an ampakine.

**11.** The process of claim 10 wherein said AMPA receptor modulator is selected from the group consisting of CX516, CX546, CX554, CX614, and CX691.

**12.** The process of claim 6 wherein said psychoactive compound comprises a benzodiazepine.

**13.** The process of claim 12 wherein said benzodiazepine is selected from the group consisting of alprazolam, bromazepam, chlordiazepoxide, clorazepate, clotiazepam, diazepam, estazolam, etizolam, fludiazepam, flumazenil, flunitrazepam, flurazepam, flutoprazepam, hloxazolam, lorazepam, medazepam, nimetazepam, nitrazepam, oxazepam, oxazolam, rimazafone, temazepam, and trizolam.

**14.** The process of claim 12 wherein said benzodiazepine is selected from the group consisting of diazepam, chlordiazepoxide, flurazepam, and triazolam.

**15.** The process of claim 6 wherein said psychoactive compound comprises a GABA antagonist.

**16.** The process of claim 15 wherein said GABA antagonist is selected from the group consisting of bicuculline, β-hydrastine, picrotoxin, and SR-95531 (Gabazine).

**17.** The process of claim 16 wherein said GABA antagonist comprises bicuculline.

**18.** The process of claim 16 wherein said GABA antagonist comprises picrotoxin.

**19.** The process of claim 1 wherein the inducing agent for producing the set of baseline oscillations is a chemical compound.

**20.** The process of claim 19 wherein said chemical compound is selected from the group consisting of cholinergic agonists, cholinesterase inhibitors, and sympathetic agonists.

**21.** The process of claim 20 wherein said chemical compound comprises a cholinergic agonist.

**22.** The process of claim 21 wherein said cholinergic agonist is selected from the group consisting of acetylcholine, arechol, carbachol, methacholine, bethanechol, muscarine, and pilocarpine.

**23.** The process of claim 22 wherein said cholinergic agonist comprises carbachol.

24. The process of claim 20 wherein said chemical compound comprises a cholinesterase inhibitor.

25. The process of claim 24 wherein said cholinesterase inhibitor is selected from the group consisting of ambenonium, demecarium, diidopropyl-fluorophosphate, echothiophate, edrophonium, huperzine and huperzine analogs, neostigmine, physotigmine, and pyridostigmine.

26. The process of claim 25 wherein said cholinesterase inhibitor comprises physostigmine.

27. The process of claim 19 wherein said chemical compound selectively binds a muscarinic M1 receptor.

28. The process of claim 1 wherein the inducing agent is an electrical stimulation.

**Patentansprüche**

1. Verfahren zur Charakterisierung psychoaktiver Verbindungen in einer Probekandidaten-Zusammensetzung in einer neuronalen Gewebeprobe umfassend:

   (a) Induzieren eines Satzes von Grundlinienschwingungen durch Inkontaktbringen der neuronalen Gewebeprobe mit einem induzierenden Agens und Aufrechterhalten des Satzes an Grundlinienschwingungen nach dem Entfernen des induzierenden Agens;
   (b) Messen einer Kombination von Parametern von dem Satz der Grundlinienschwingungen und von einem Satz von resultierenden Schwingungen, die nach dem Kontakt der neuronalen Gewebeprobe mit der Probekandidaten-Zusammensetzung erzeugt wurden;
   (c) Vergleichen der Kombination von Parametern von dem Satz der Grundlinienschwingungen und resultierenden Schwingungen; und
   (d) Charakterisieren der Probekandidaten-Zusammensetzung basierend auf den Unterschieden zwischen der Kombination von Parametern von dem Satz der Grundlinienschwingungen und den resultierenden Schwingungen.

2. Verfahren nach Anspruch 1, wobei der Schritt des Charakterisierens eine Verbindungsklasse identifiziert, von der die psychoaktive Verbindung ein Mitglied ist.

3. Verfahren nach Anspruch 2, weiter umfassend den Schritt des Vergleichens der relativen Unterschiede zwischen der identifizierten Verbindungsklasse und einer oder mehreren unterschiedlichen psychoaktiven Verbindungsklassen.

4. Verfahren nach Anspruch 2, weiter umfassend den Schritt des Hervorhebens der psychoaktiven Verbindung von anderen Mitgliedern der Verbindungsklasse.

5. Verfahren nach Anspruch 1, wobei die Kombination der Parameter Frequenz und Leistung umfasst.

6. Verfahren nach Anspruch 1, wobei die psychoaktive Verbindung ausgewählt ist aus der Gruppe bestehend aus AMPA-Antagonisten, AMPA-Rezeptormodulatoren, Anxiolytika, Antikonvulsiva, Antidepressiva, Antipsychotika, Benzodiazepinen, Zentralnervensystem-Stimulanzien, dopaminergen Agenzien, GABA-Antagonisten, Hypnotika und narkotisierenden Analgetika.

7. Verfahren nach Anspruch 6, wobei die psychoaktive Verbindung einen AMPA-Antagonist umfasst.

8. Verfahren nach Anspruch 7, wobei der AMPA-Antagonist ausgewählt ist aus der Gruppe bestehend aus CNQX, DNQX, GYKI 52466HCl, Joro-Spinne-Toxin, 1-Naphthyl-acetylspermin, NS257 und NBQX.

9. Verfahren nach Anspruch 6, wobei die psychoaktive Verbindung einen AMPA-Rezeptormodulator umfasst.

10. Verfahren nach Anspruch 9, wobei der AMPA-Rezeptormodulator ein Ampakin umfasst.

11. Verfahren nach Anspruch 10, wobei der AMPA-Rezeptormodulator ausgewählt ist aus der Gruppe bestehend aus CX516, CX546, CX554, CX614 and CX691.

**12.** Verfahren nach Anspruch 6, wobei die psychoaktive Verbindung ein Benzodiazepin umfasst.

**13.** Verfahren nach Anspruch 12, wobei das Benzodiazepin ausgewählt ist aus der Gruppe bestehend aus Alprazolam, Bromazepam, Chlordiazepoxid, Clorazepat, Clotiazepam, Diazepam, Estazolam, Etizolam, Fludiazepam, Flumazenil, Flunitrazepam, Flurazepam, Flutoprazepam, Haloxazolam, Lorazepam, Medazepam, Nimetazepam, Nitrazepam, Oxazepam, Oxazolam, Rimazafon, Temazepam und Trizolam.

**14.** Verfahren nach Anspruch 12, wobei das Benzodiazepin ausgewählt ist aus der Gruppe bestehend aus Diazepam, Chlordiazepoxid, Flurazepam und Triazolam.

**15.** Verfahren nach Anspruch 6, wobei die psychoaktive Verbindung einen GABA-Antagonisten umfasst.

**16.** Verfahren nach Anspruch 15, wobei der GABA-Antagonist ausgewählt ist aus der Gruppe bestehend aus Bicucullin, β-Hydrastin, Picrotoxin und SR-95531 (Gabazin).

**17.** Verfahren nach Anspruch 16, wobei der GABA-Antagonist Bicucullin umfasst.

**18.** Verfahren nach Anspruch 16, wobei der GABA-Antagonist Picrotoxin umfasst.

**19.** Verfahren nach Anspruch 1, wobei das induzierende Agens zur Erzeugung des Satzes von Grundlinienschwingungen eine chemischen Verbindung ist.

**20.** Verfahren nach Anspruch 19, wobei die chemische Verbindung ausgewählt ist aus der Gruppe bestehend aus cholinergischen Agonisten, Cholinesterase-Inhibitoren und sympathetischen Agonisten.

**21.** Verfahren nach Anspruch 20, wobei die chemische Verbindung einen cholinergischen Agonisten umfasst.

**22.** Verfahren nach Anspruch 21, wobei der cholinergische Agonist ausgewählt ist aus der Gruppe bestehend aus Acetylcholin, Arechol, Carbachol, Methacholin, Bethanechol, Muscarin und Pilocarpin.

**23.** Verfahren nach Anspruch 22, wobei der cholinergische Agonist Carbachol umfasst.

**24.** Verfahren nach Anspruch 20, wobei die chemischen Verbindung einen Cholinesterase-Inhibitor umfasst.

**25.** Verfahren nach Anspruch 24, wobei der Cholinesterase-Inhibitor ausgewählt ist aus der Gruppe bestehend aus Ambenonium, Demecarium, Diidopropyl-Fluorophosphat, Echothiophat, Edrophonium, Huperzin und Huperzin-Analoga, Neostigmin, Physostigmin und Pyridostigmin.

**26.** Verfahren nach Anspruch 25, wobei der Cholinesterase-Inhibitor Physostigmin umfasst.

**27.** Verfahren nach Anspruch 19, wobei die chemische Verbindung einen muscarinischen M1-Rezeptor selektiv bindet.

**28.** Verfahren nach Anspruch 1, wobei das induzierende Agens eine elektrische Stimulation ist.

## Revendications

**1.** Procédé de caractérisation de composés psycho-actifs dans une composition d'échantillon candidat dans un échantillon de tissu neuronal comprenant :

a) induction d'un ensemble d'oscillations de ligne de base par mise en contact de l'échantillon de tissu neuronal avec un agent d'induction et maintien de l'ensemble d'oscillations de ligne de base après élimination par lavage de l'agent d'induction ;
b) mesure d'une combinaison de paramètres de l'ensemble d'oscillations de ligne de base et d'un ensemble d'oscillations résultantes générées après mise en contact de l'échantillon de tissu neuronal avec la composition d'échantillon candidat ;
c) comparaison de ladite combinaison de paramètres de l'ensemble d'oscillations de ligne de base et des oscillations résultantes ; et

d) caractérisation de ladite composition d'échantillon candidat sur la base des différences entre ladite combinaison de paramètres de l'ensemble d'oscillations de ligne de base et des oscillations résultantes.

**2.** Procédé selon la revendication 1 dans lequel l'étape de caractérisation identifie une classe de composés dont ledit composé psycho-actif est un membre.

**3.** Procédé selon la revendication 2 comprenant de plus l'étape de comparaison de différences relatives entre ladite classe de composés identifiée et une ou plusieurs classes de composés psycho-actifs différentes.

**4.** Procédé selon la revendication 2 comprenant de plus l'étape de distinction dudit composé psycho-actif par rapport à d'autres membres de la classe de composés.

**5.** Procédé selon la revendication 1 dans lequel ladite combinaison de paramètres comprend fréquence et puissance.

**6.** Procédé selon la revendication 1 dans lequel ledit composé psycho-actif est choisi dans le groupe consistant en des antagonistes AMPA, modulateurs de récepteur AMPA, médicaments anti-anxiété, anticonvulsifs, médicaments antidépresseurs, médicaments antipsychotiques, benzodiazépines, stimulants du système nerveux central, agents dopaminergiques, antagonistes GABA, médicaments hypnotiques, et analgésiques narcotiques.

**7.** Procédé selon la revendication 6 dans lequel ledit composé psycho-actif comprend un antagoniste AMPA.

**8.** Procédé selon la revendication 7 dans lequel ledit antagoniste AMPA est choisi dans le groupe consistant en CNQX, DNQX, GYKI 52466 HCl, toxine d'araignée Joro, 1-naphtyl acétyle spermine, NS257, et NBQX.

**9.** Procédé selon la revendication 6 dans lequel ledit composé psycho-actif comprend un modulateur de récepteur AMPA.

**10.** Procédé selon la revendication 9 dans lequel ledit modulateur de récepteur AMPA comprend une ampakine.

**11.** Procédé selon la revendication 10 dans lequel ledit modulateur de récepteur AMPA est choisi dans le groupe consistant en CX516, CX546, CX554, CX614, et CX691.

**12.** Procédé selon la revendication 6 dans lequel ledit composé psycho-actif comprend une benzodiazépine.

**13.** Procédé selon la revendication 12 dans lequel ladite benzodiazépine est choisie dans le groupe consistant en alprazolam, bromazépam, chlordiazépoxide, clorazépate, clotiazépam, diazépam, estalozam, étizolam, fludiazépam, flumazénil, flunitrazépam, flurazépam, flutoprazépam, cloxazolam, lorazépam, médazépam, nimétazépam, nitrazépam, oxazépam, oxazolam, rimazafone, témazépam, et trizolam.

**14.** Procédé selon la revendication 12 dans lequel ladite benzodiazépine est choisie dans le groupe consistant en diazépam, chlordiazépoxide, flurazépam, et triazolam.

**15.** Procédé selon la revendication 6 dans lequel ledit composé psycho-actif comprend un antagoniste GABA.

**16.** Procédé selon la revendication 15 dans lequel ledit antagoniste GABA est choisi dans le groupe consistant en bicuculline, β-hydrastine, picrotoxine, et SR-95531 (Gabazine).

**17.** Procédé selon la revendication 16 dans lequel ledit antagoniste GABA comprend de la bicuculline.

**18.** Procédé selon la revendication 16 dans lequel ledit antagoniste GABA comprend de la picrotoxine.

**19.** Procédé selon la revendication 1 dans lequel l'agent d'induction pour production de l'ensemble d'oscillations de ligne de base est un composé chimique.

**20.** Procédé selon la revendication 19 dans lequel ledit composé chimique est choisi dans le groupe consistant en des agonistes cholinergiques, inhibiteurs de cholinestérase, et agonistes sympathiques.

**21.** Procédé selon la revendication 20 dans lequel ledit composé chimique comprend un agoniste cholinergique.

**22.** Procédé selon la revendication 21 dans lequel ledit agoniste cholinergique est choisi dans le groupe consistant en acétylcholine, aréchol, carbachol, méthacholine, béthanéchol, muscarine, et pilocarpine.

**23.** Procédé selon la revendication 22 dans lequel ledit agoniste cholinergique comprend du carbachol.

**24.** Procédé selon la revendication 20 dans lequel ledit composé chimique comprend un inhibiteur de cholinestérase.

**25.** Procédé selon la revendication 24 dans lequel ledit inhibiteur de cholinestérase est choisi dans le groupe consistant en ambénonium, démécarium, diidopropyl-fluorophosphate, échotiophate, édrophonium, huperzine et analogues d'huperzine, néostigmine, physotigmine, et pyridostigmine.

**26.** Procédé selon la revendication 25 dans lequel ledit inhibiteur de cholinestérase comprend de la physostigmine.

**27.** Procédé selon la revendication 19 dans lequel ledit composé chimique se lie sélectivement au récepteur muscarinique M1.

**28.** Procédé selon la revendication 1 dans lequel l'agent d'induction est une stimulation électrique.

FIG. 1

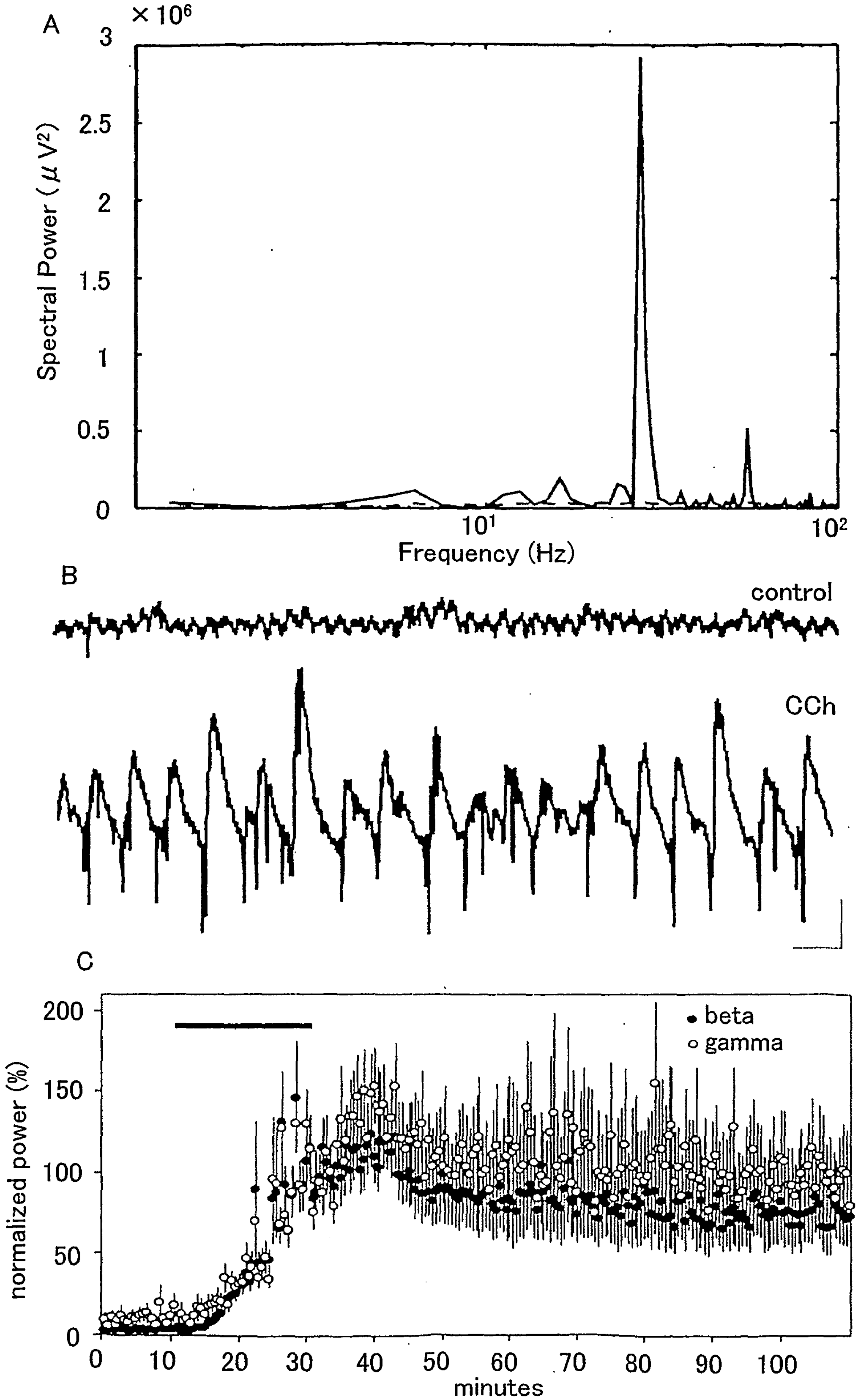
A
×10^6
3
2.5
2
1.5
1
0.5
0
Spectral Power (μV²)
10¹
10²
Frequency (Hz)
B
control
CCh
C
200
150
100
50
0
normalized power (%)
• beta
○ gamma
0
10
20
30
40
50
60
70
80
90
100
minutes

FIG. 2

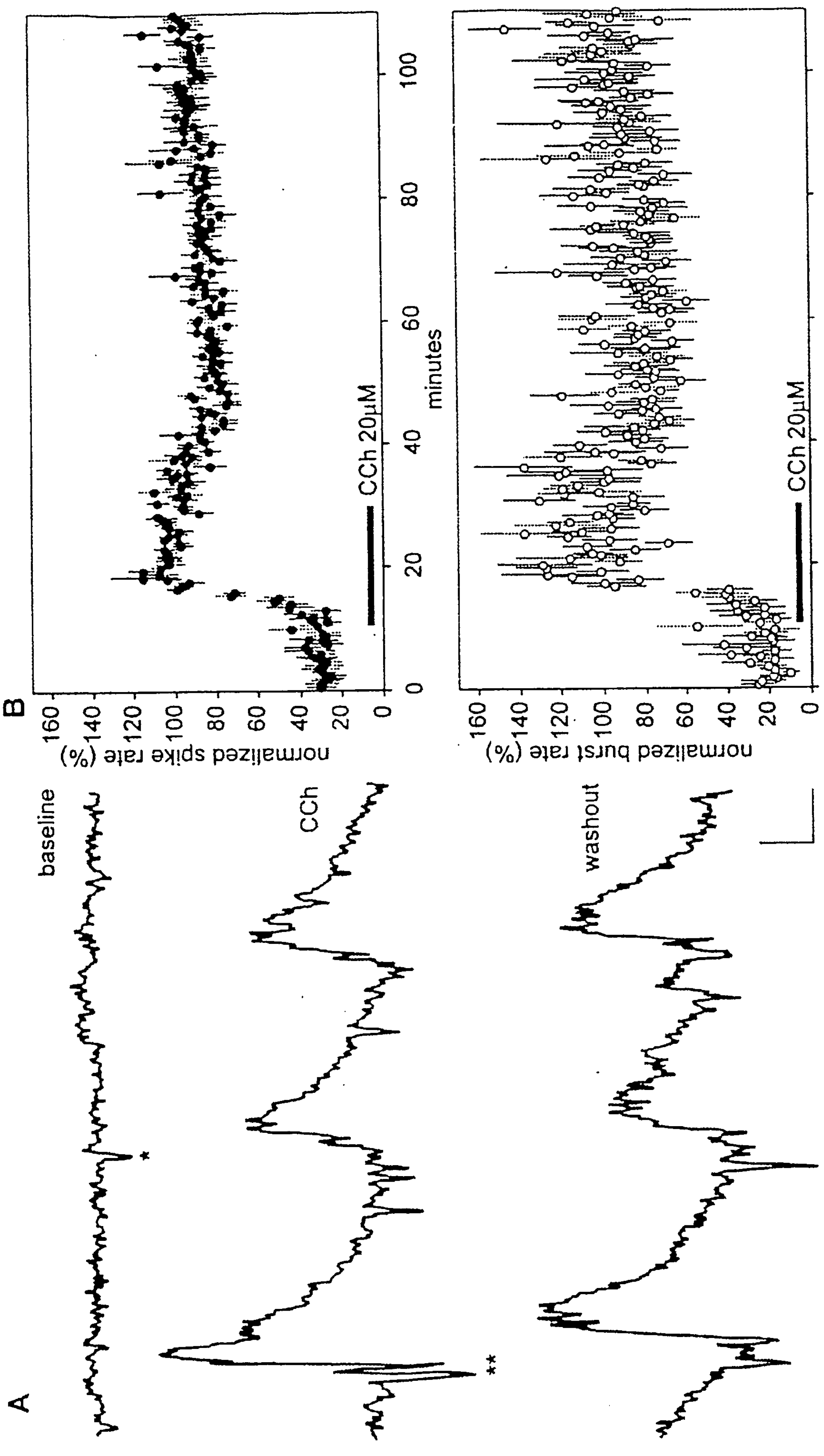
A
baseline
CCh
washout
*
**
B
normalized spike rate (%)
normalized burst rate (%)
CCh 20µM
minutes
0
20
40
60
80
100
120
140
160

FIG. 3

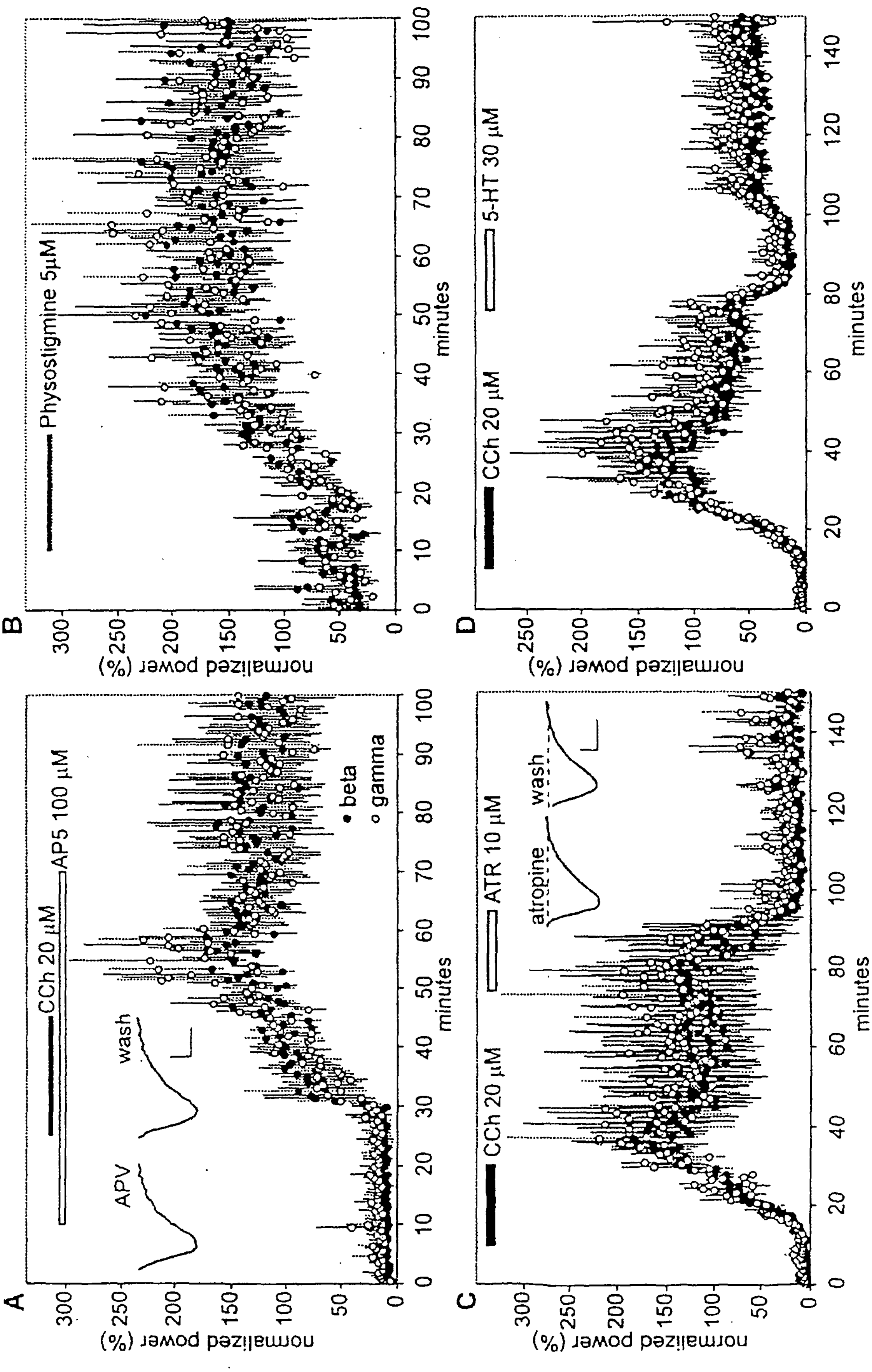
A
B
C
D
normalized power (%)
minutes
CCh 20 µM
AP5 100 µM
APV
wash
• beta
○ gamma
Physostigmine 5µM
ATR 10 µM
atropine
5-HT 30 µM

FIG. 4

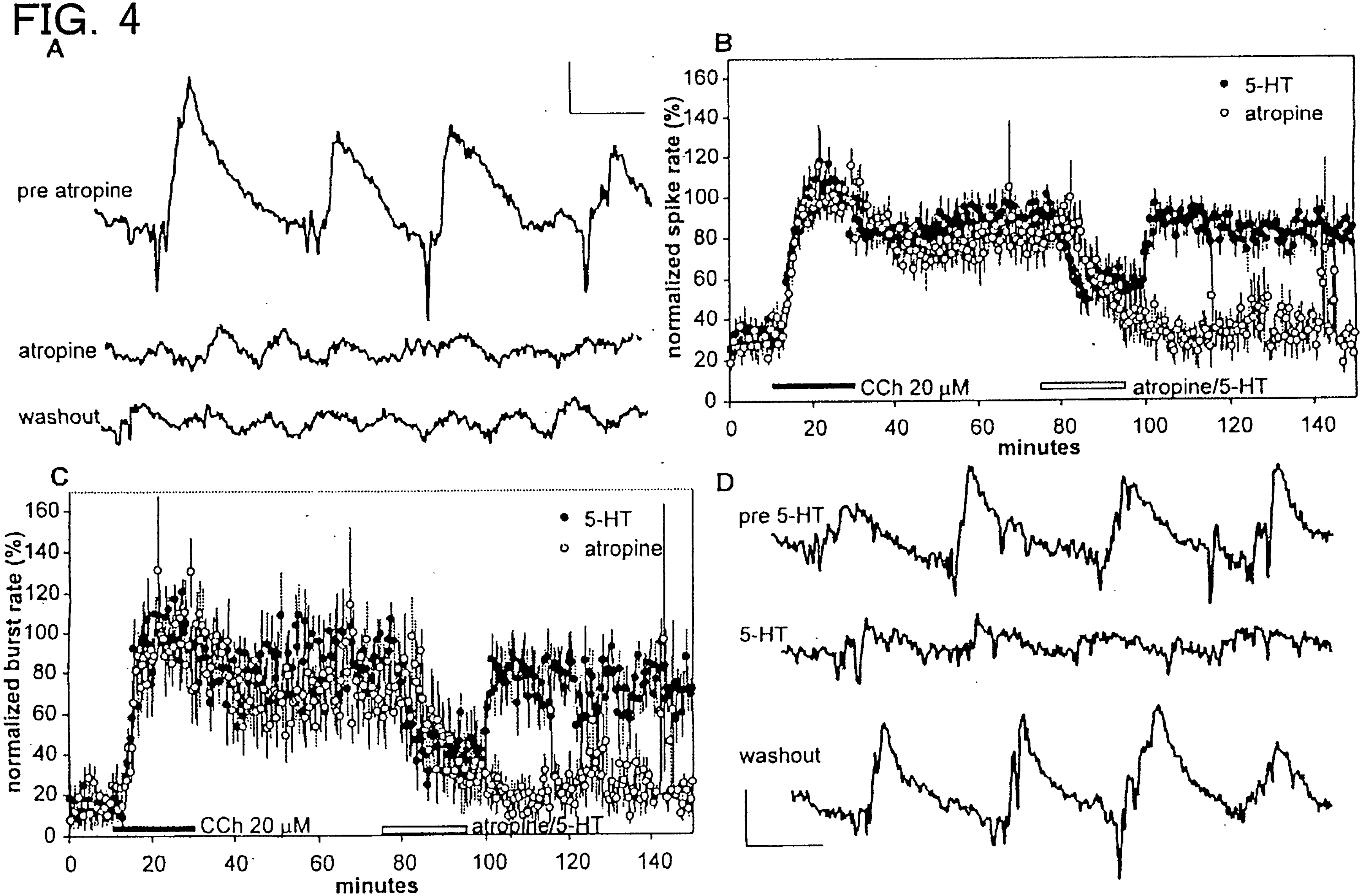
A
pre atropine
atropine
washout
B
normalized spike rate (%)
• 5-HT
○ atropine
CCh 20 μM
atropine/5-HT
minutes
C
normalized burst rate (%)
• 5-HT
○ atropine
CCh 20 μM
atropine/5-HT
minutes
D
pre 5-HT
5-HT
washout

FIG. 5

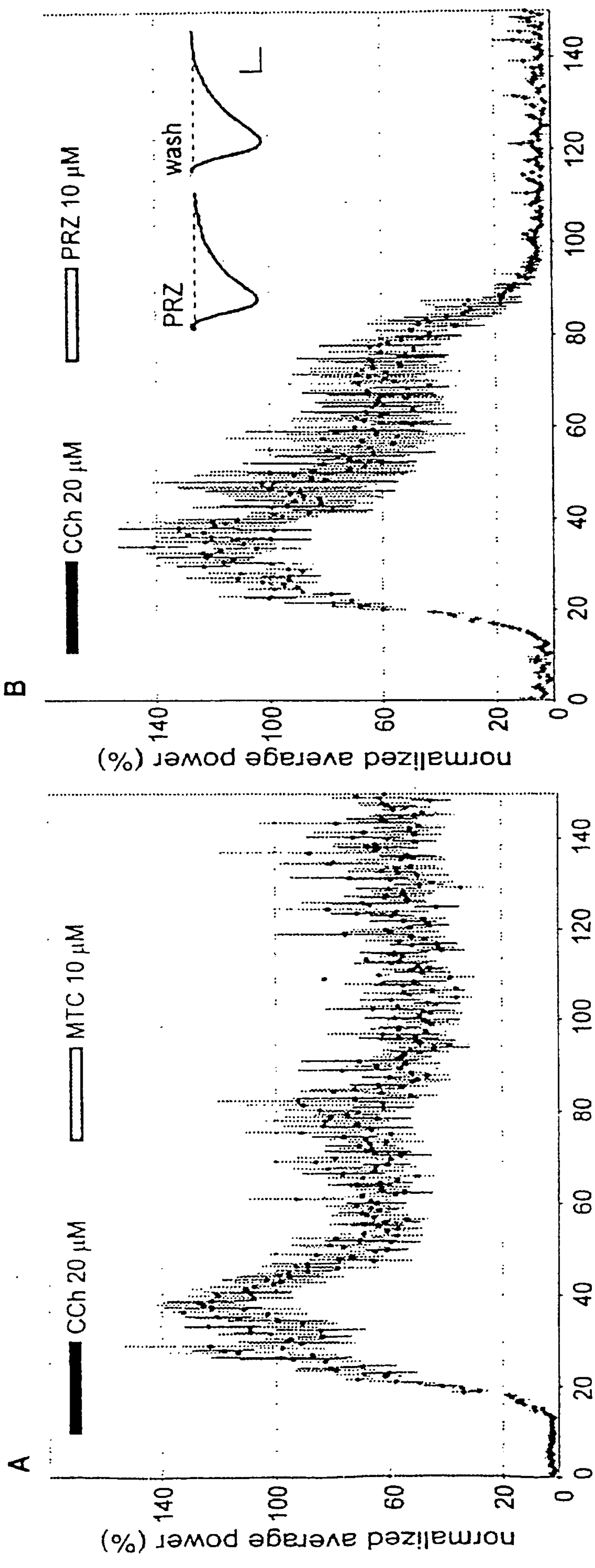
A
CCh 20 μM
MTC 10 μM
normalized average power (%)
minutes
B
CCh 20 μM
PRZ 10 μM
PRZ
wash
normalized average power (%)
minutes

FIG. 6

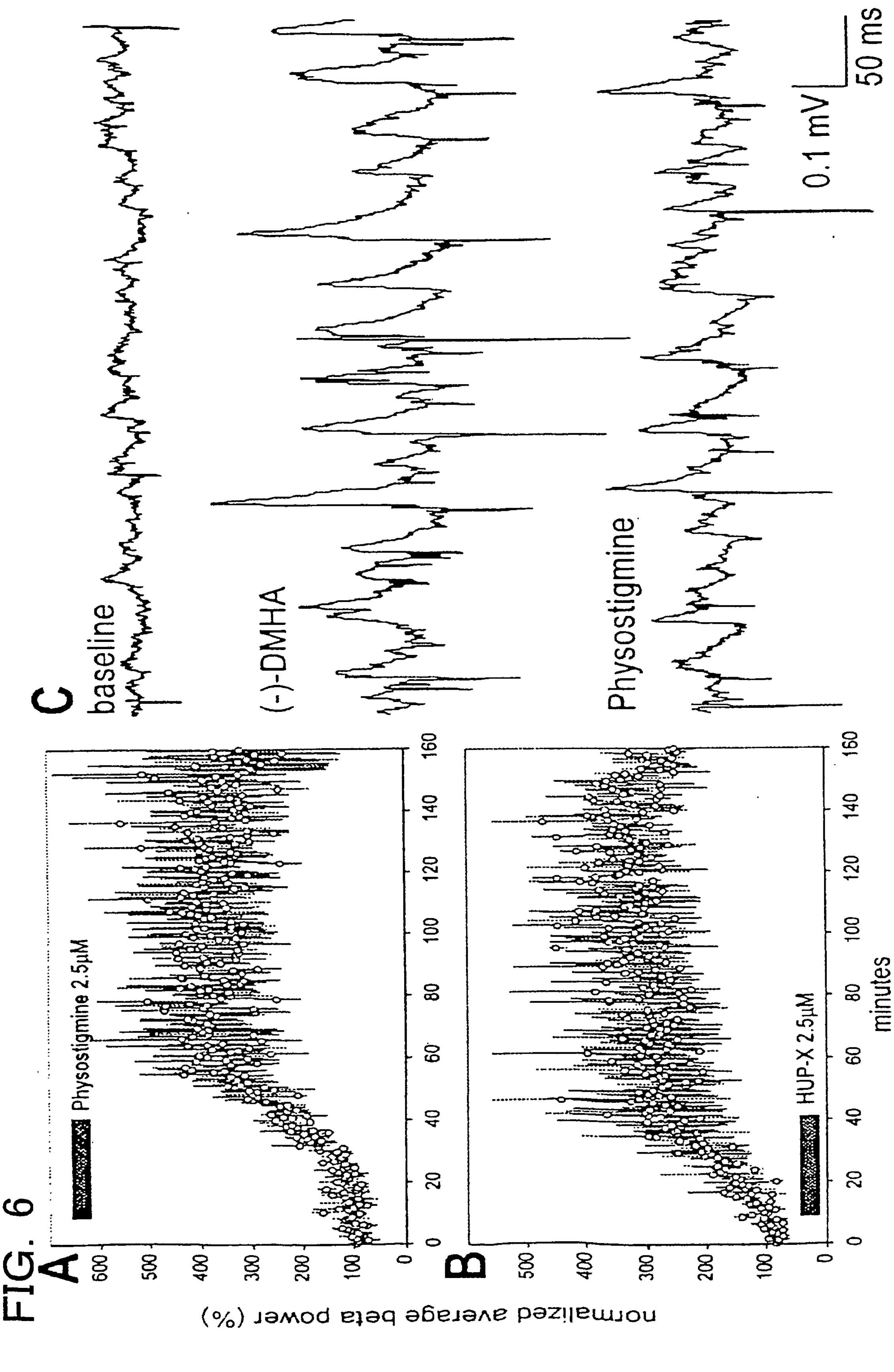
A
B
C
normalized average beta power (%)
minutes
Physostigmine 2.5μM
HUP-X 2.5μM
baseline
(-)-DMHA
Physostigmine
0.1 mV
50 ms
0
100
200
300
400
500
600
20
40
60
80
100
120
140
160

FIG. 6

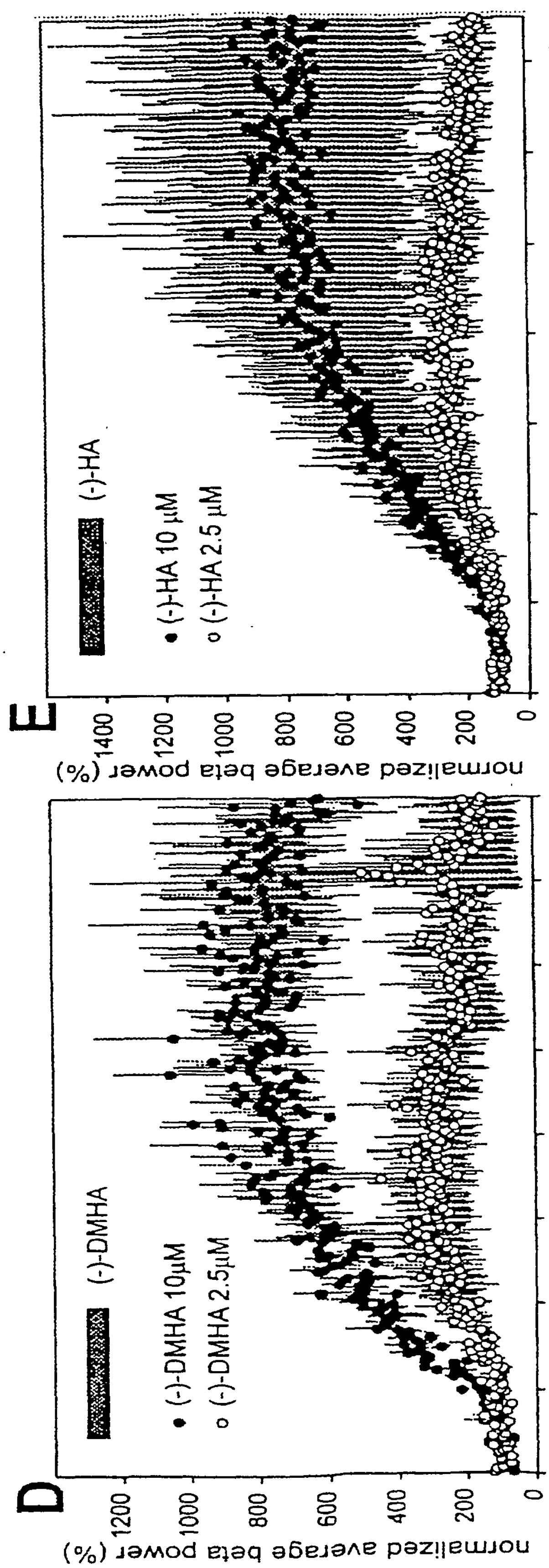
D
(-)-DMHA
(-)-DMHA 10µM
(-)-DMHA 2.5µM
normalized average beta power (%)
minutes
E
(-)-HA
(-)-HA 10 µM
(-)-HA 2.5 µM
normalized average beta power (%)
minutes

FIG. 7

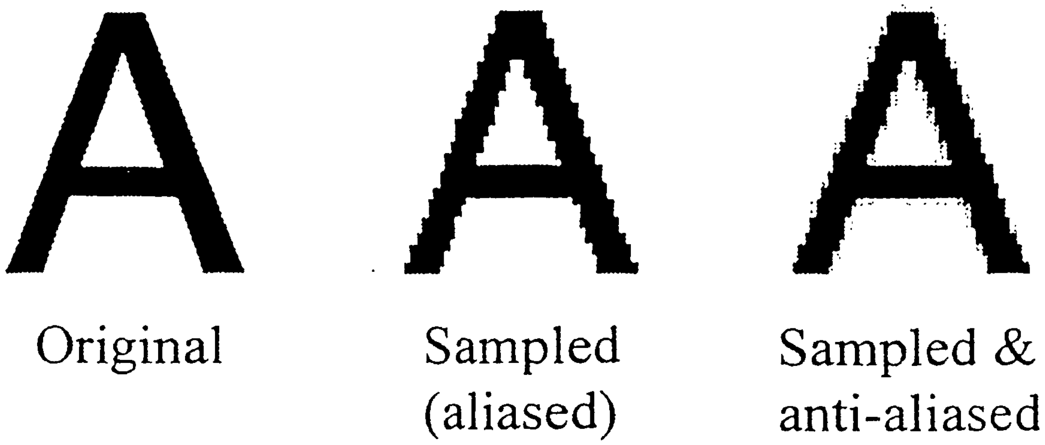
Original
Sampled (aliased)
Sampled & anti-aliased

FIG. 8

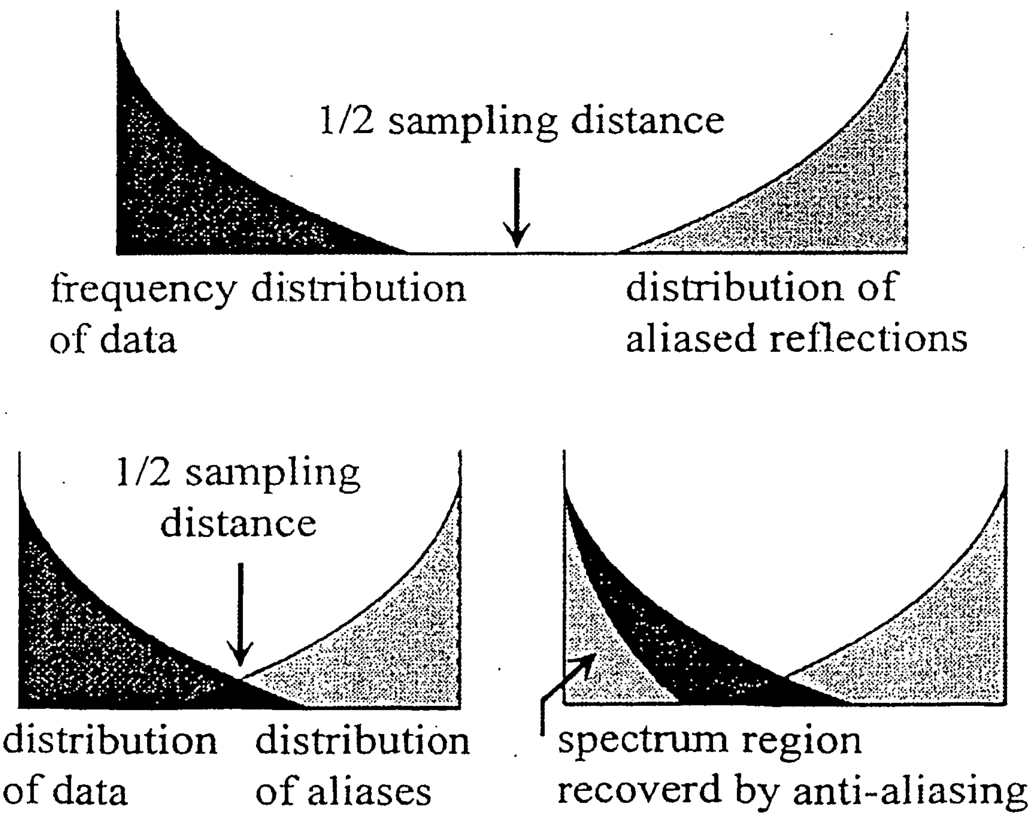
1/2 sampling distance
frequency distribution of data
distribution of aliased reflections
1/2 sampling distance
distribution of data
distribution of aliases
spectrum region recoverd by anti-aliasing

FIG. 9

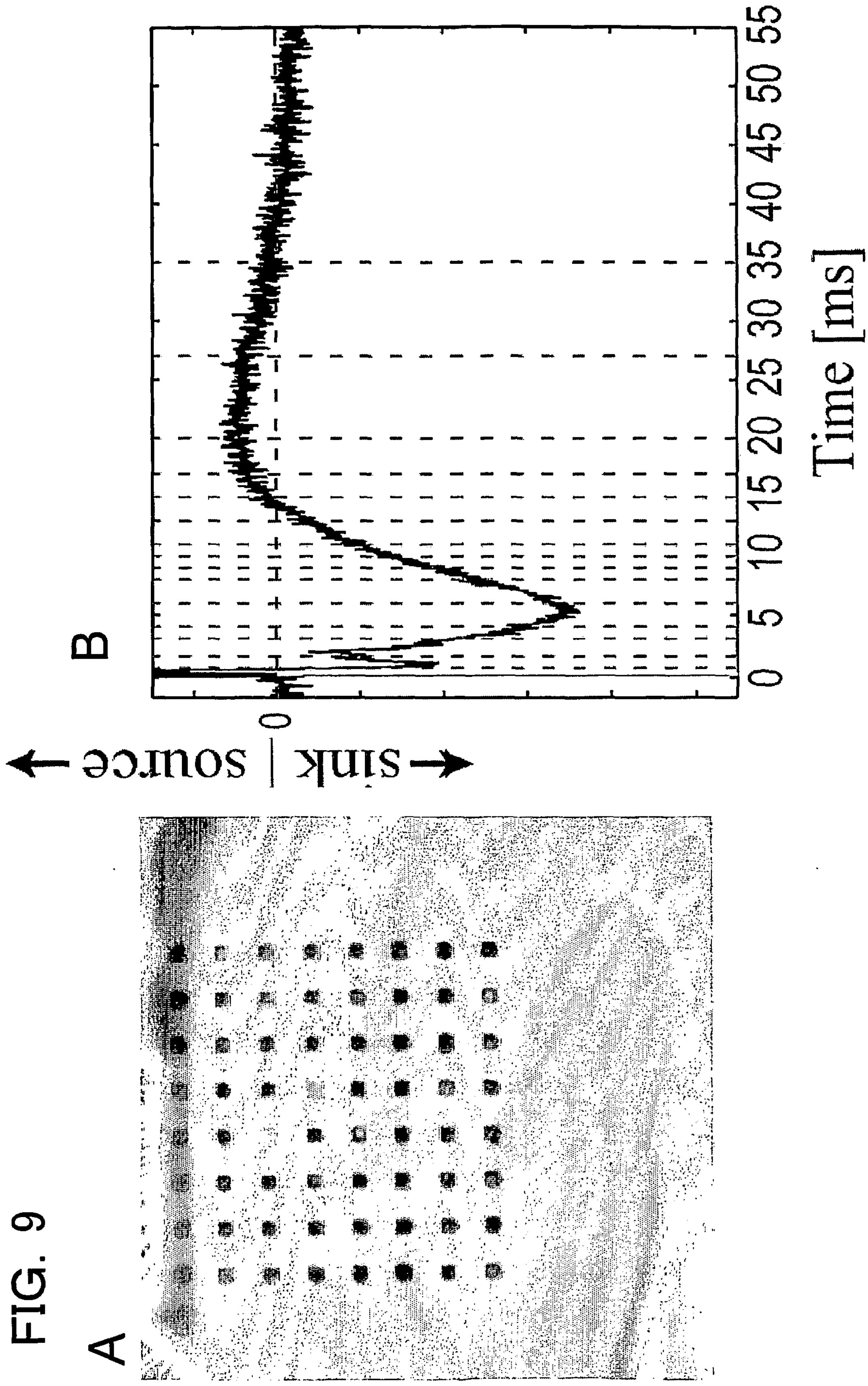

FIG. 10

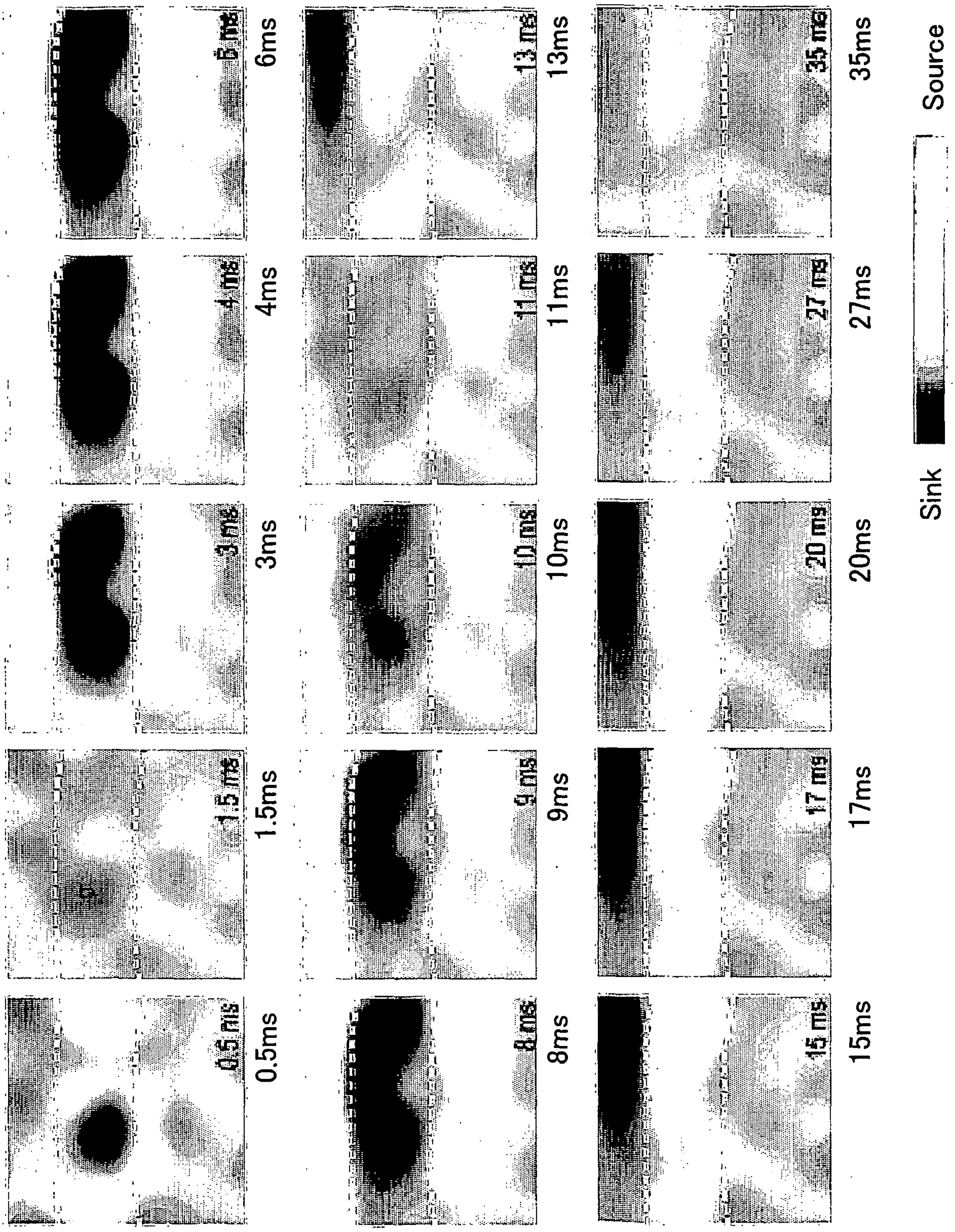
0.5ms
1.5ms
3ms
4ms
6ms
8ms
9ms
10ms
11ms
13ms
15ms
17ms
20ms
27ms
35ms
Sink
Source

FIG. 11

A

B

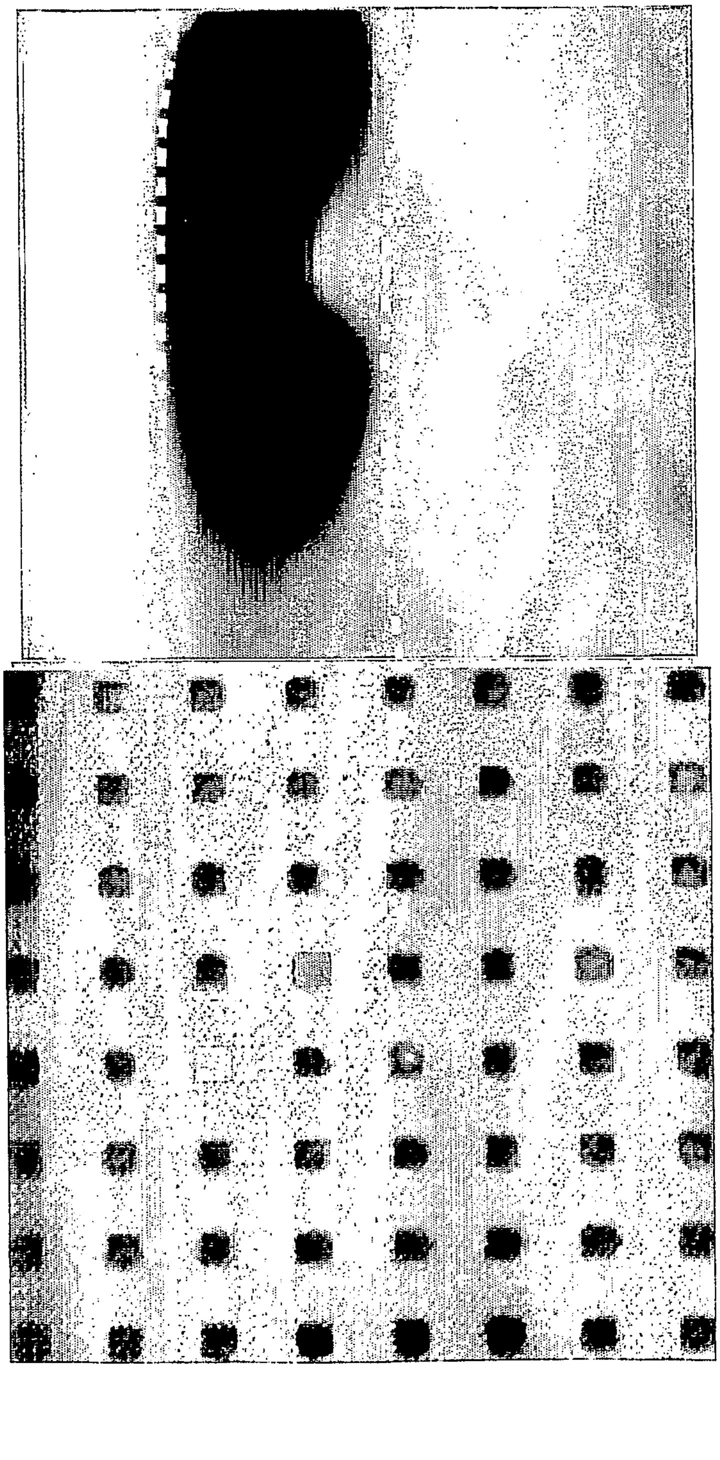

FIG. 12

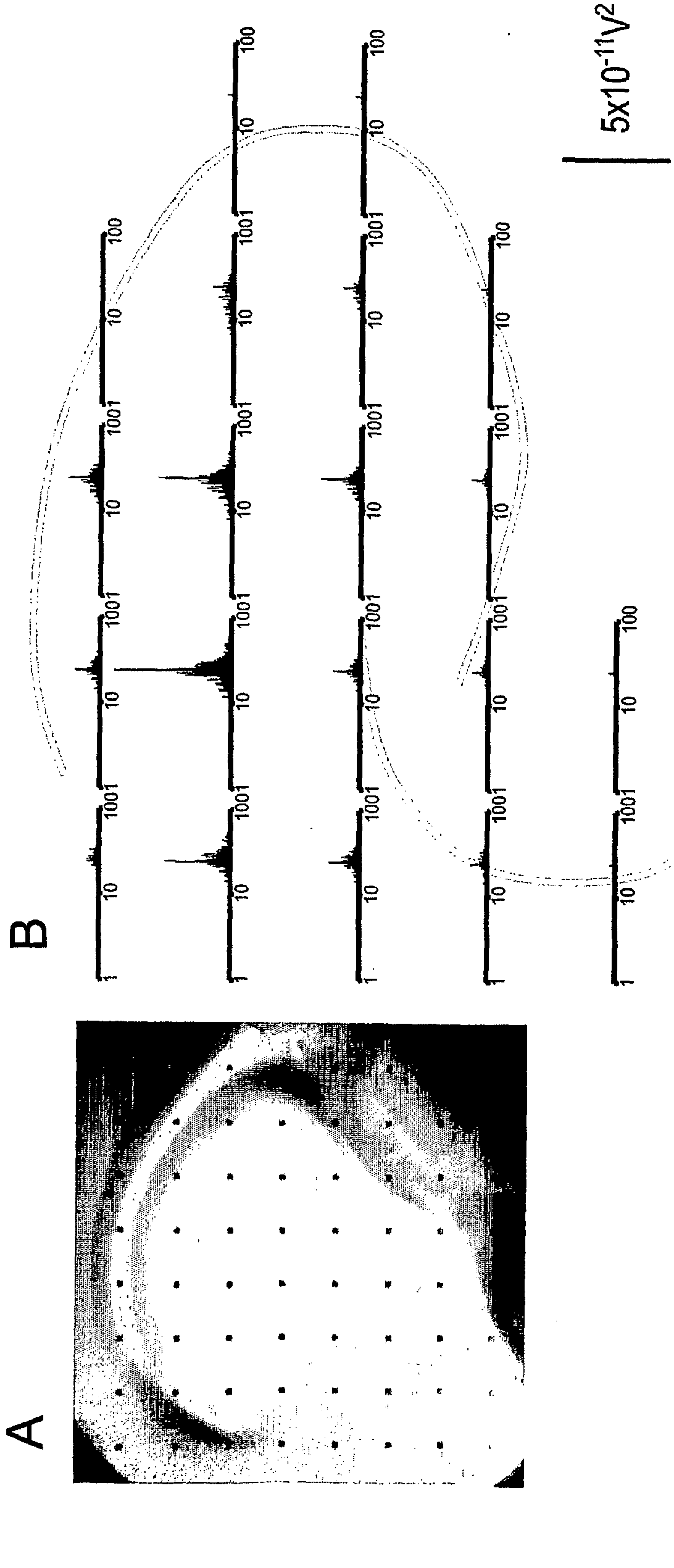

FIG. 12

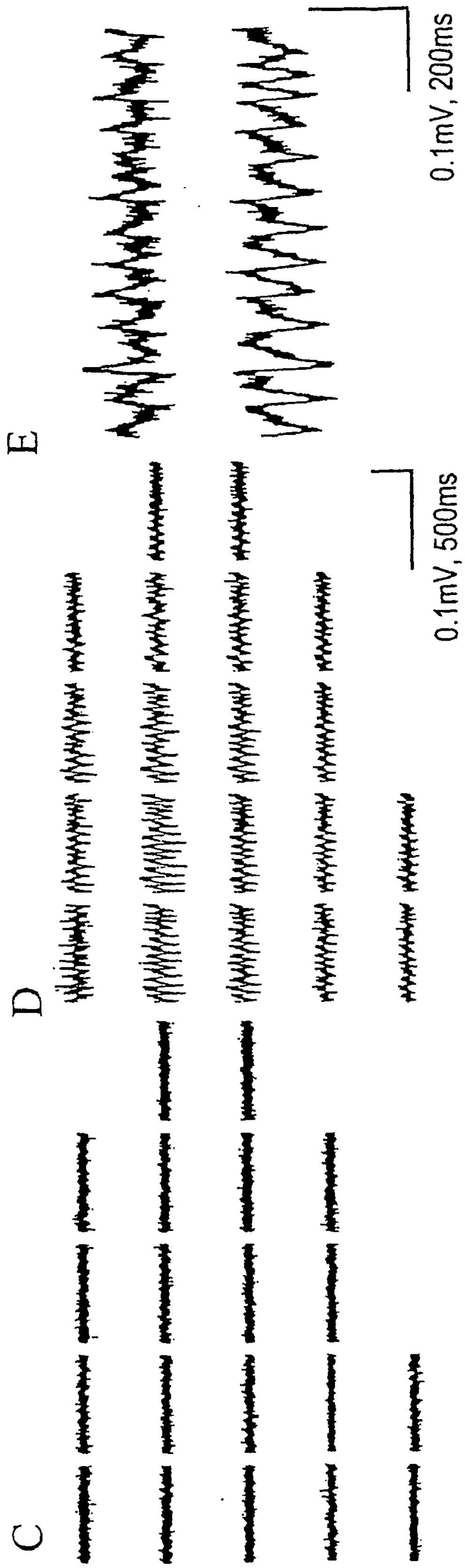

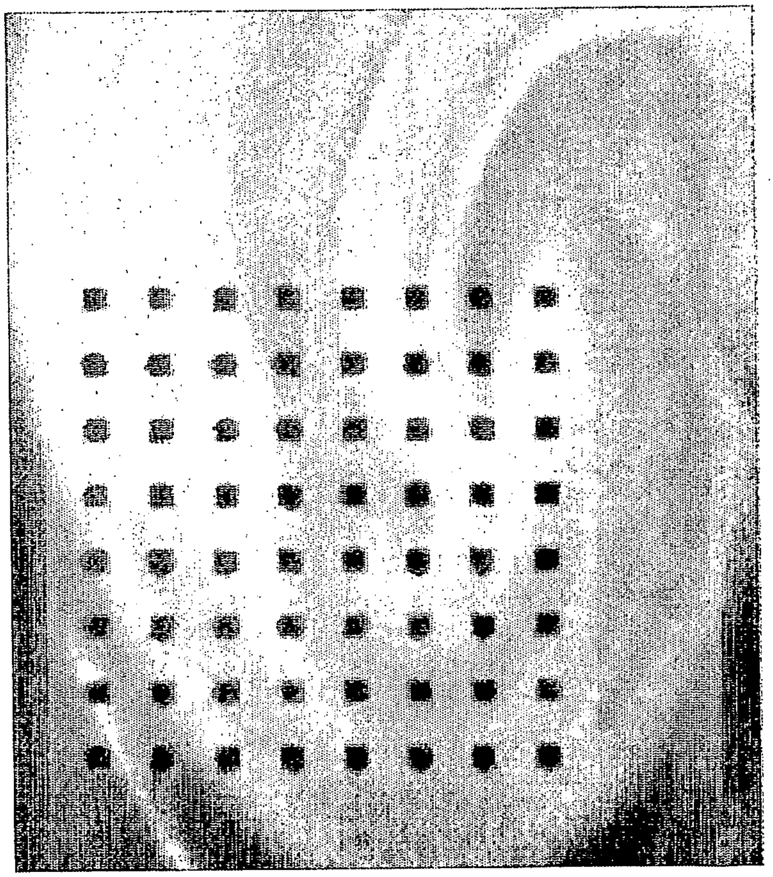

FIG. 13A

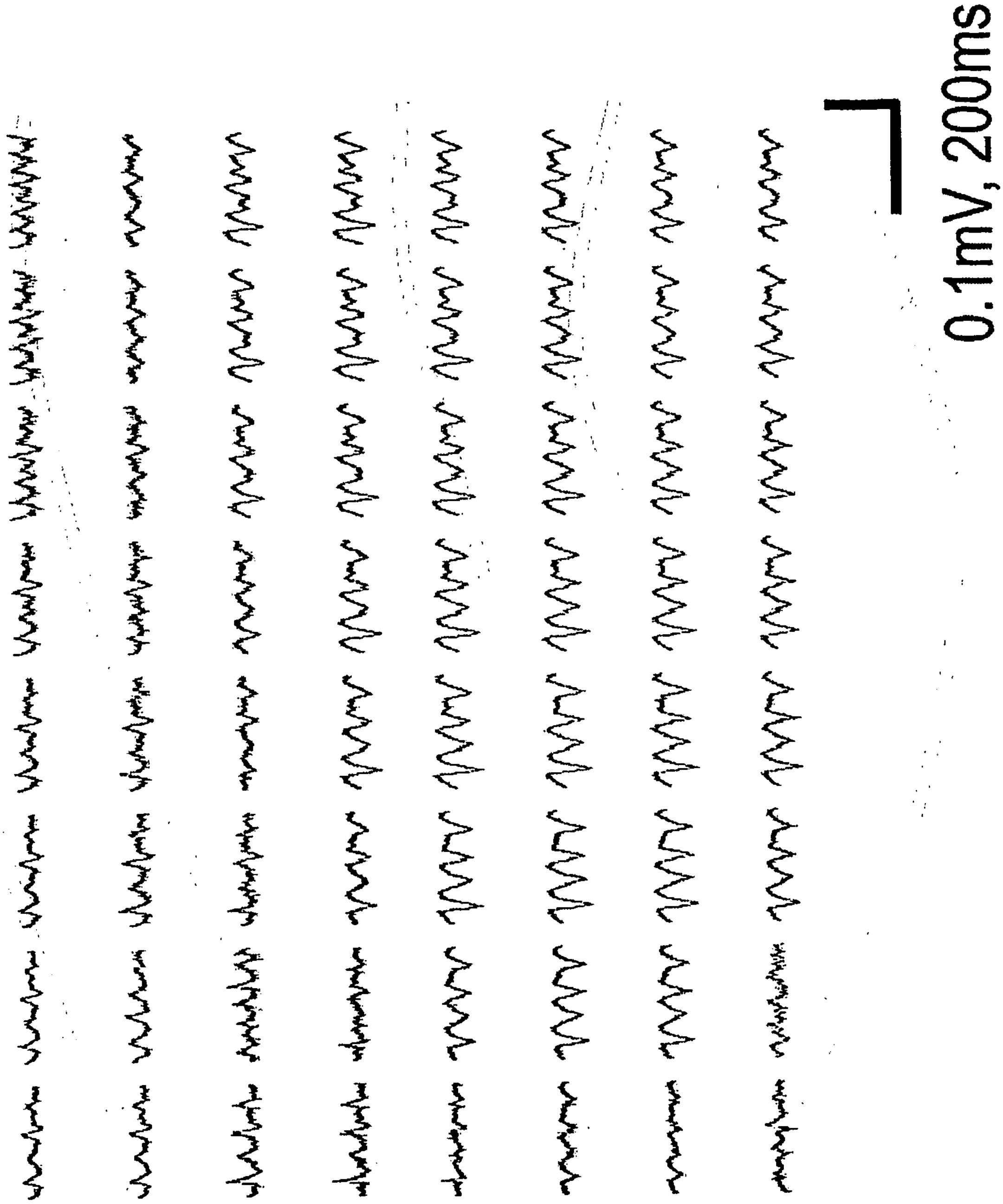


FIG. 13B

FIG. 13C

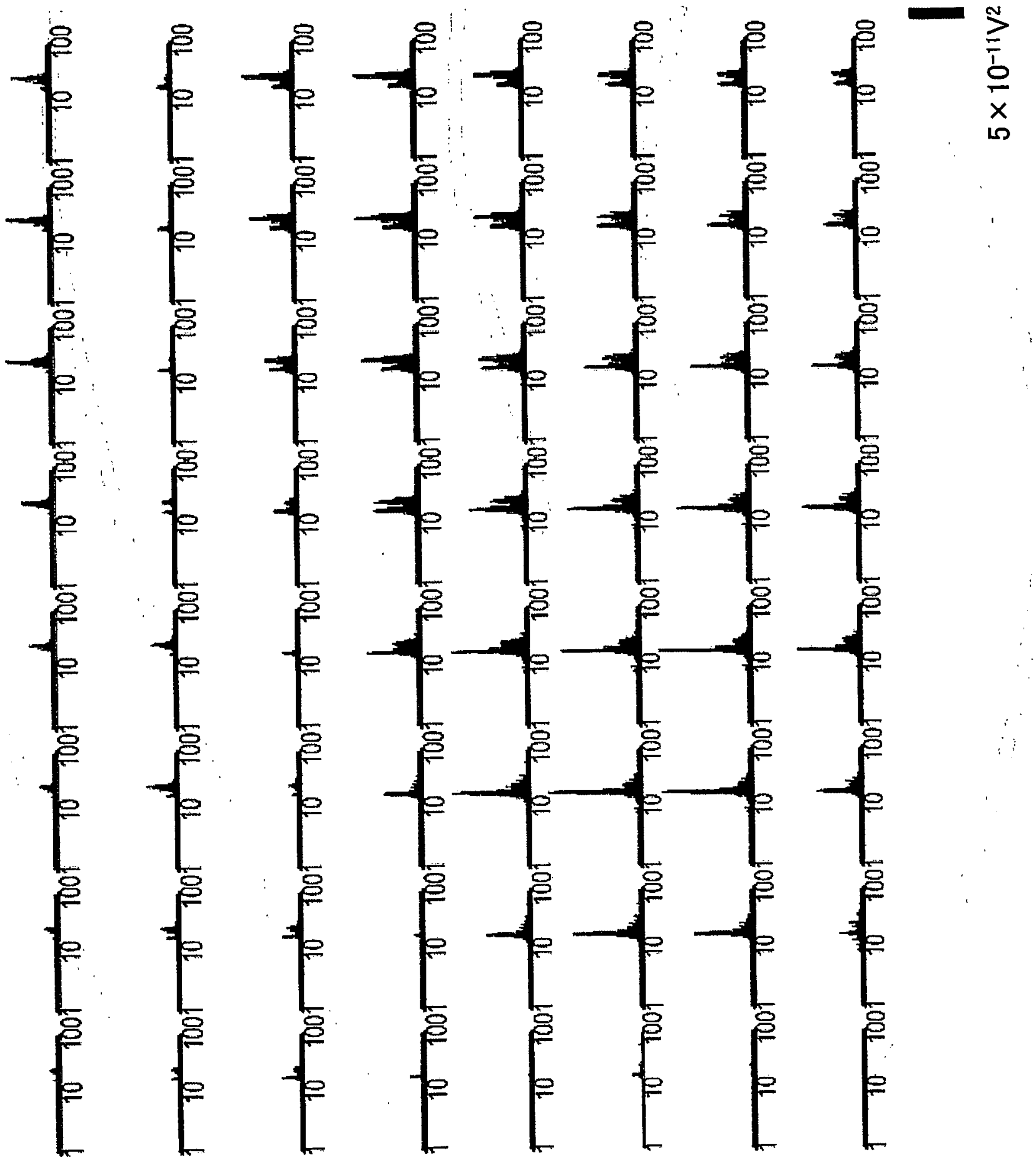

FIG. 14

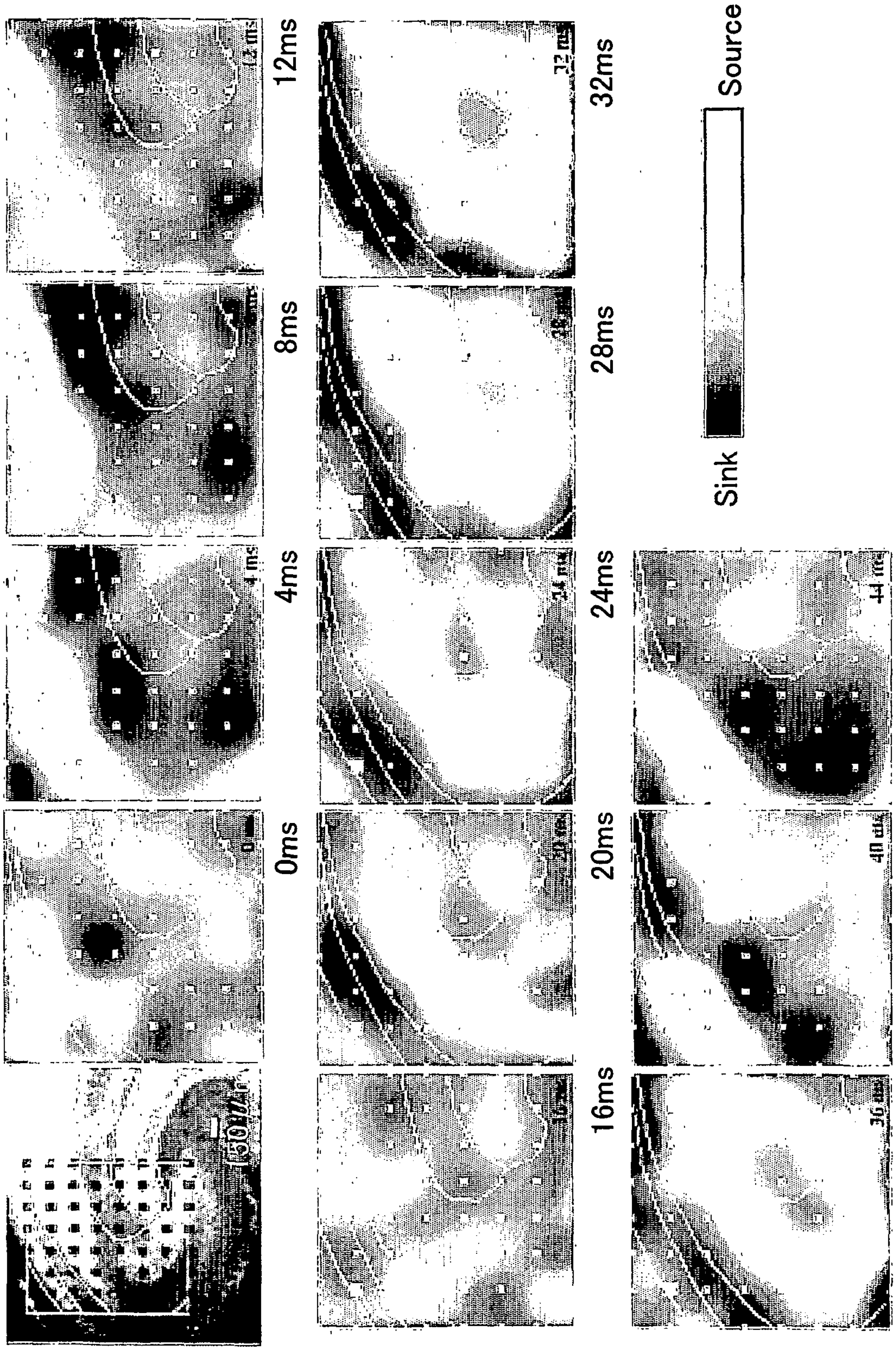
0ms
4ms
8ms
12ms
16ms
20ms
24ms
28ms
32ms
36ms
40ms
44ms
Sink
Source

FIG. 15

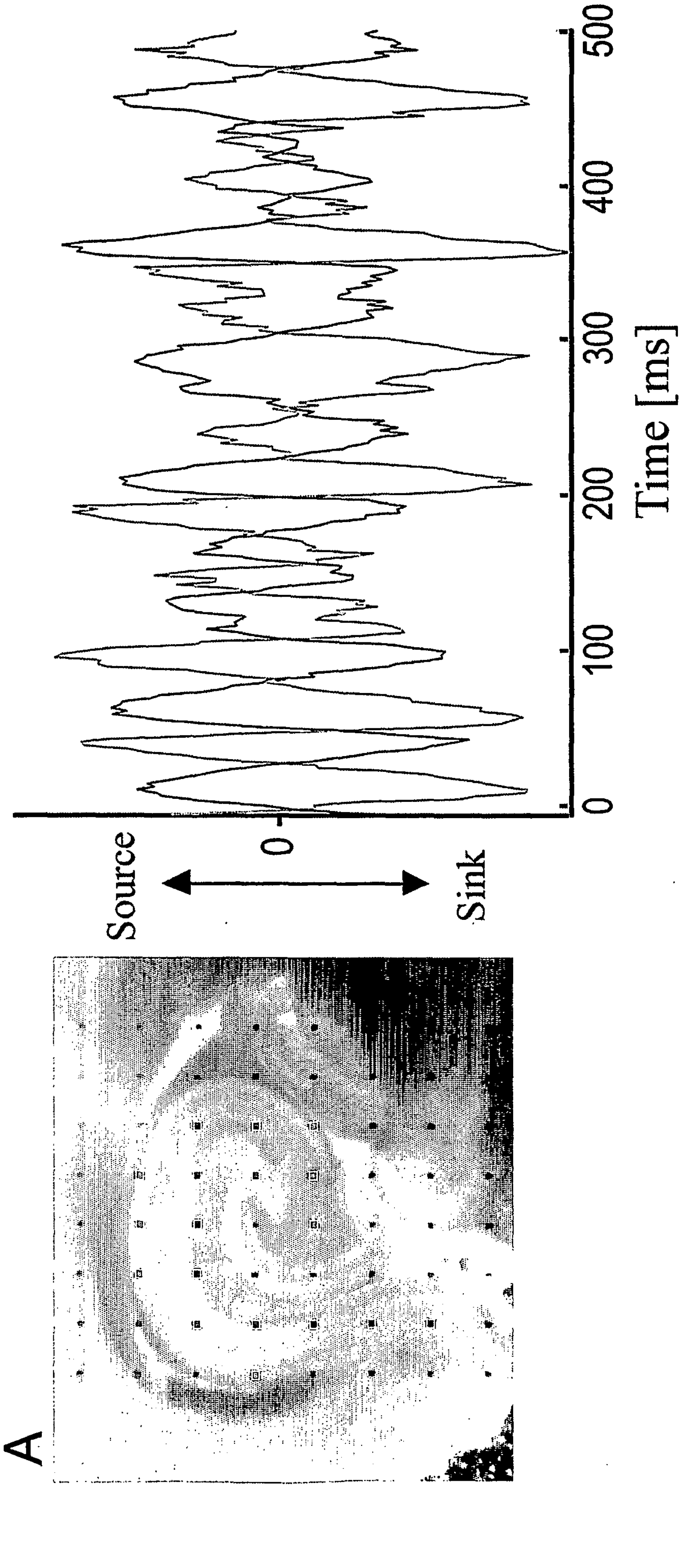

FIG. 16

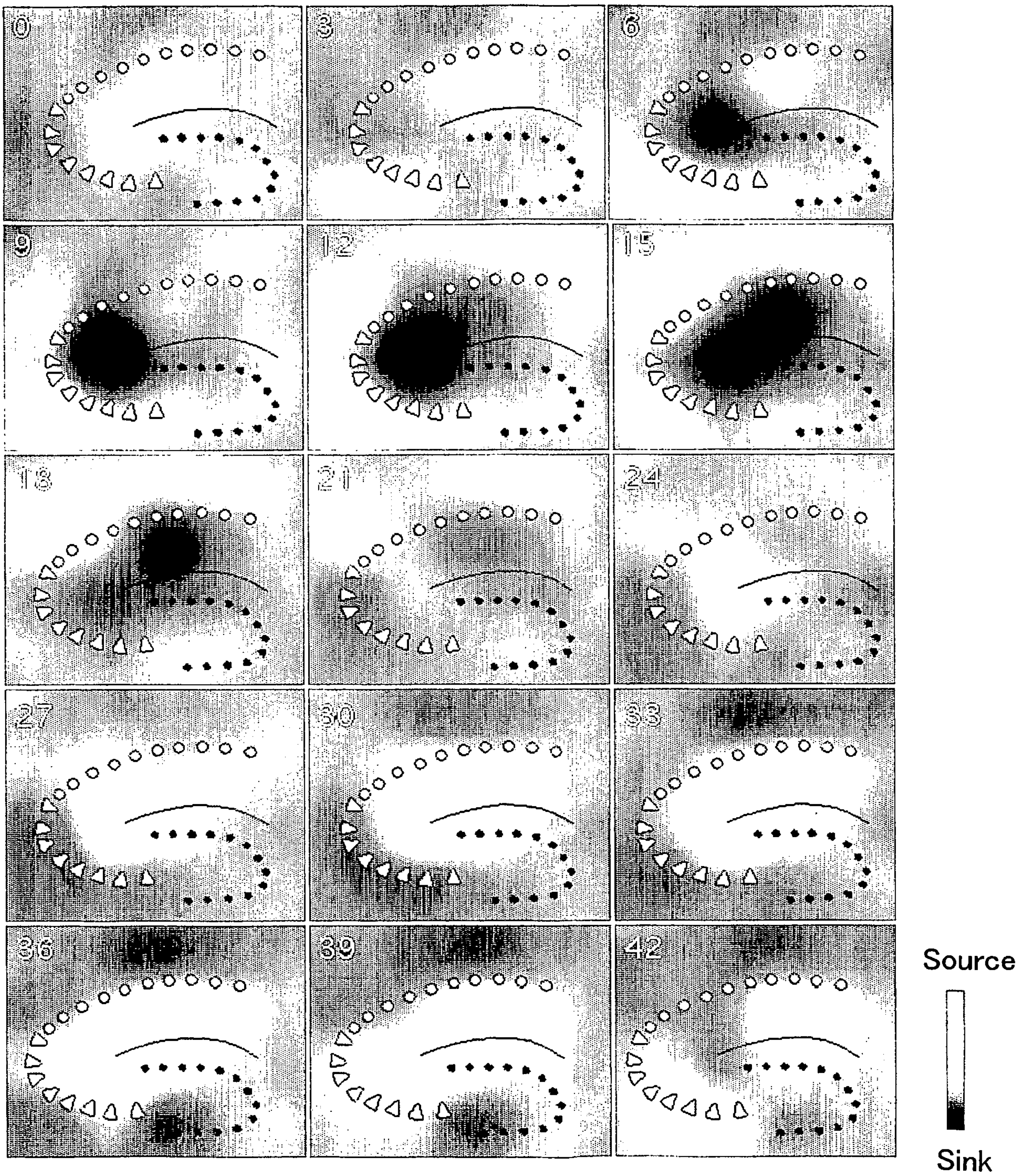

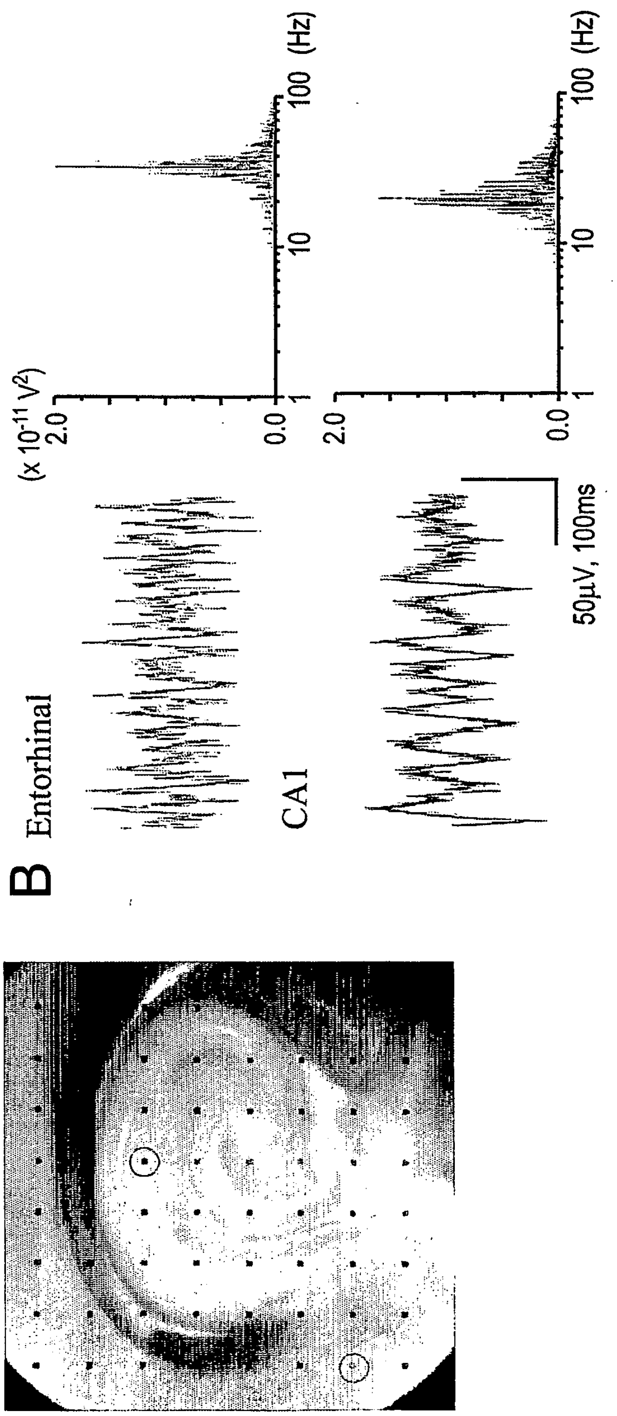


FIG. 17 A

FIG. 17

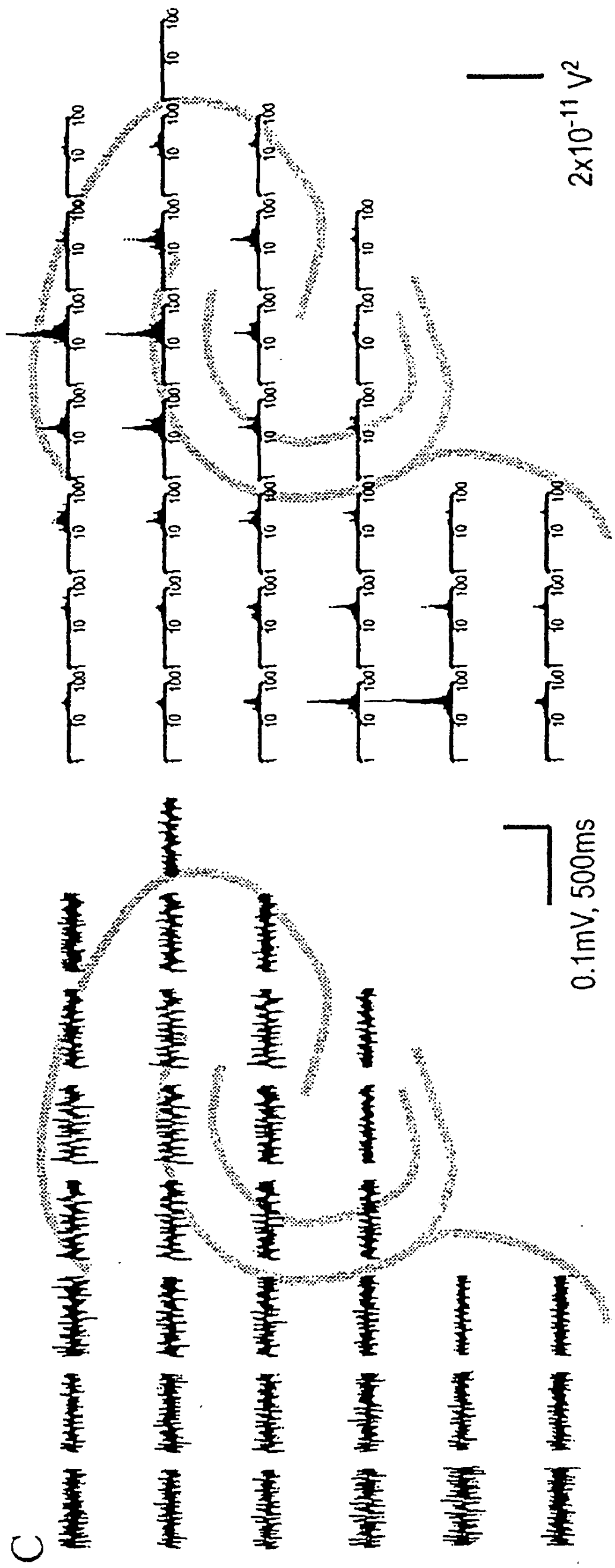

FIG. 18

A

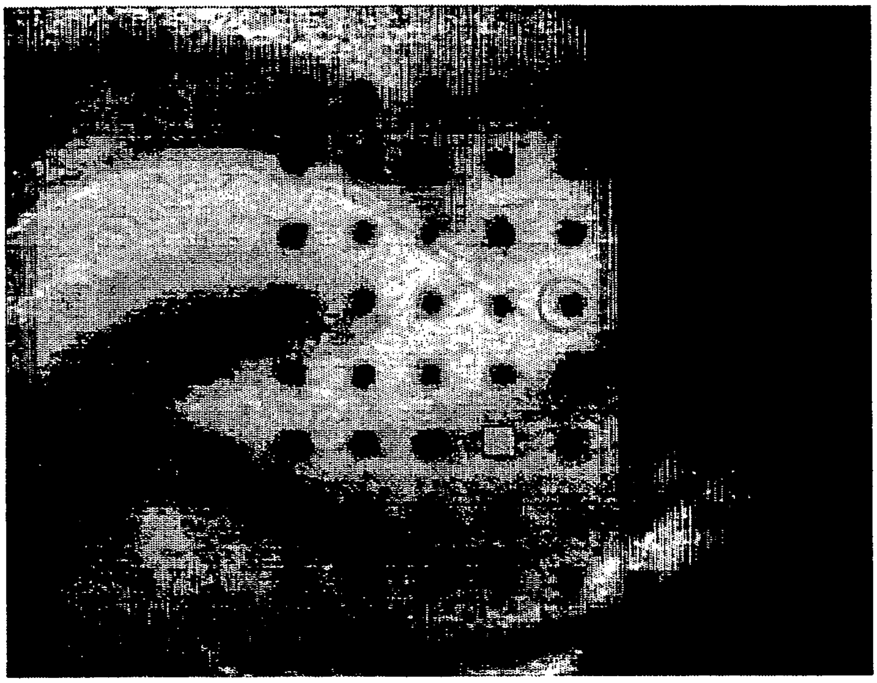

B

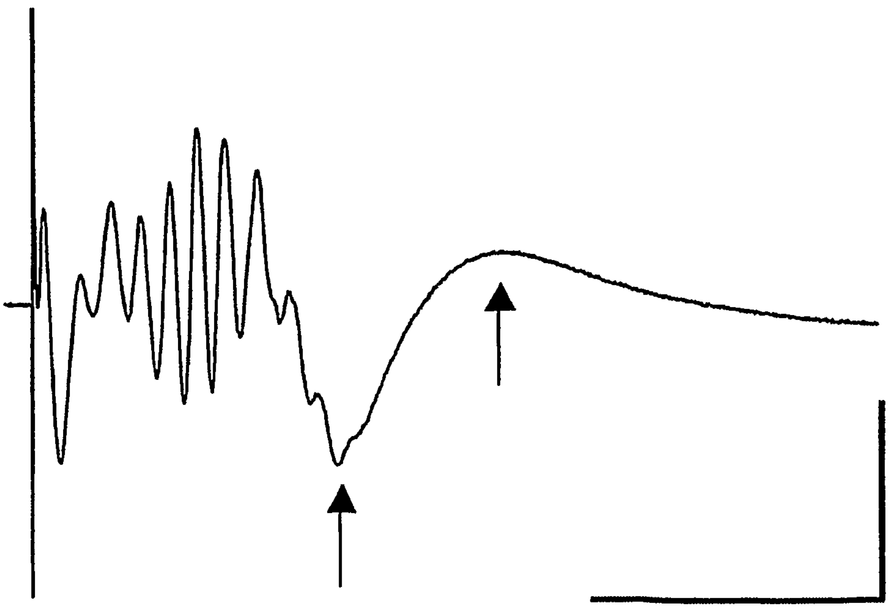

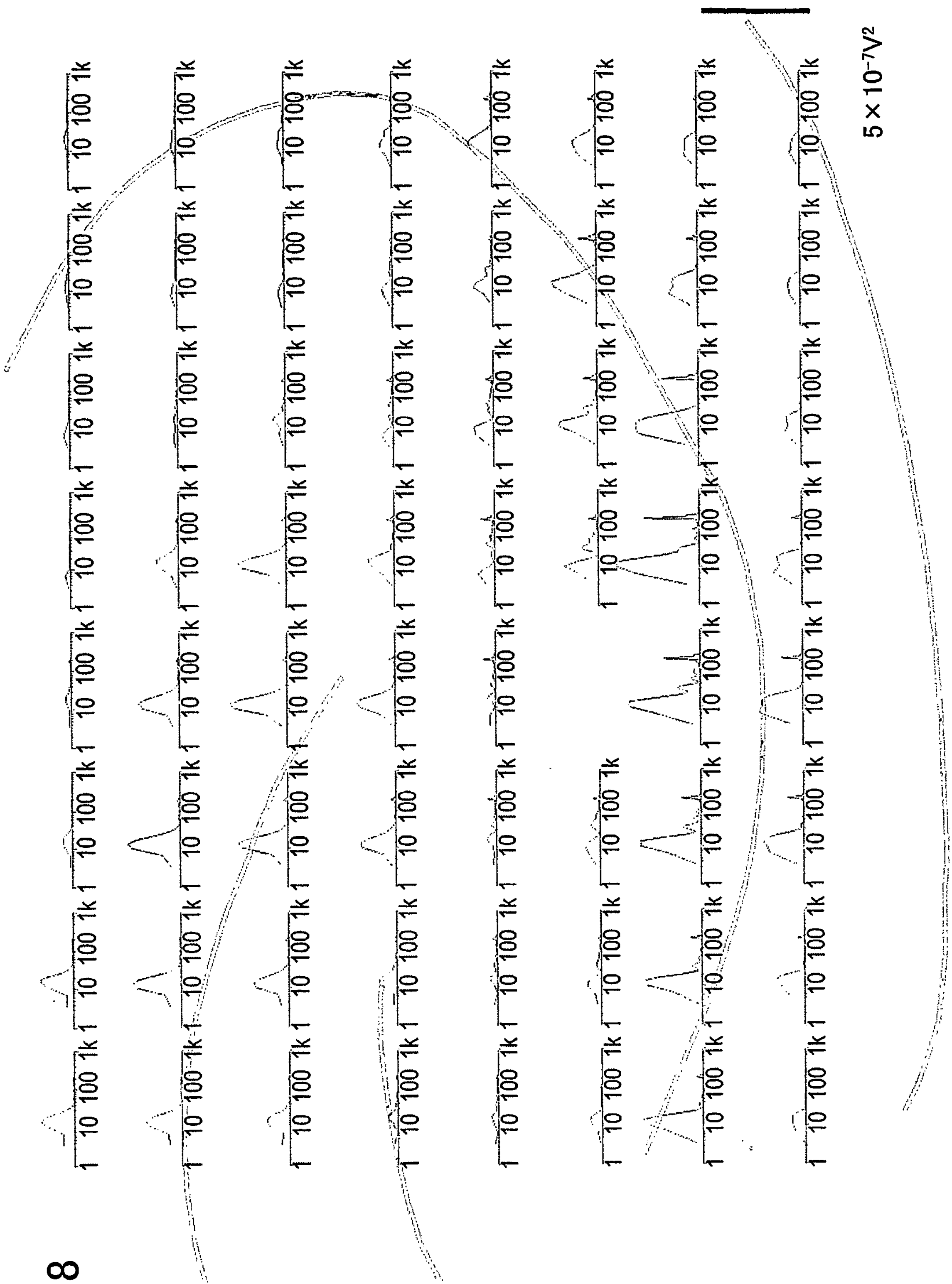


FIG. 18
C

FIG. 18

D

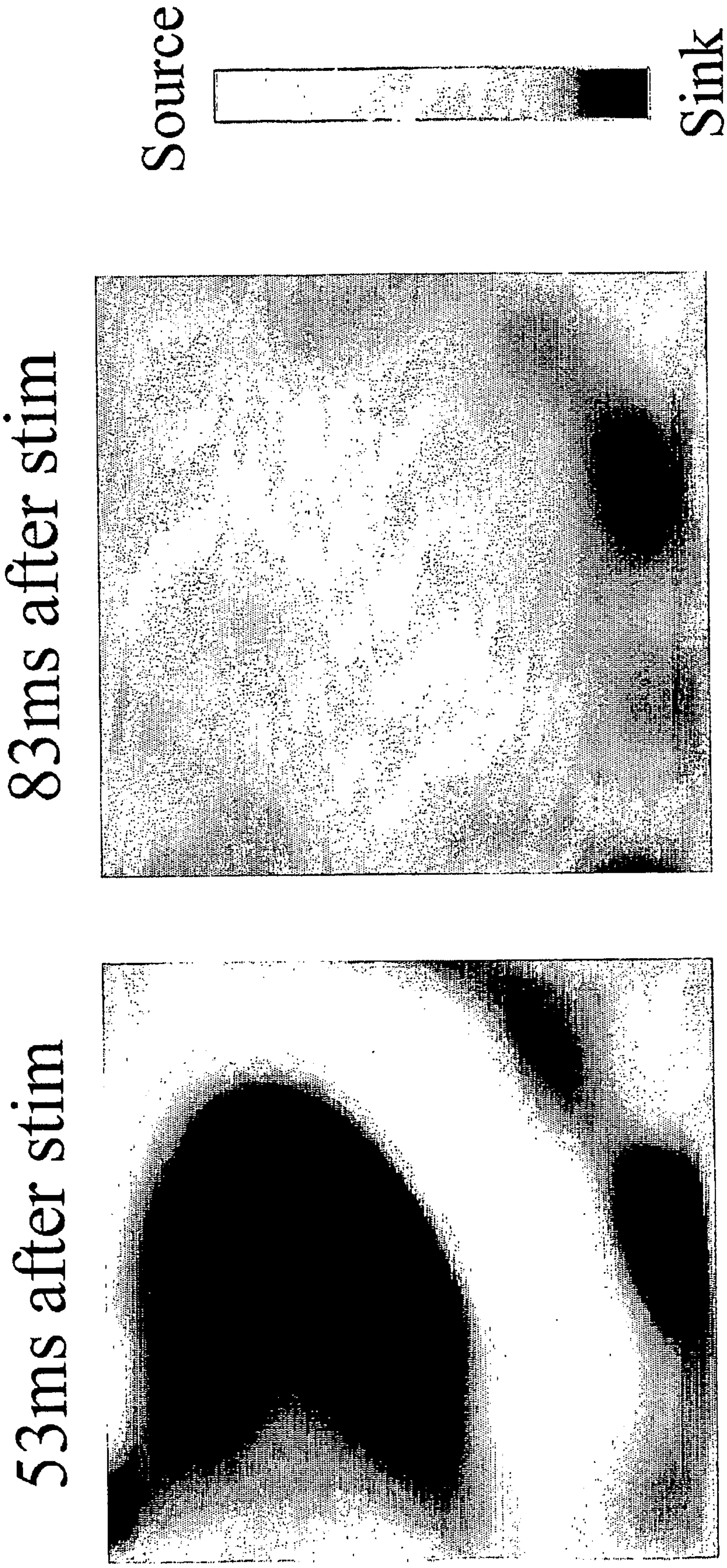

FIG. 19

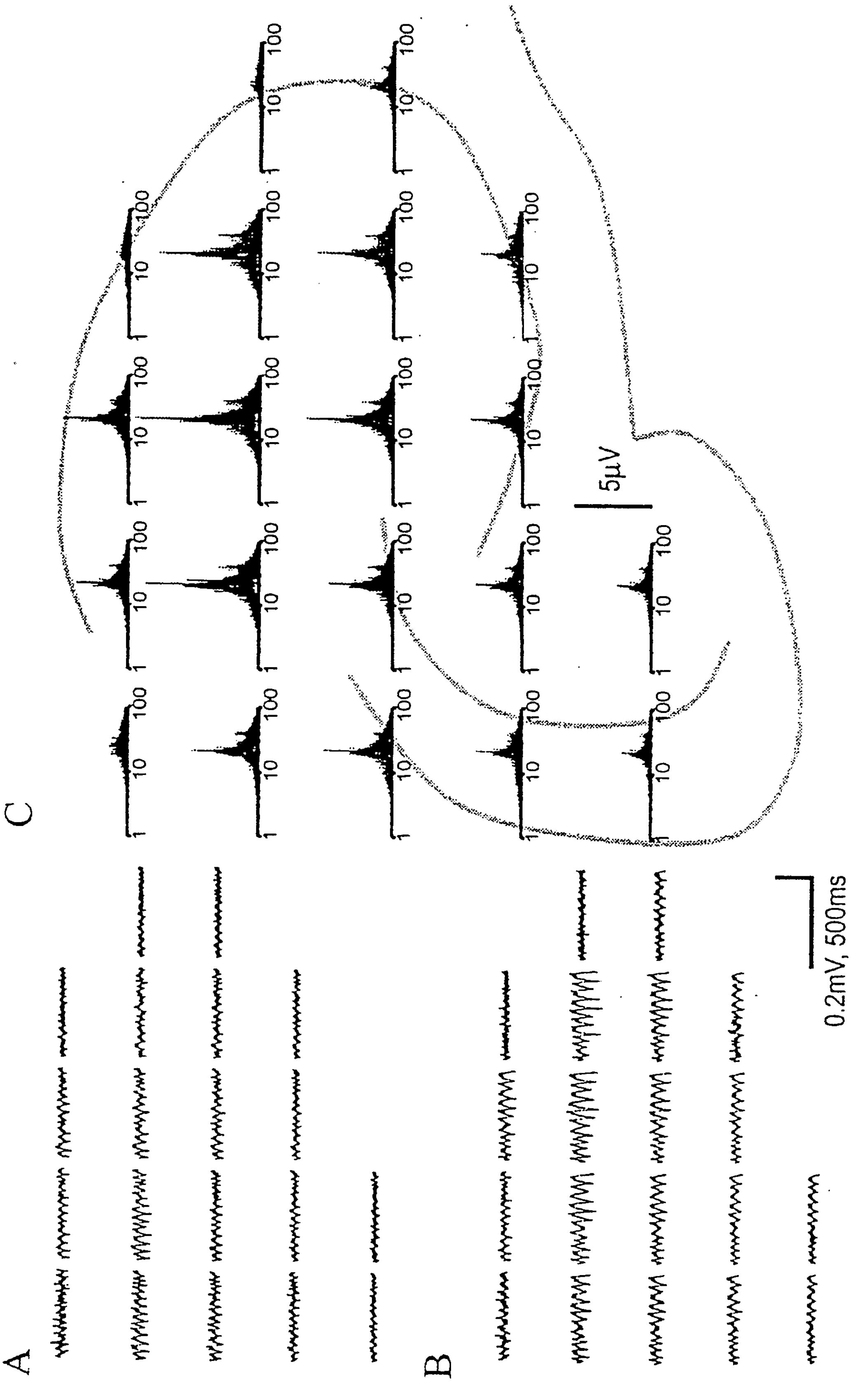

FIG. 20

A

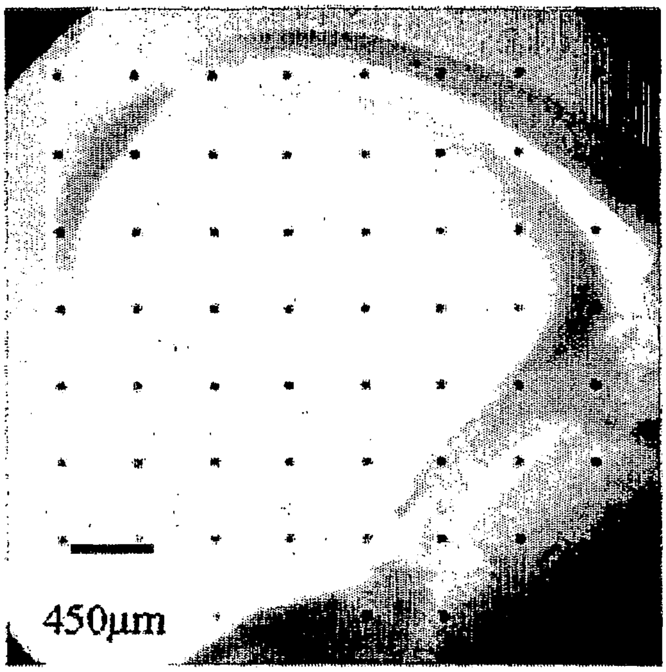


B

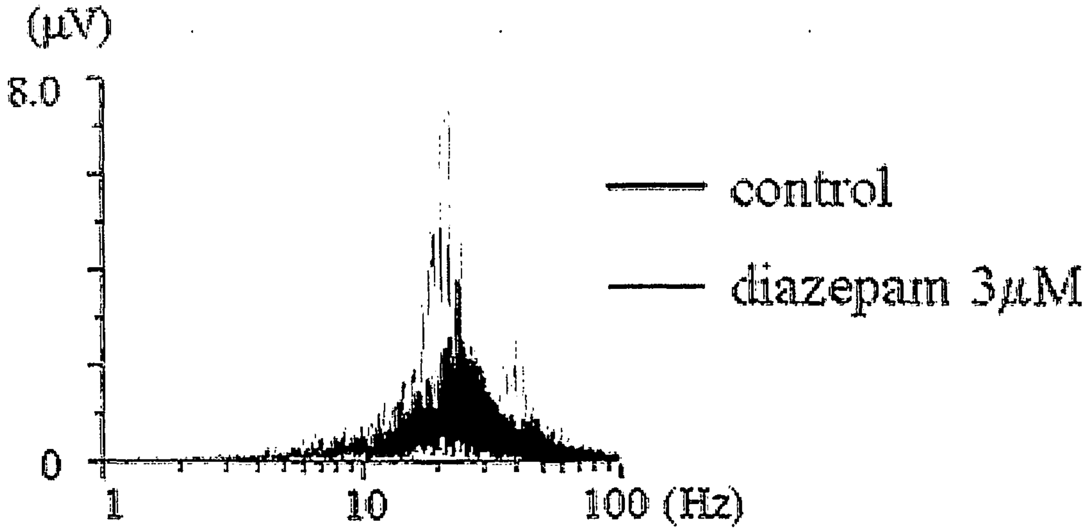

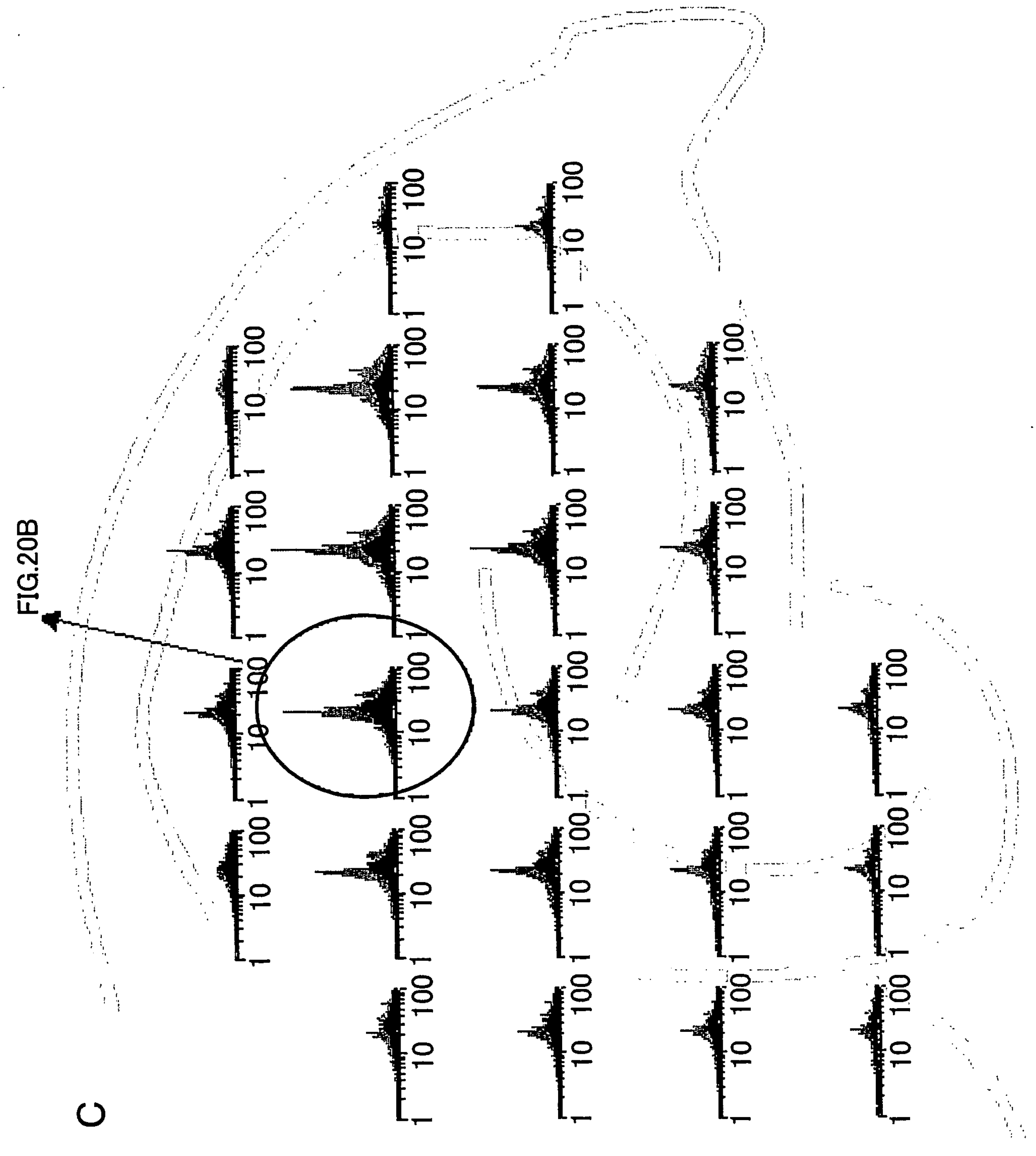


FIG. 20

FIG. 21

A

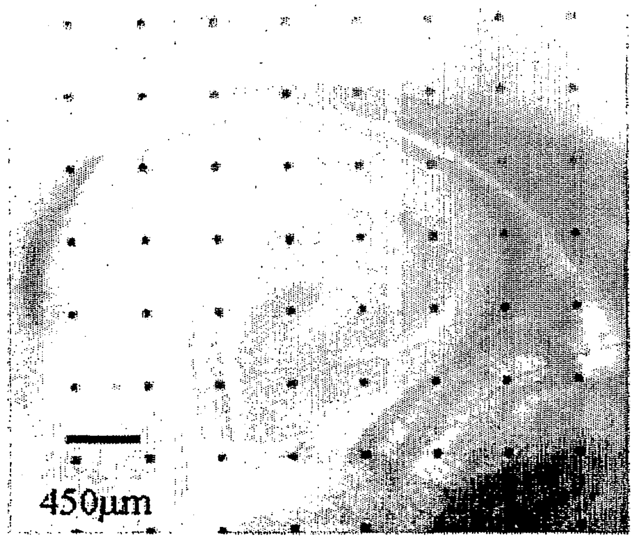


B

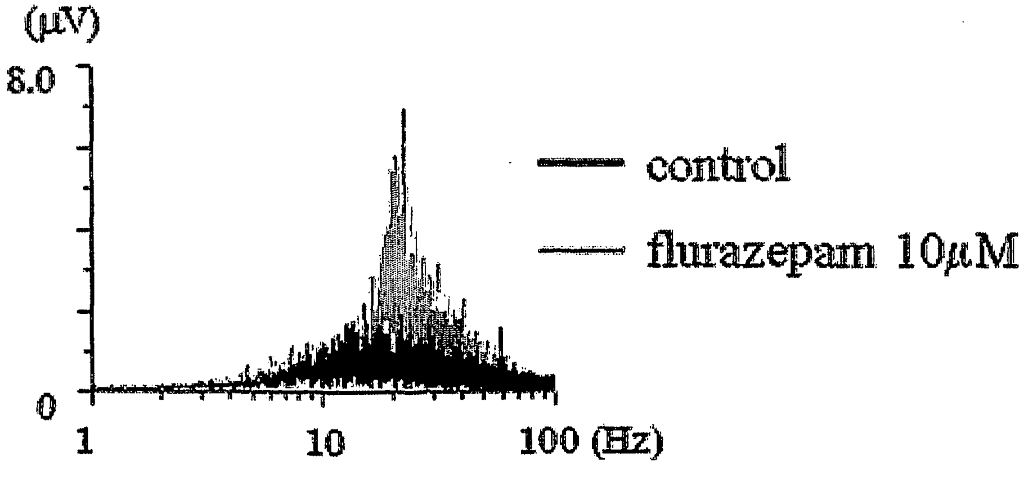

FIG. 21

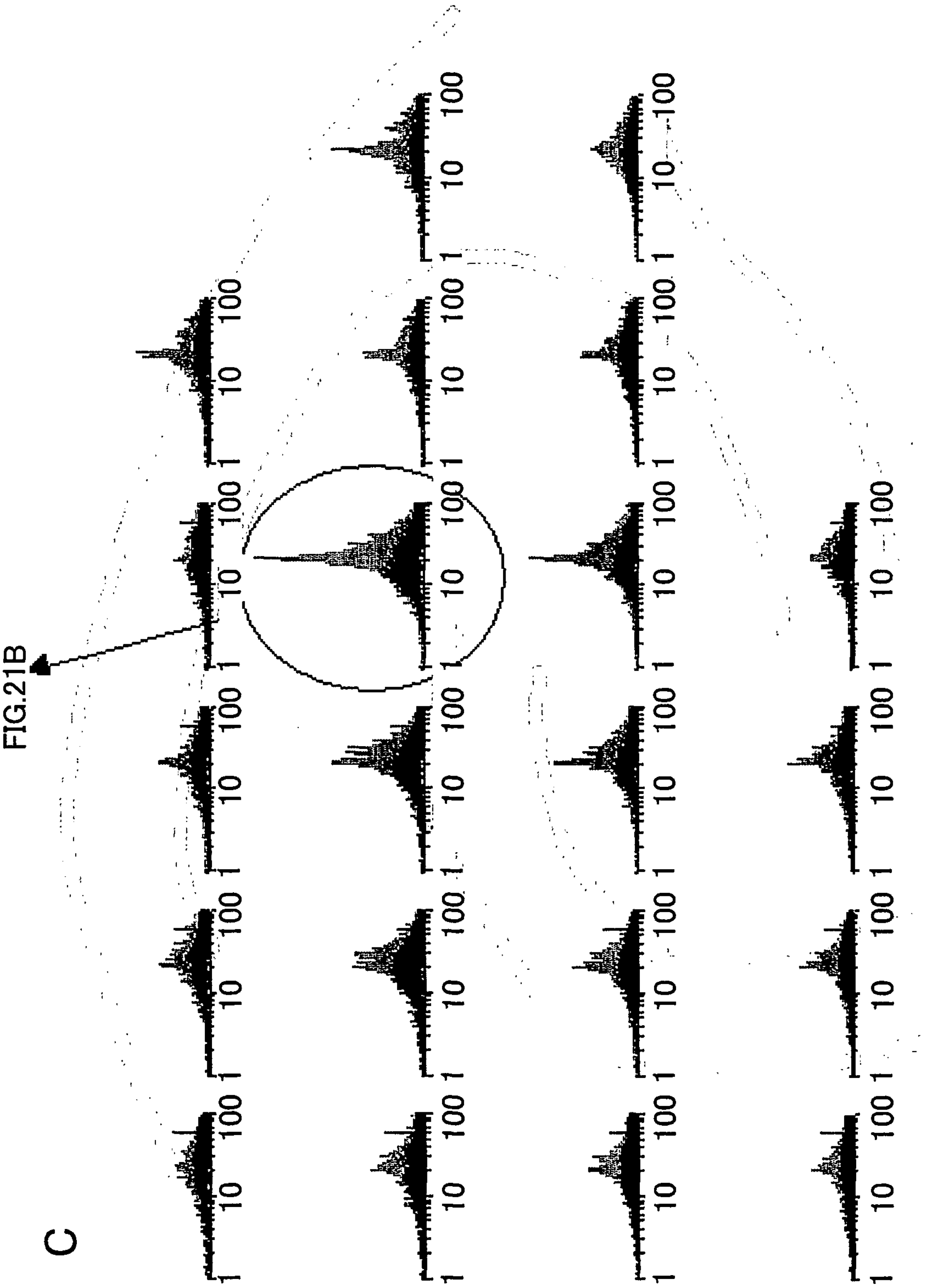

FIG. 22

A

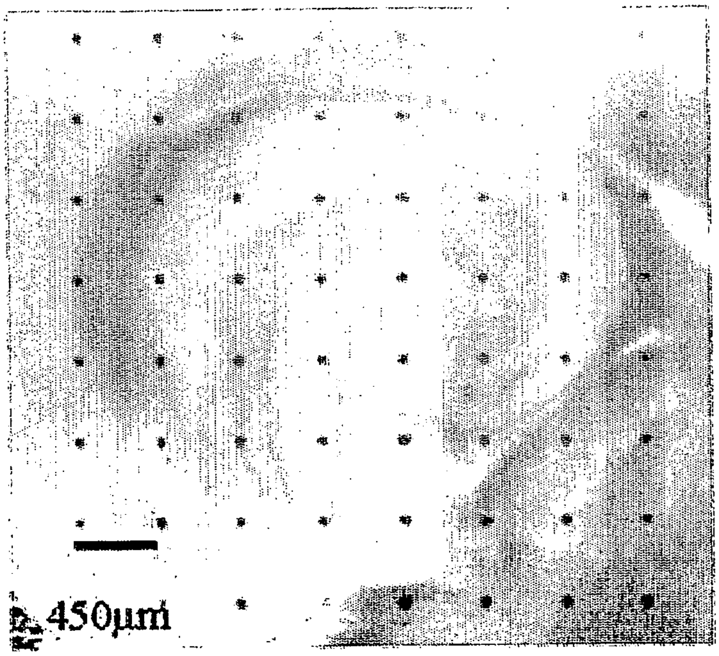


B

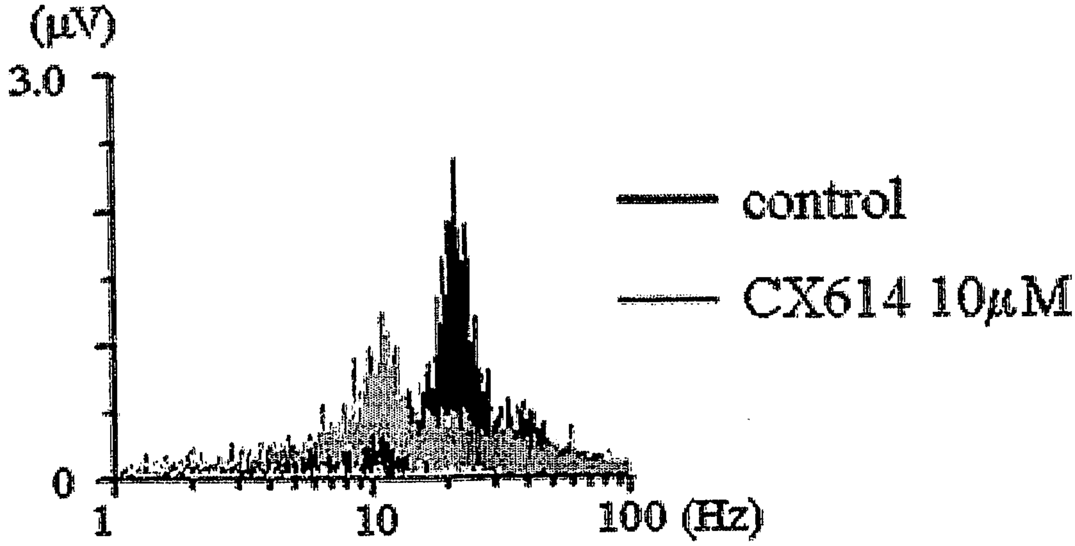

FIG. 22

C

FIG.22B

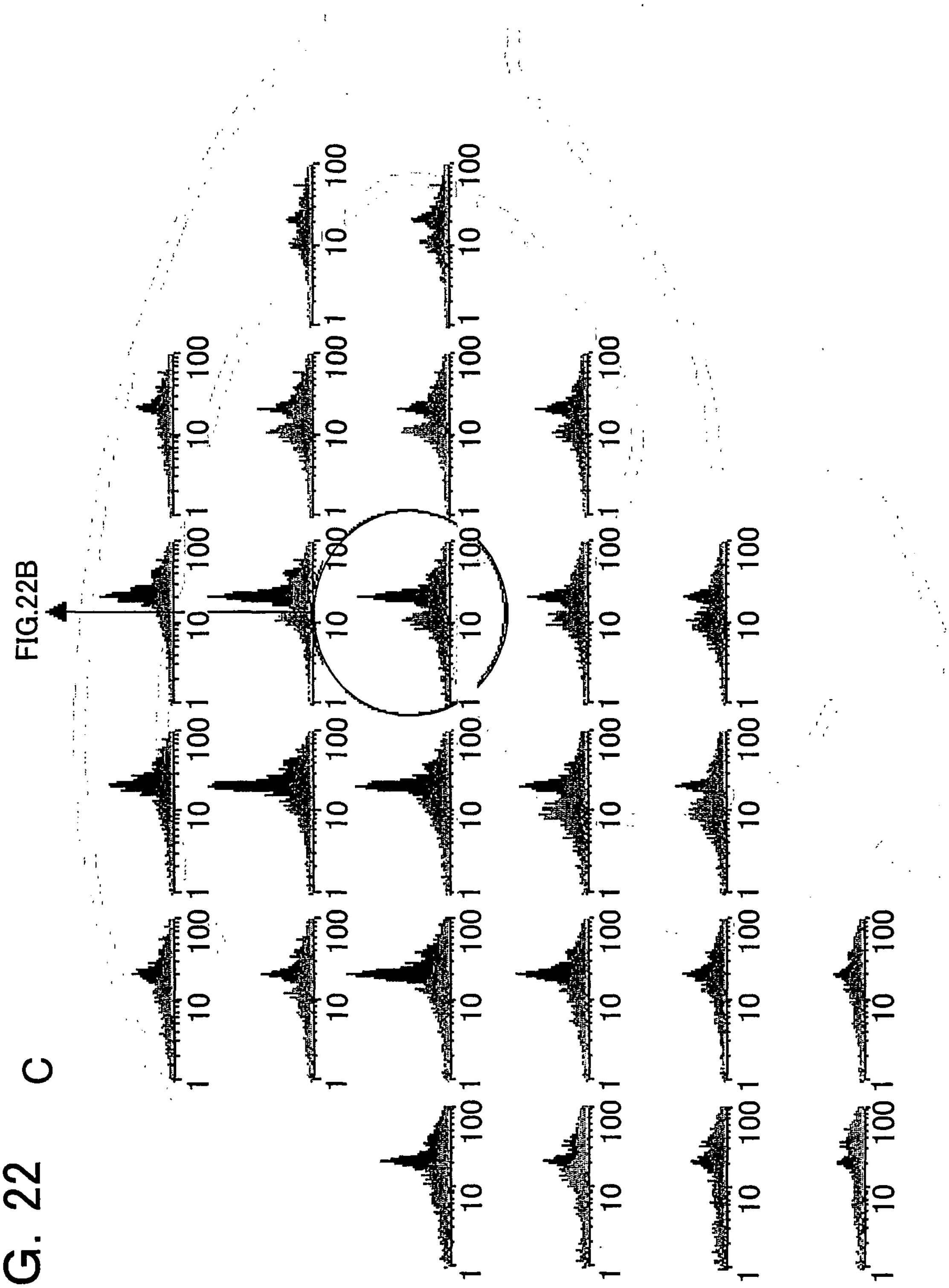

FIG. 23

A

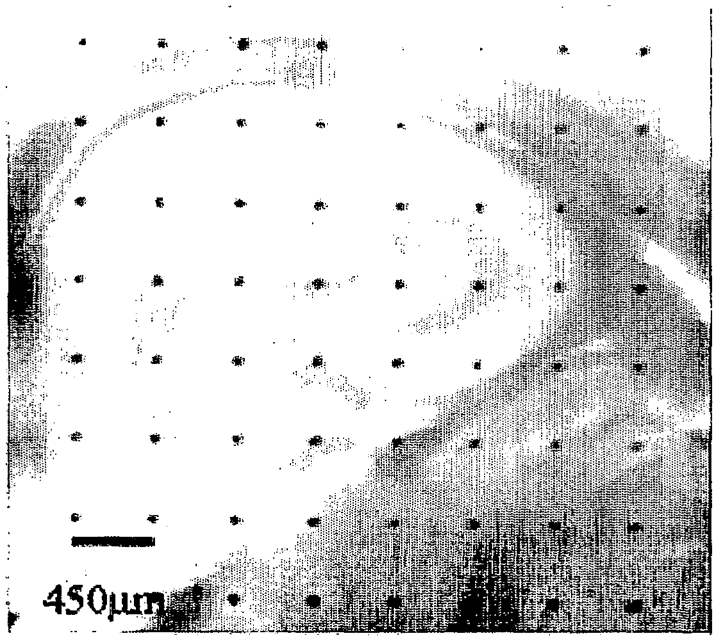


B

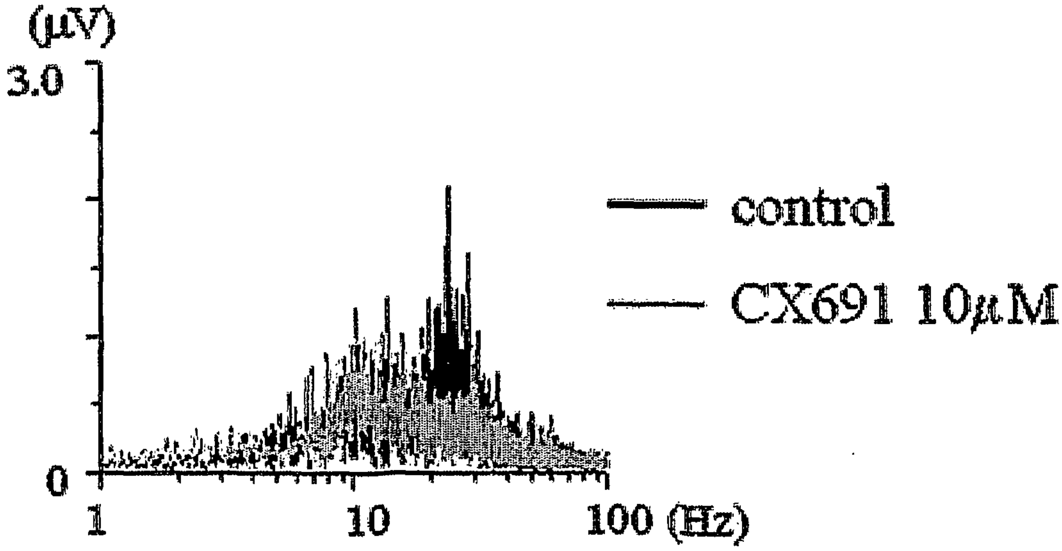

FIG. 23

C

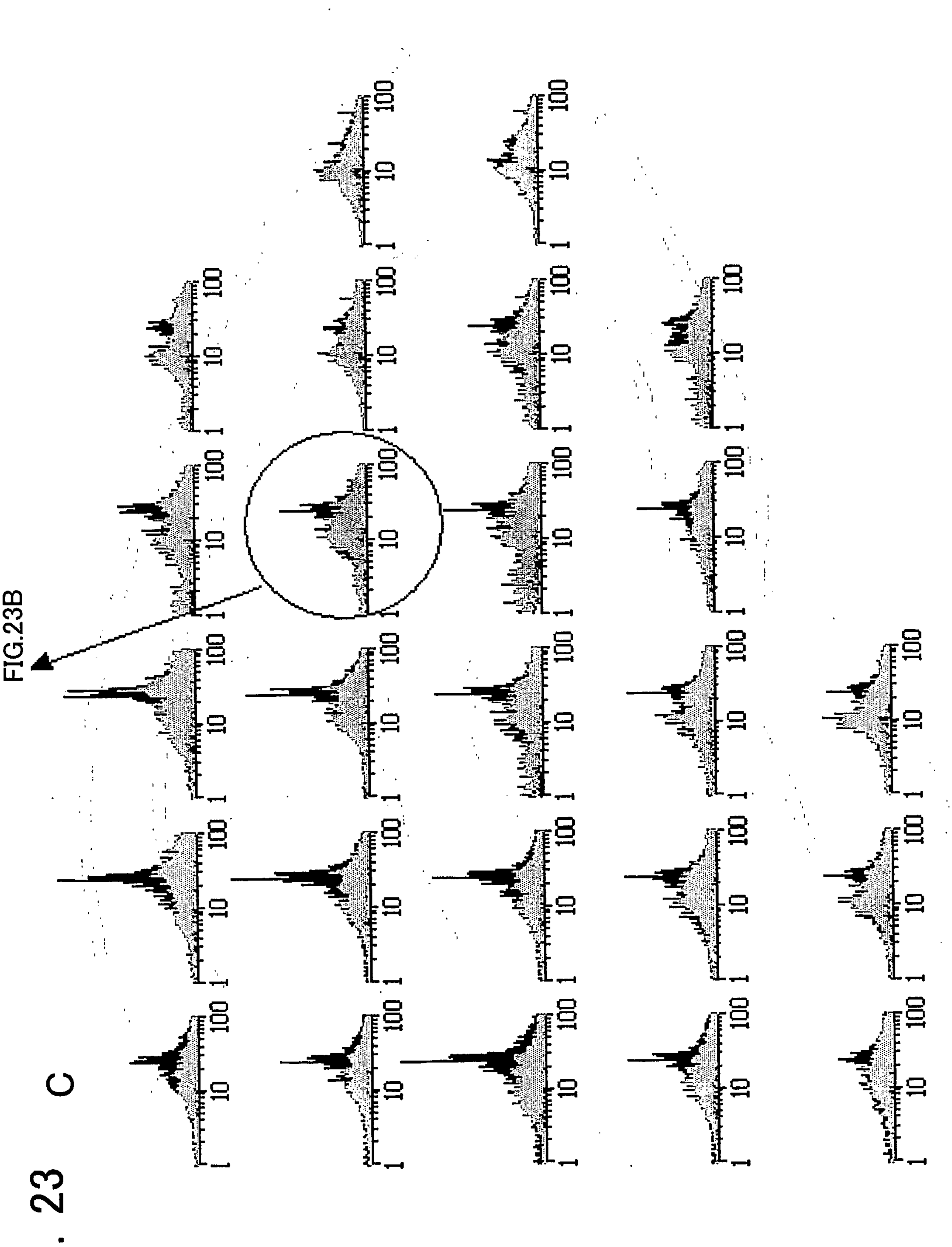

FIG. 24

(A)

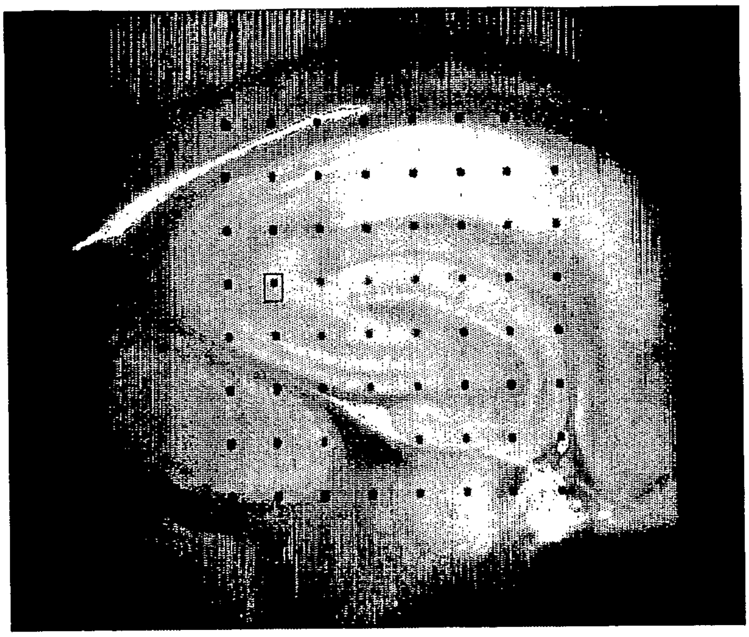

(B)

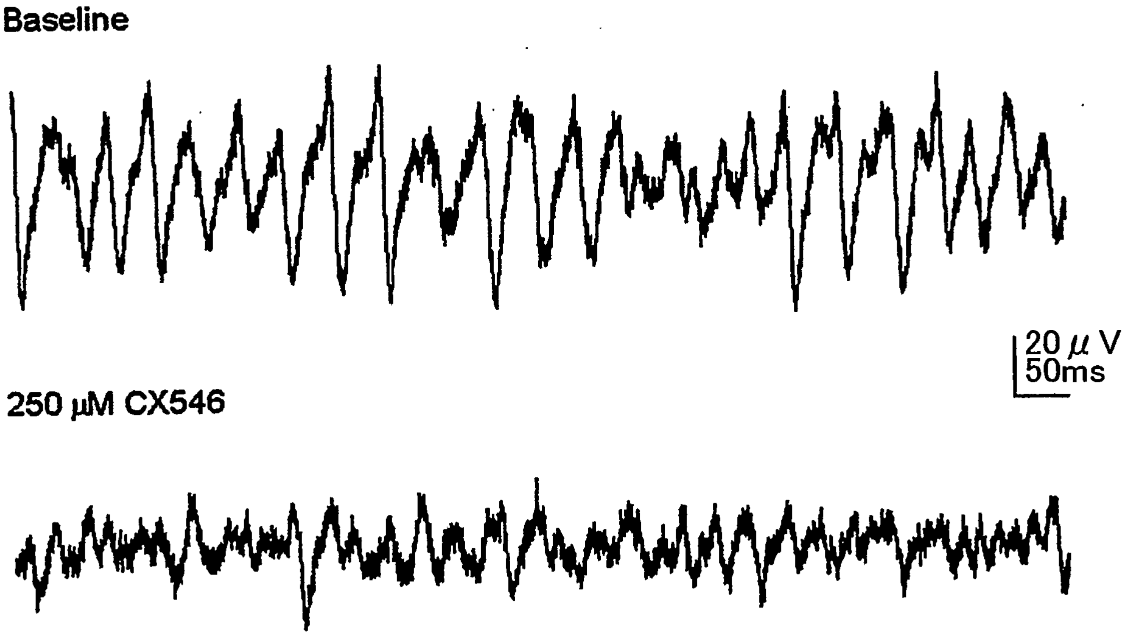

FIG. 25

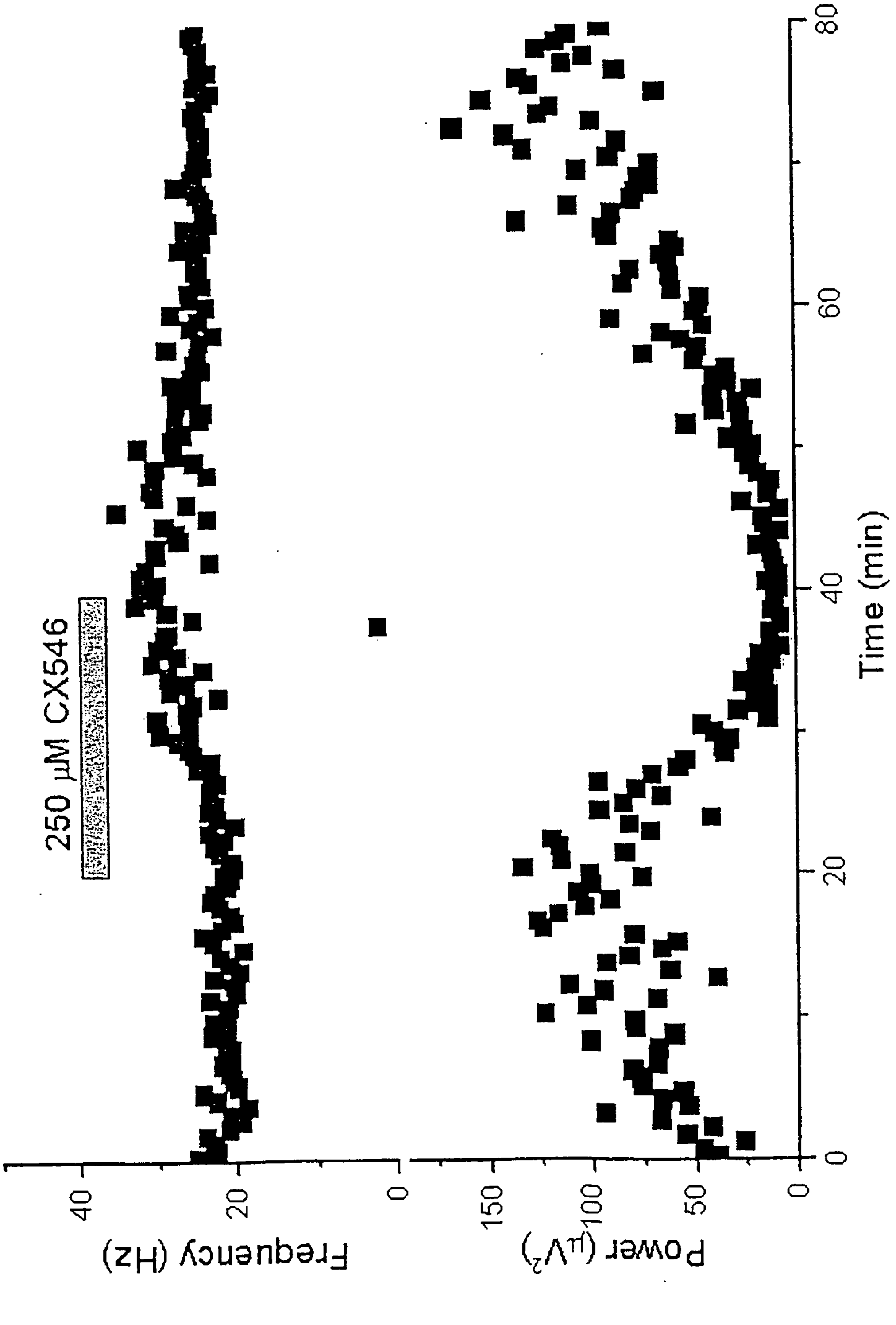
250 µM CX546
Frequency (Hz)
40
20
0
Power (µV²)
150
100
50
0
0
20
40
60
80
Time (min)

FIG. 26

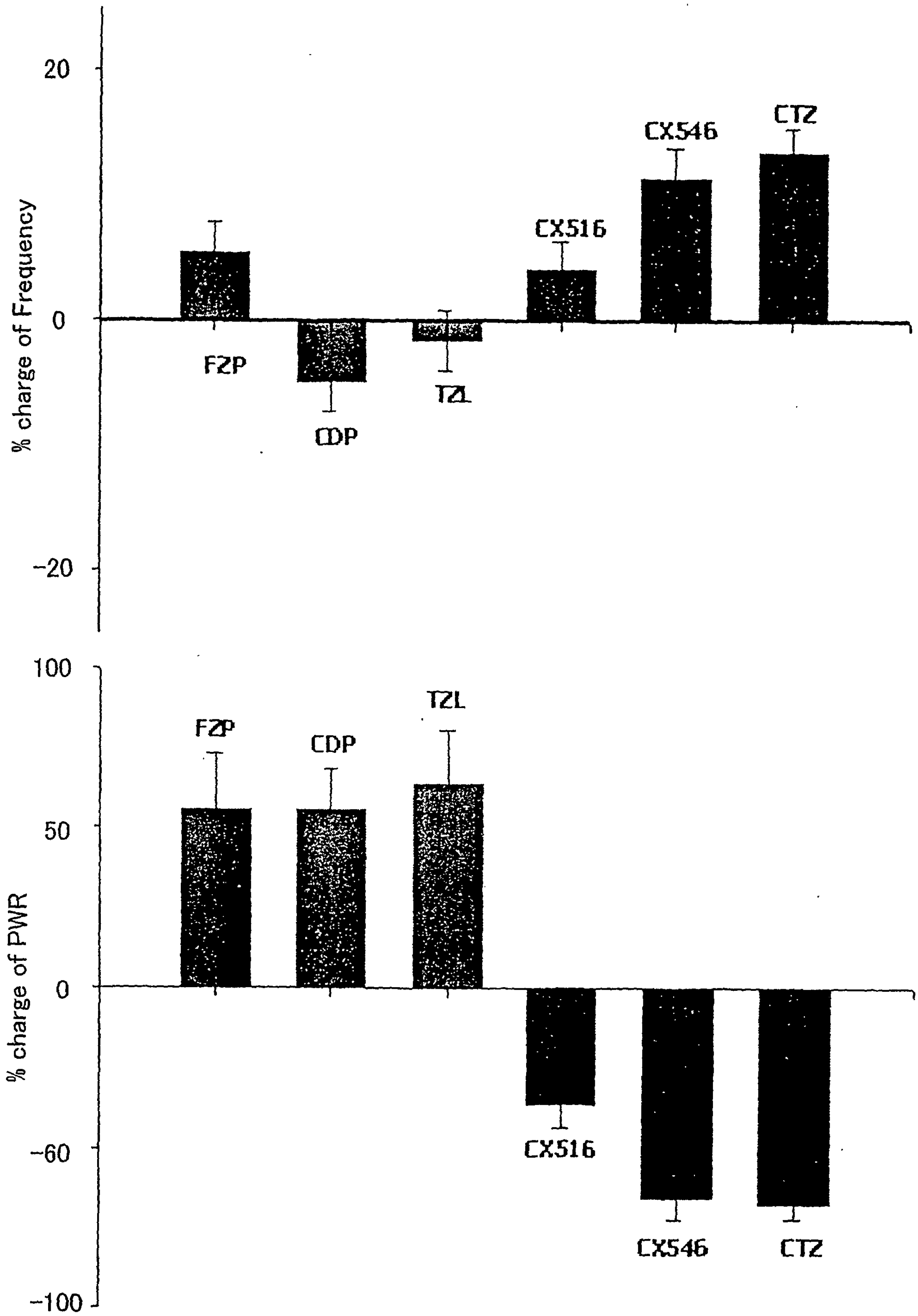
20
0
-20
% charge of Frequency
FZP
CDP
TZL
CX516
CX546
CTZ
100
50
0
-60
-100
% charge of PWR
FZP
CDP
TZL
CX516
CX546
CTZ

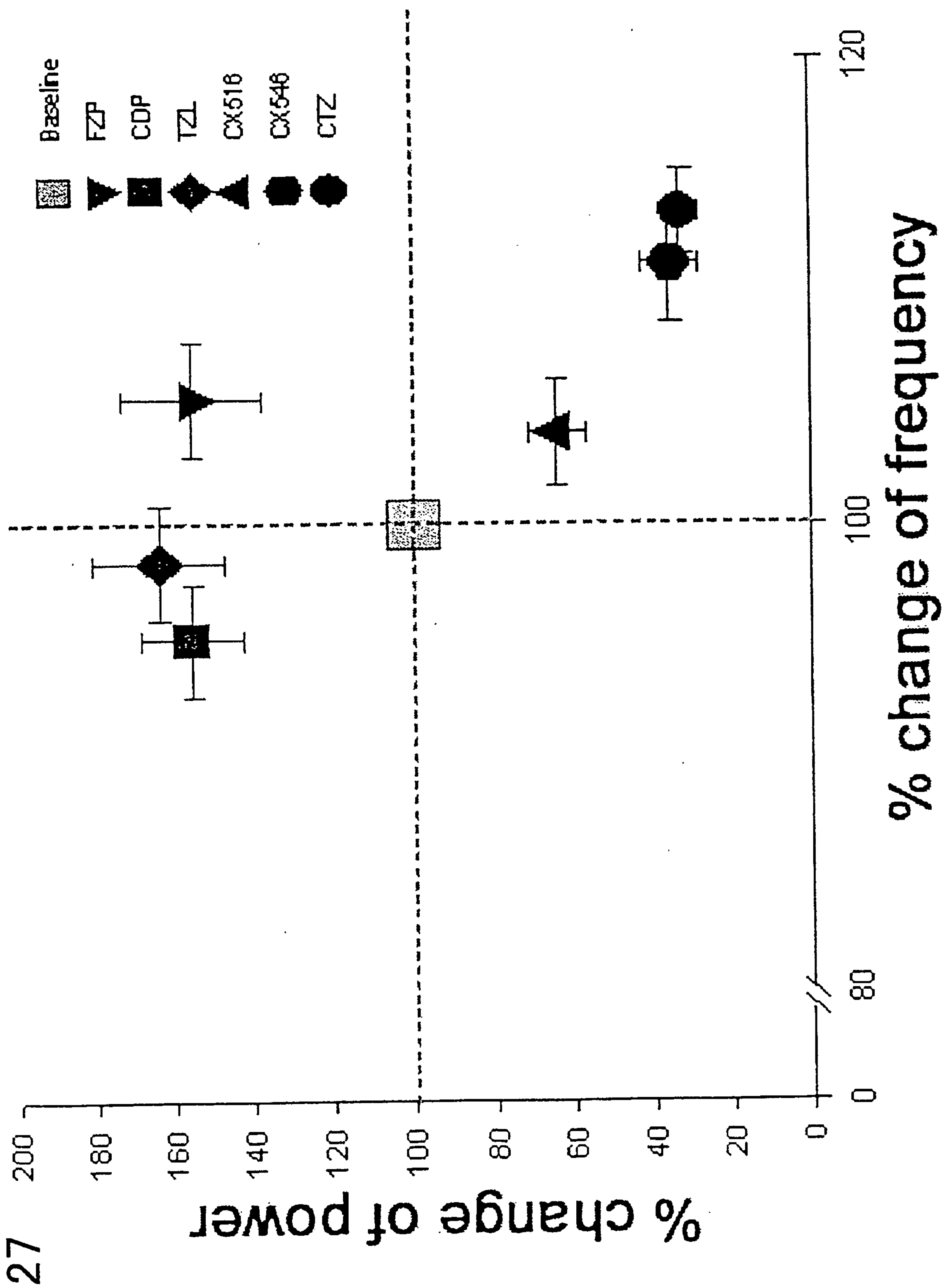
Baseline
FZP
CDP
TZL
CX516
CX546
CTZ
% change of power
200
180
160
140
120
100
80
60
40
20
0
% change of frequency
0
80
100
120

FIG. 27

FIG. 28

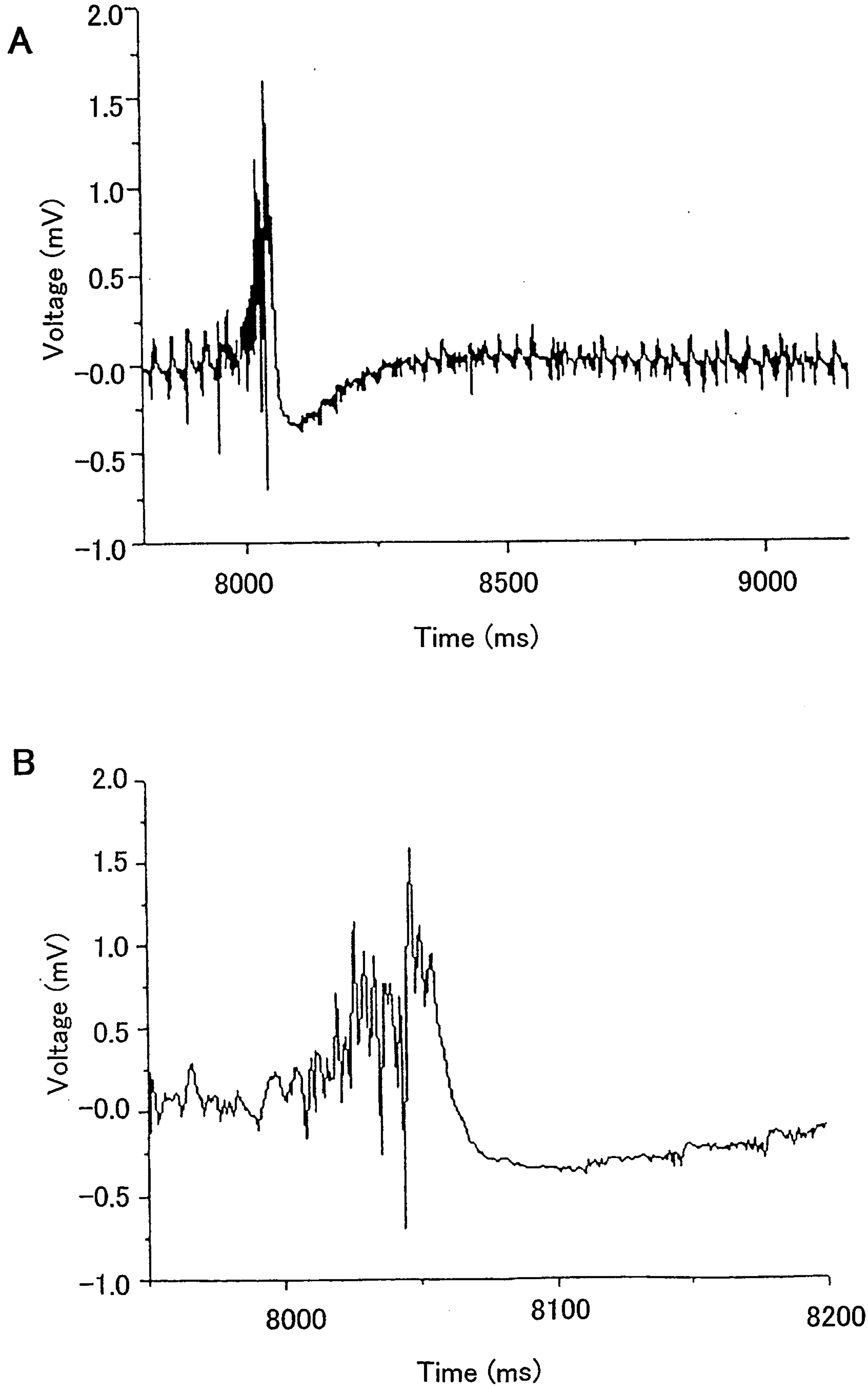
A
2.0
1.5
1.0
0.5
-0.0
-0.5
-1.0
Voltage (mV)
8000
8500
9000
Time (ms)
B
2.0
1.5
1.0
0.5
-0.0
-0.5
-1.0
Voltage (mV)
8000
8100
8200
Time (ms)

FIG. 29

A

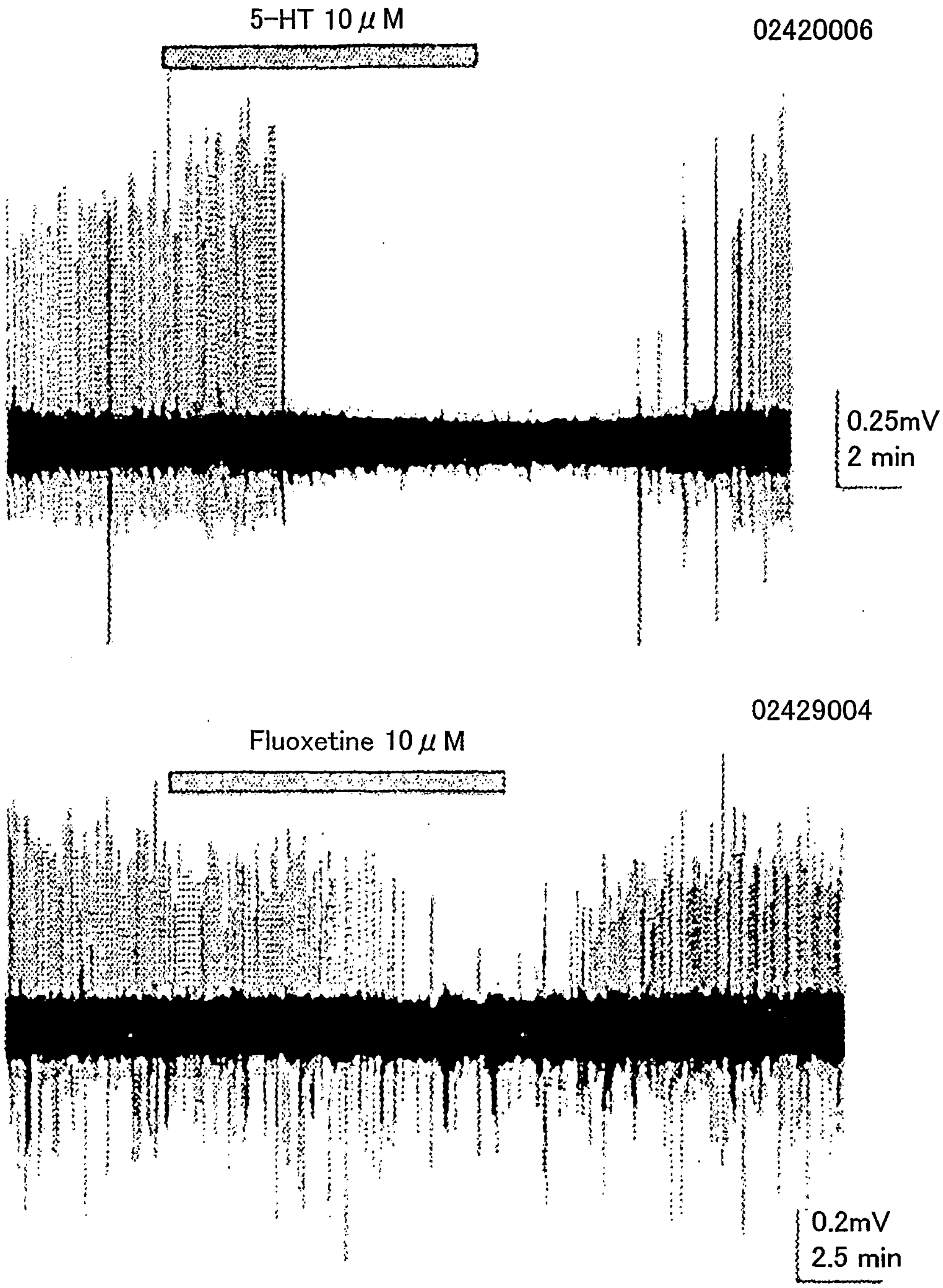
5-HT 10 μM
02420006
0.25mV
2 min
02429004
Fluoxetine 10 μM
0.2mV
2.5 min

FIG. 29 B

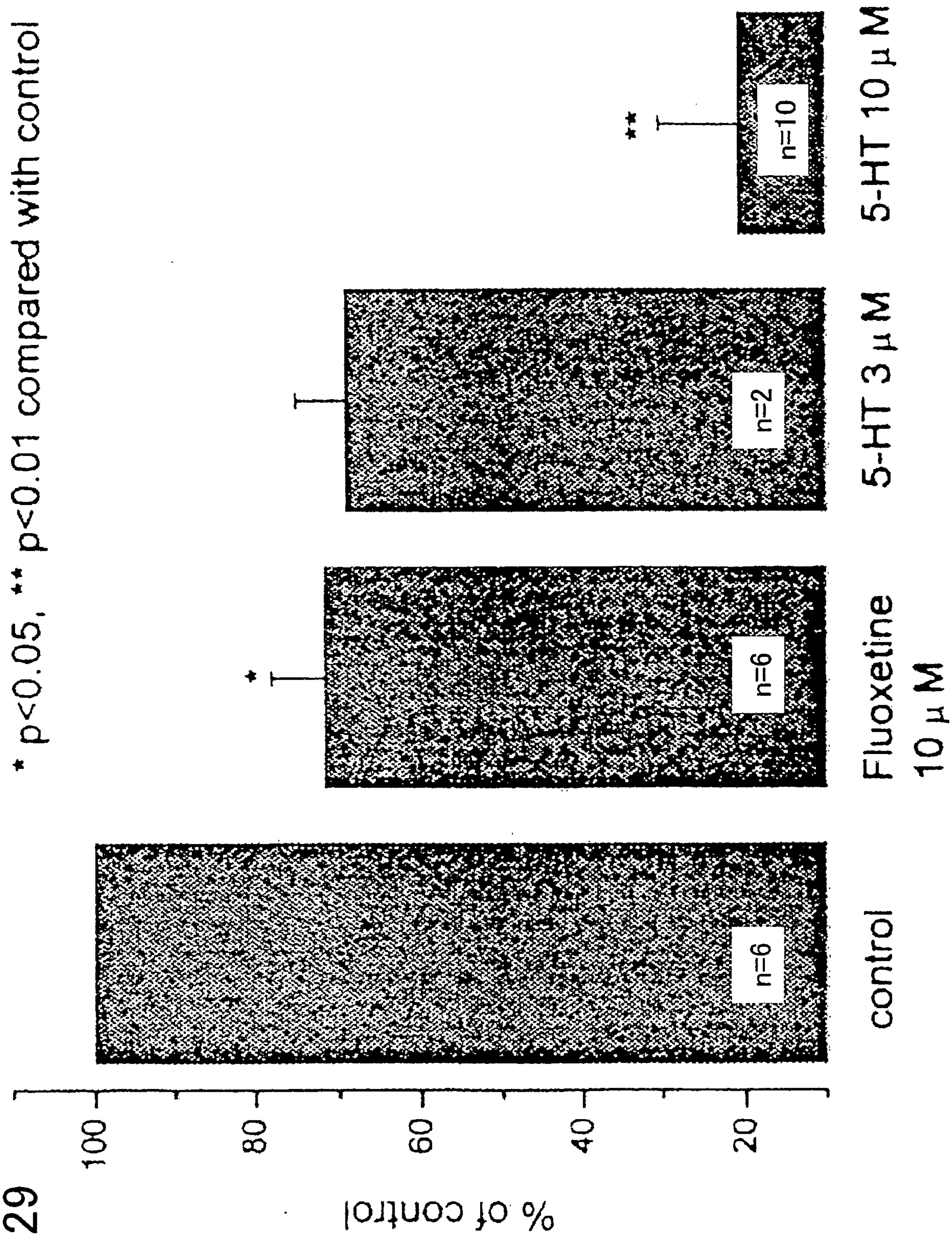
* p<0.05, ** p<0.01 compared with control
% of control
100
80
60
40
20
n=6
n=6
n=2
n=10
*
**
control
Fluoxetine 10 μ M
5-HT 3 μ M
5-HT 10 μ M

FIG. 30

A

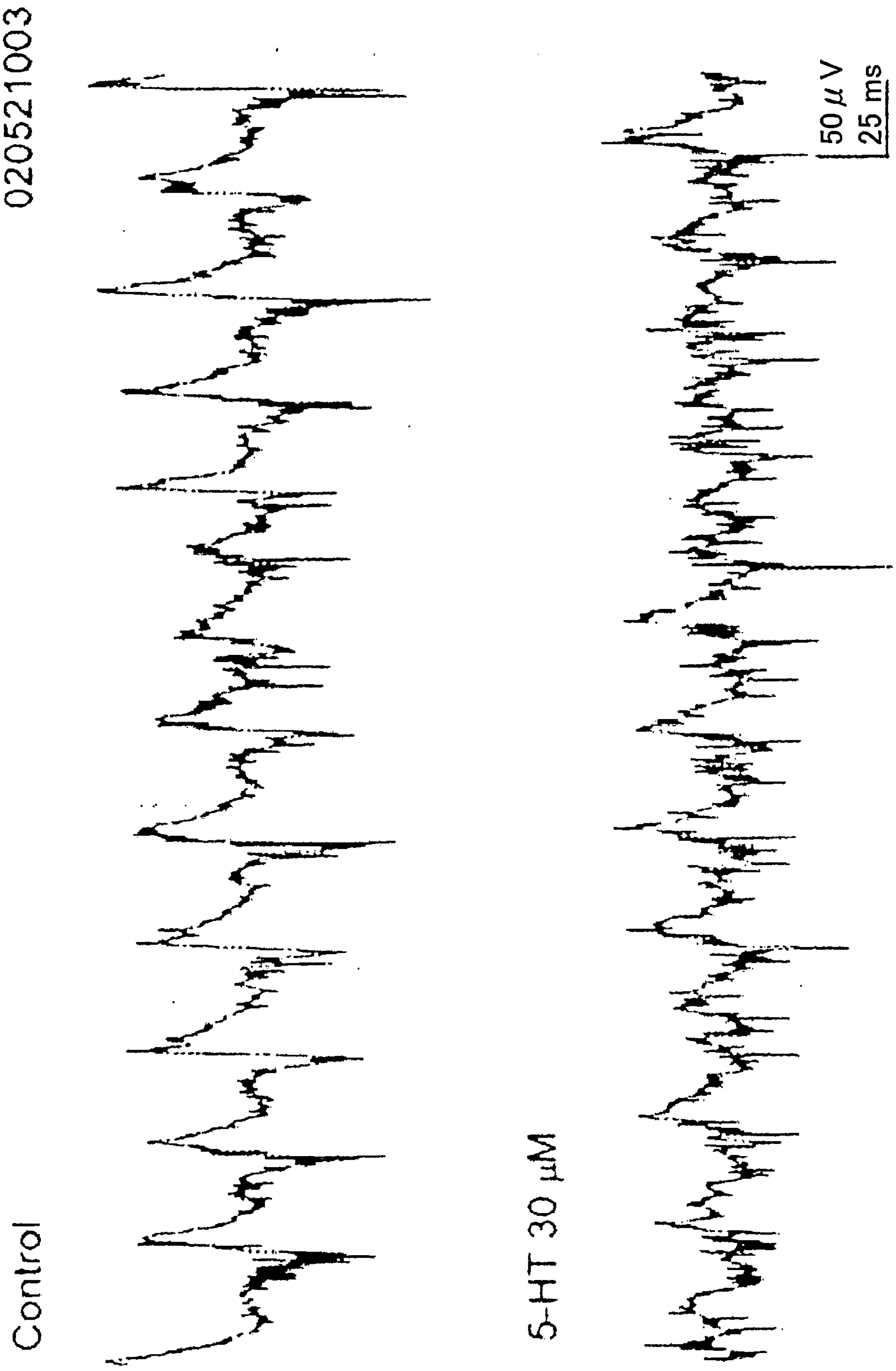

FIG. 30
B

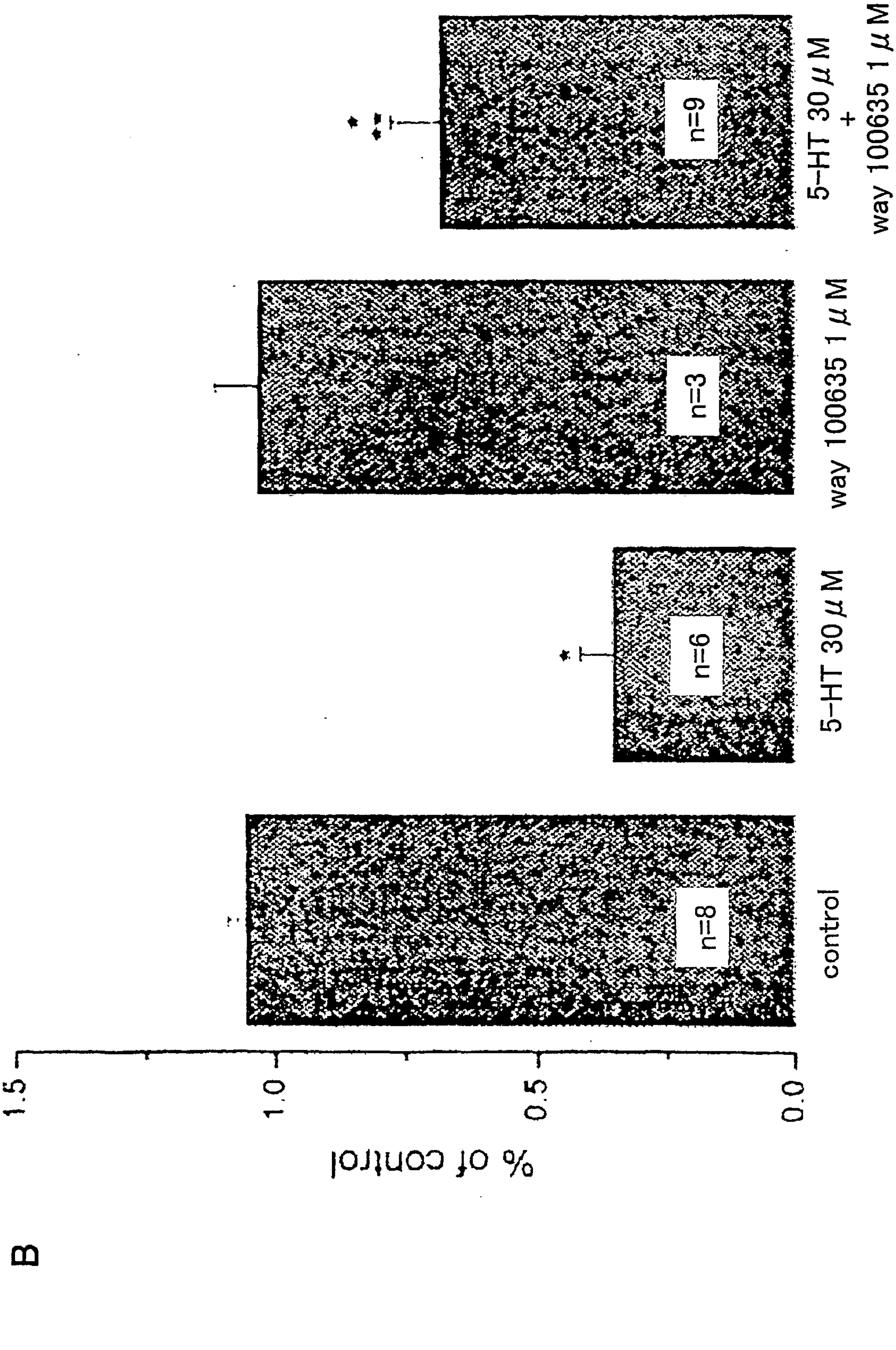
% of control
1.5
1.0
0.5
0.0
n=8
n=6
n=3
n=9
*
**
control
5-HT 30 μM
way 100635 1 μM
5-HT 30 μM
+
way 100635 1 μM

FIG. 31

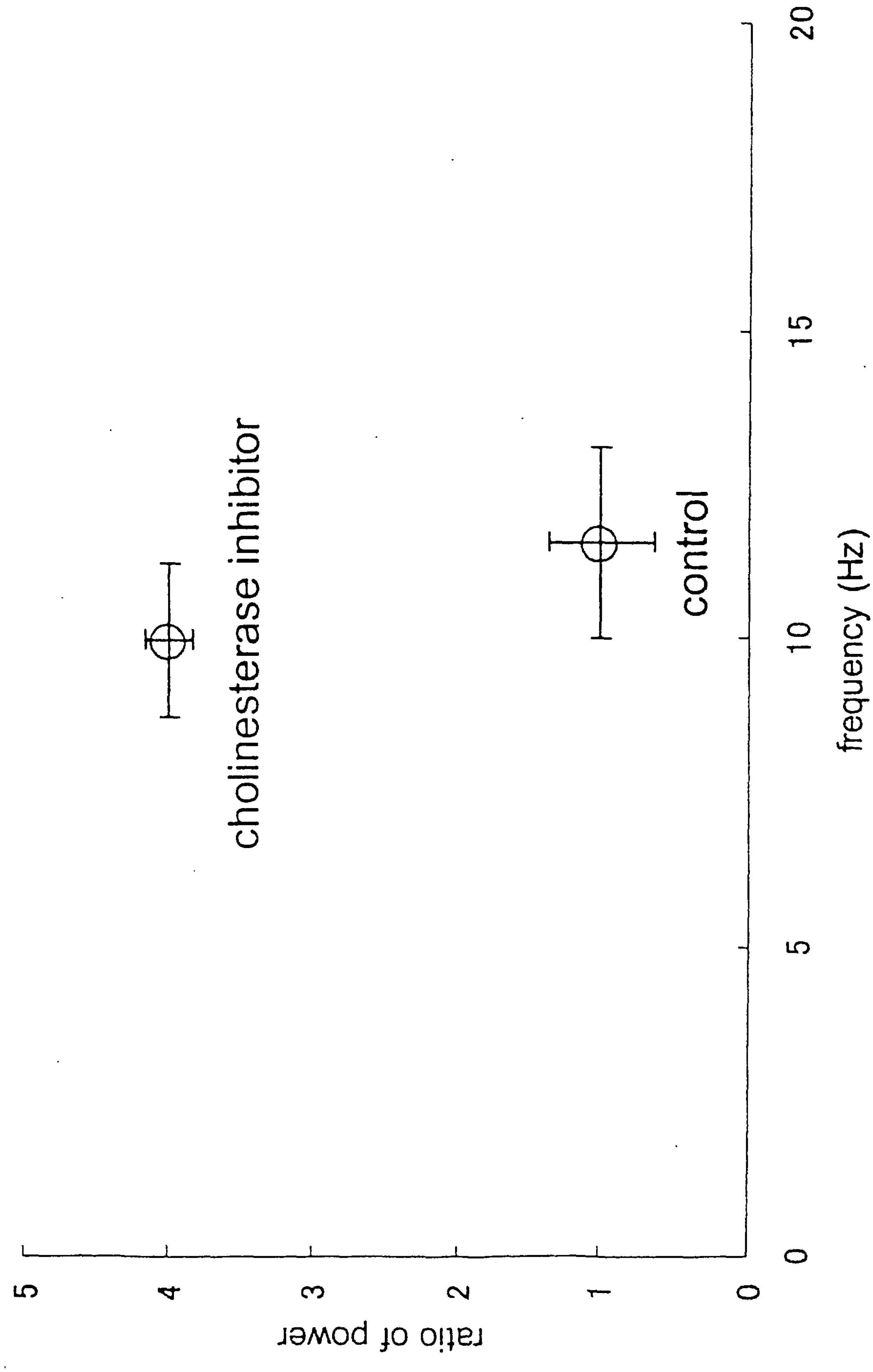
ratio of power
0
1
2
3
4
5
frequency (Hz)
0
5
10
15
20
cholinesterase inhibitor
control

FIG. 32

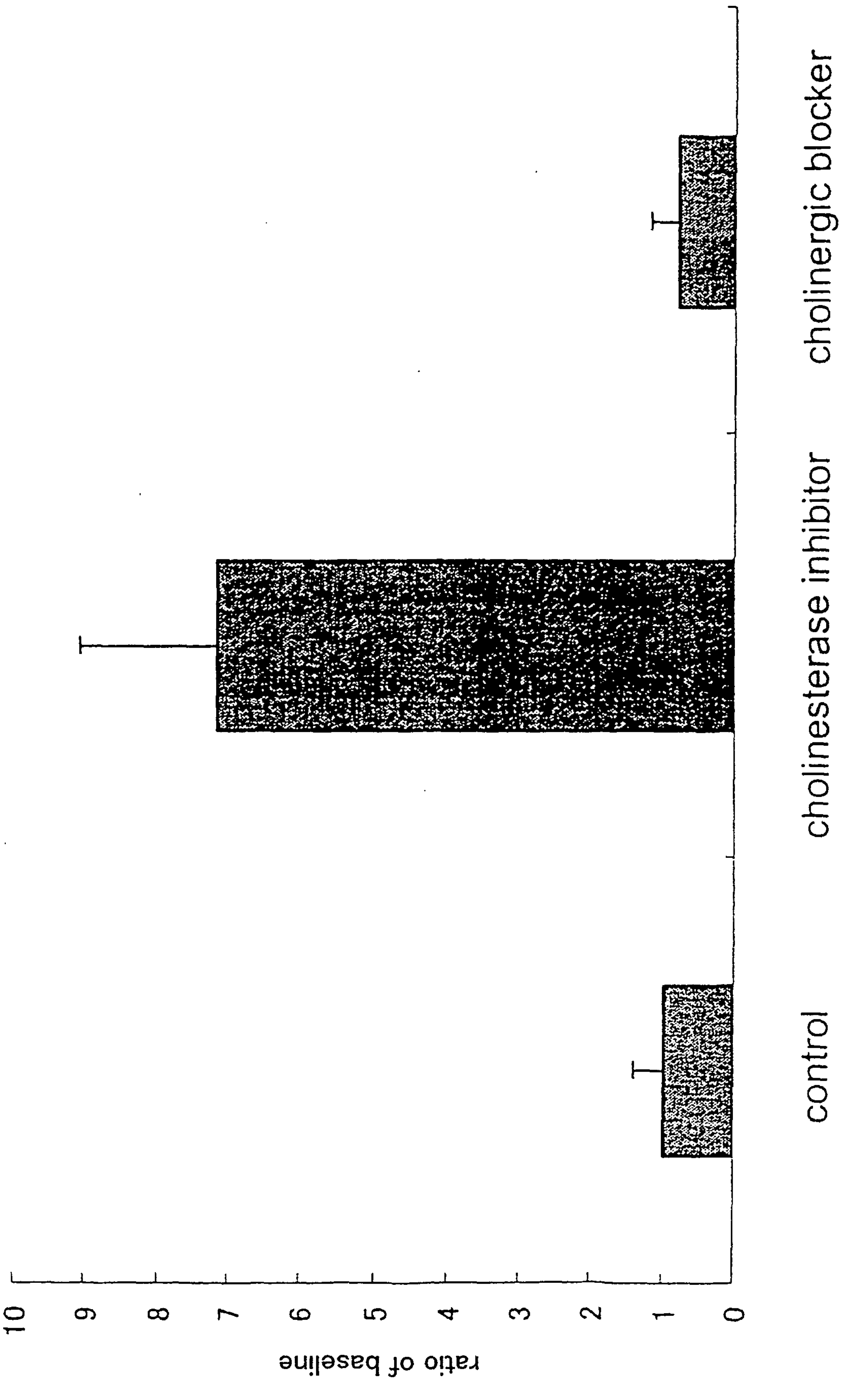
ratio of baseline
10
9
8
7
6
5
4
3
2
1
0
control
cholinesterase inhibitor
cholinergic blocker

FIG. 33

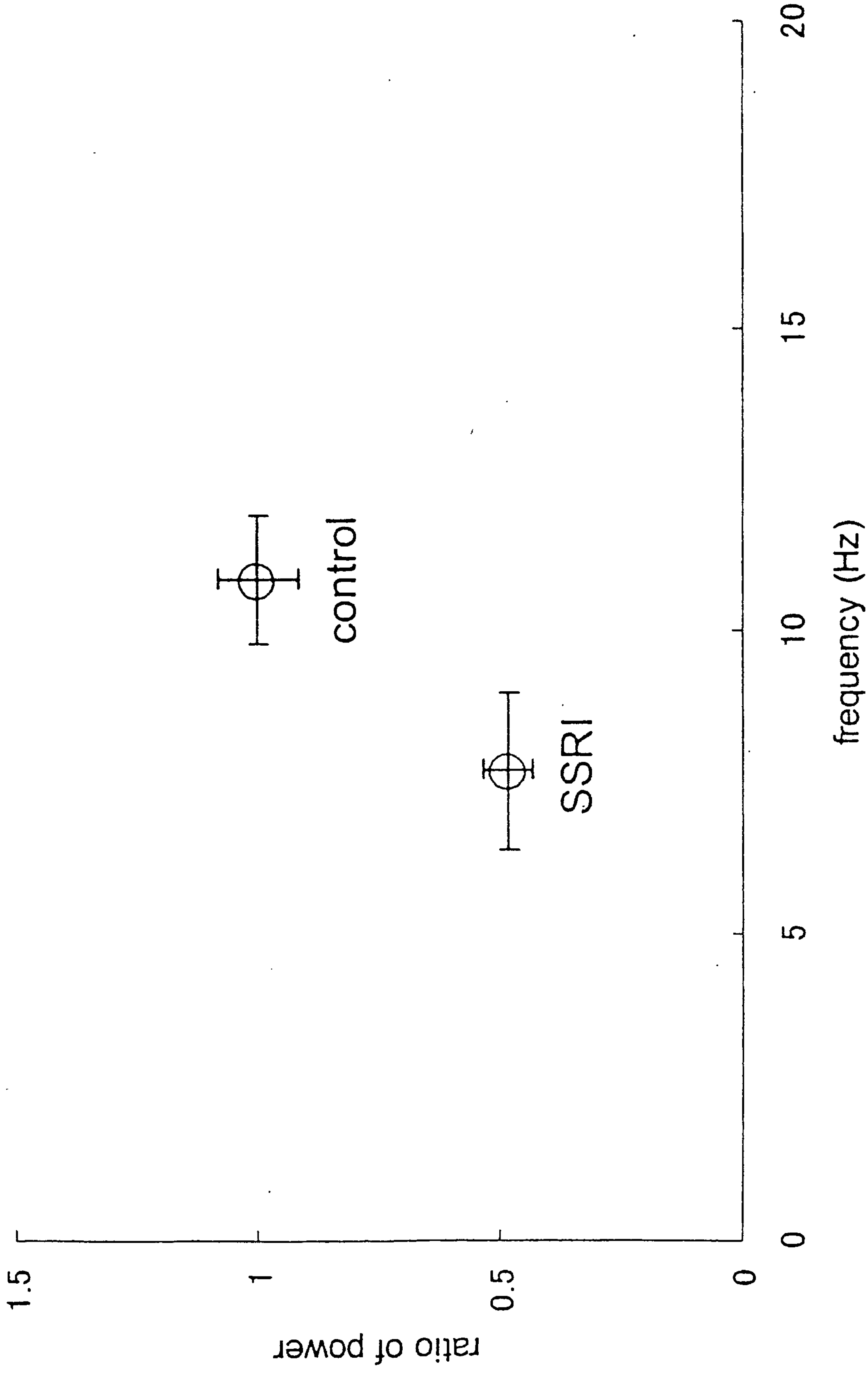

FIG. 34

A

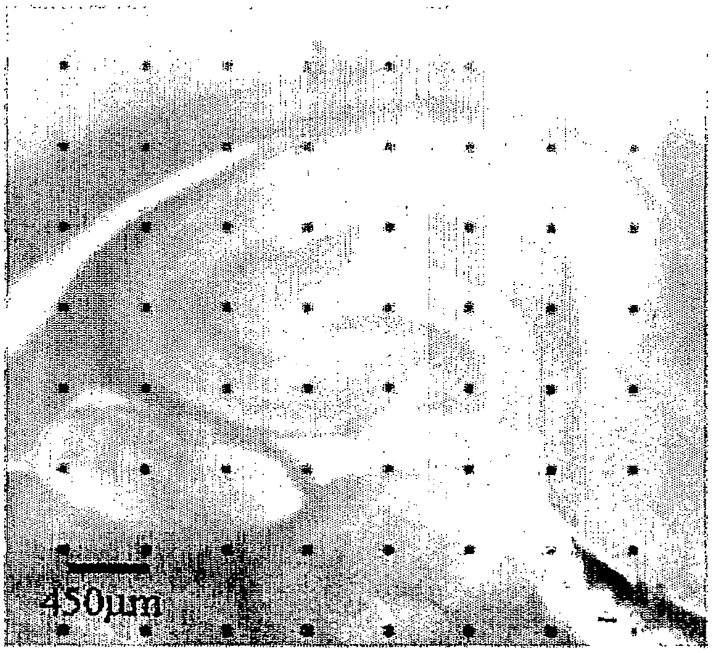


B

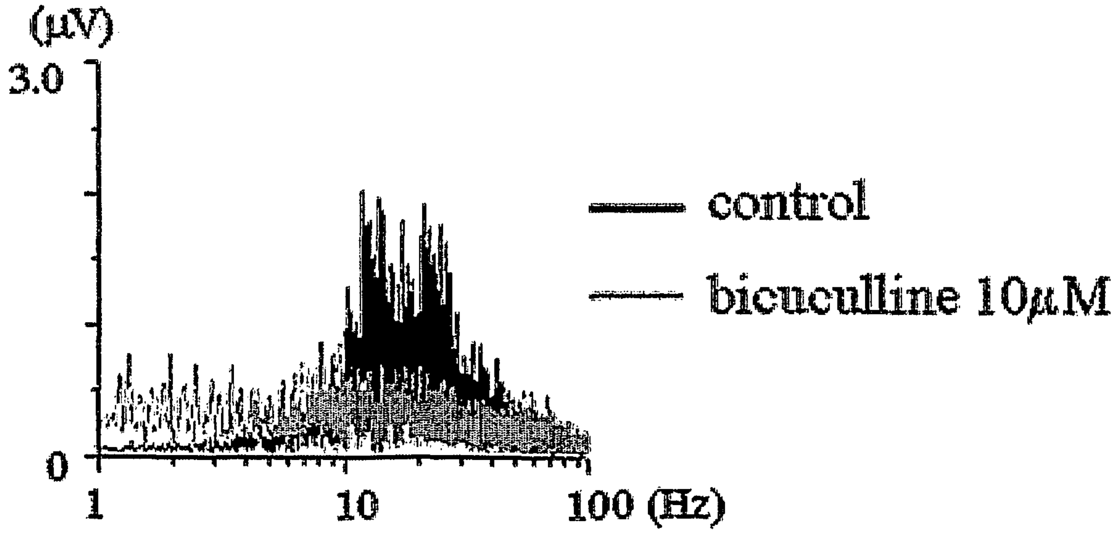

FIG. 34

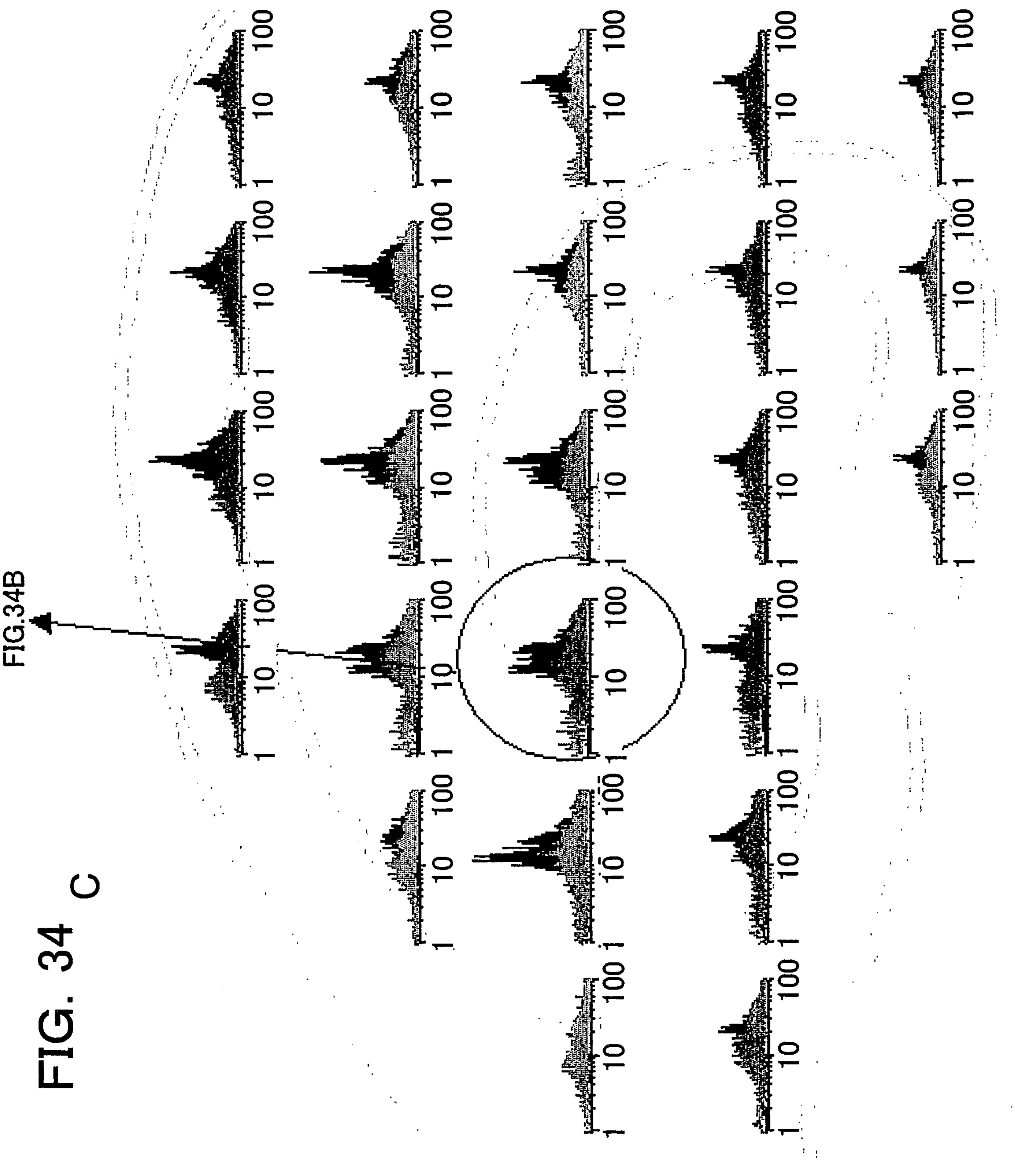

FIG. 35

A

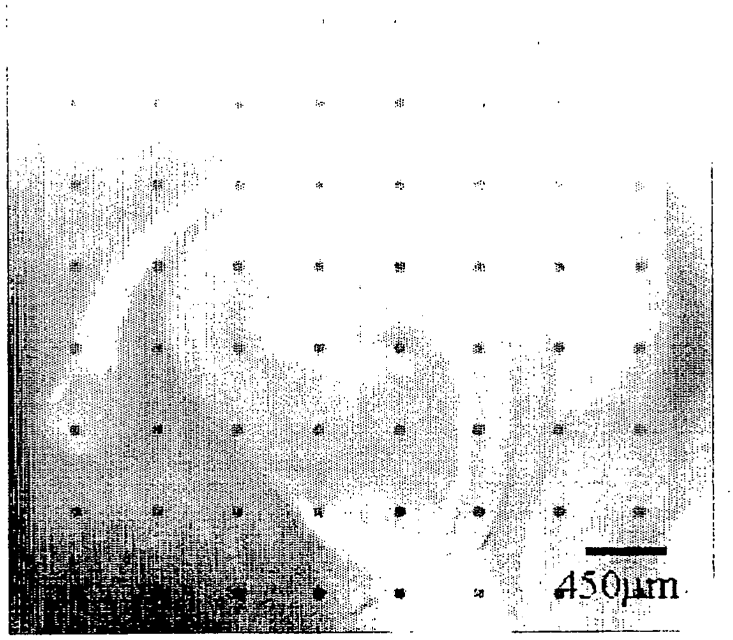


B

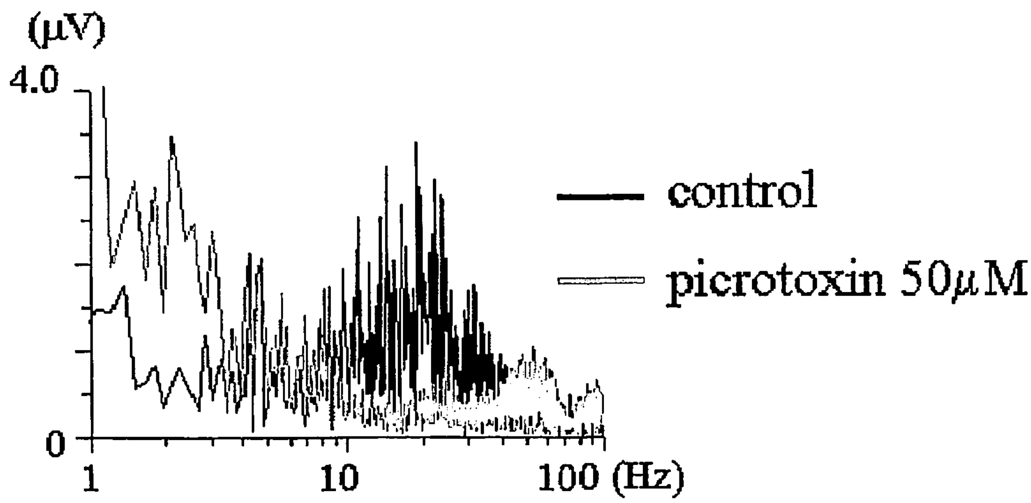

FIG. 35

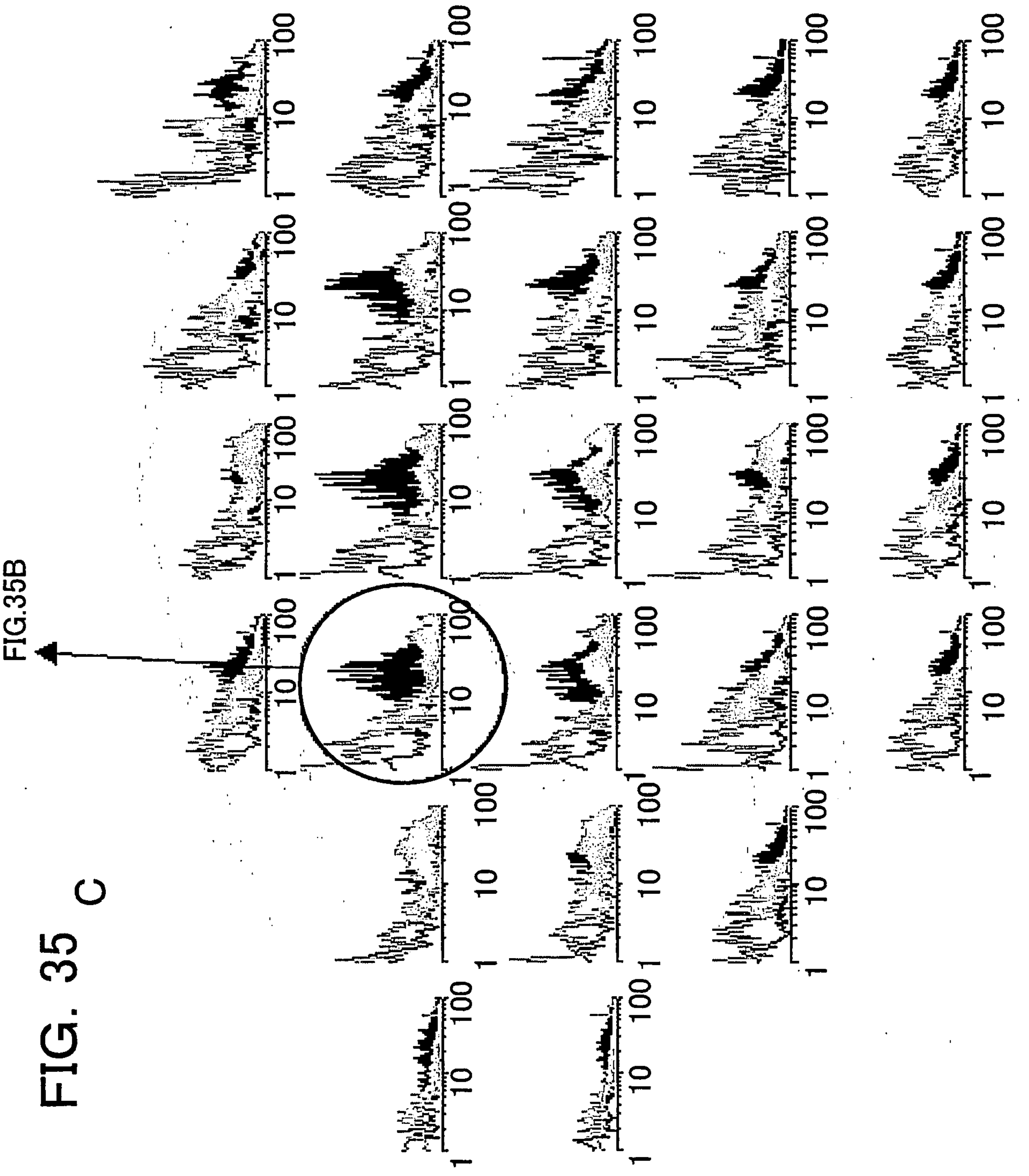

FIG. 36

A

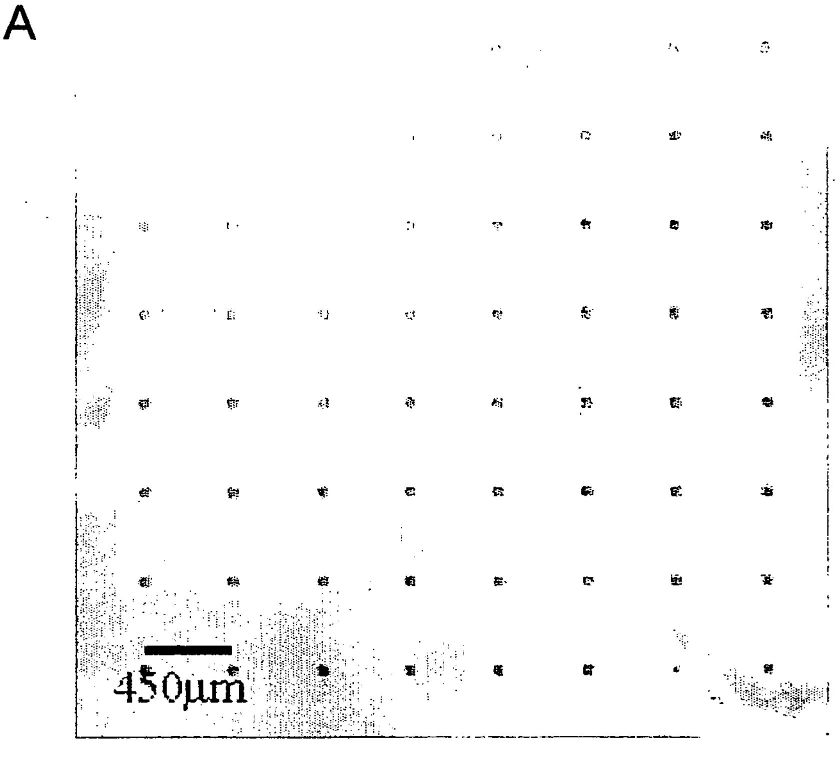


B

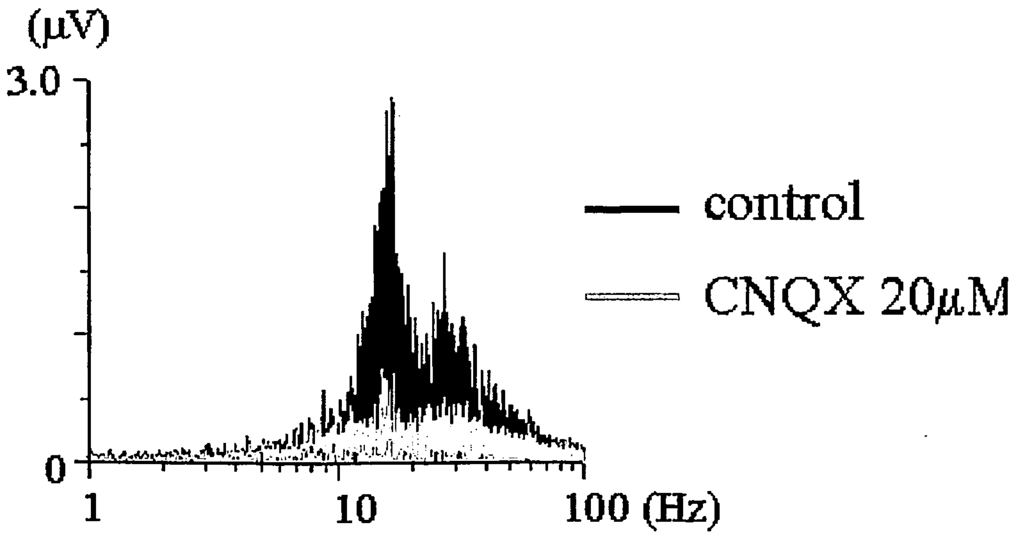

FIG. 36 C

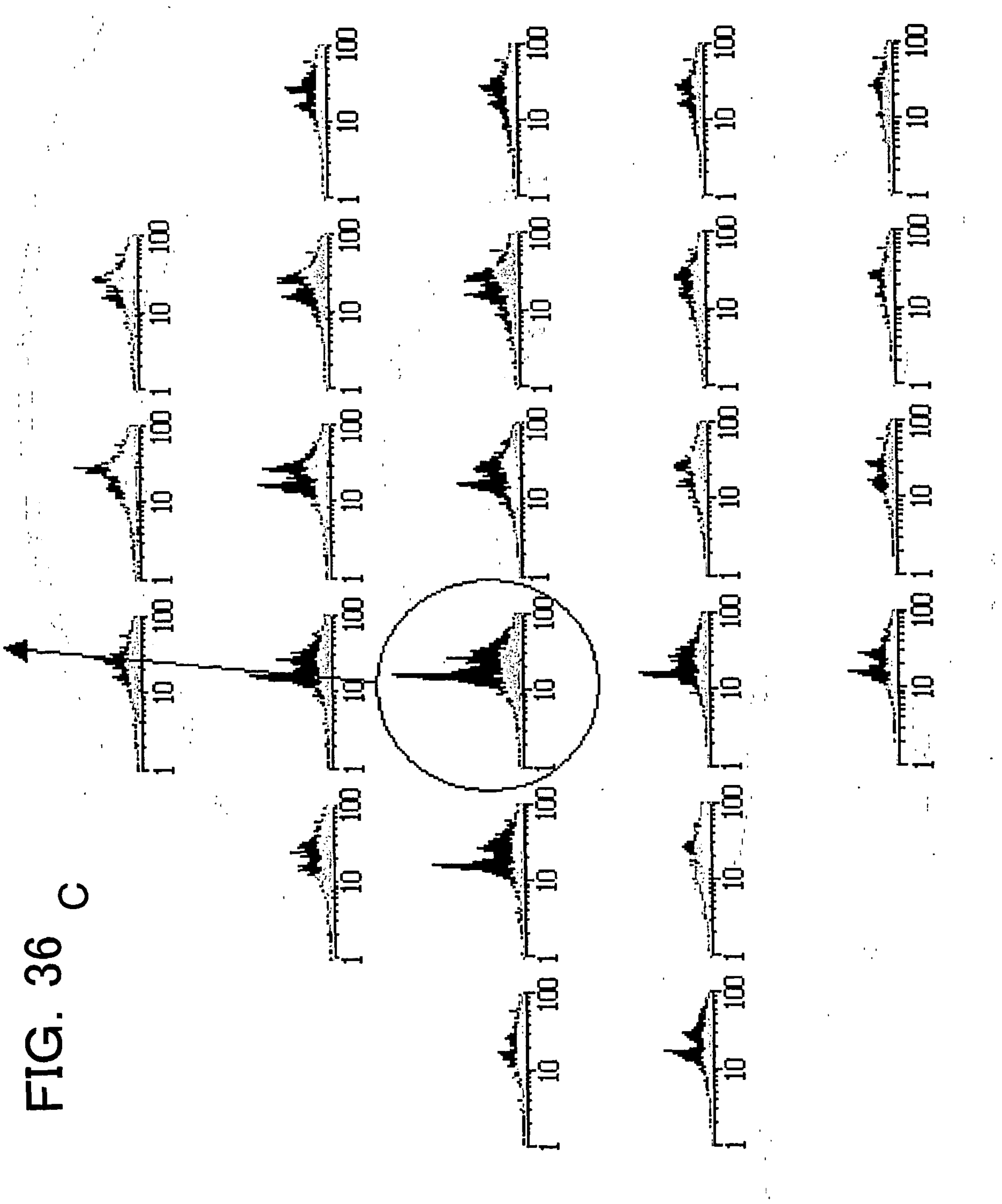

FIG. 37

A

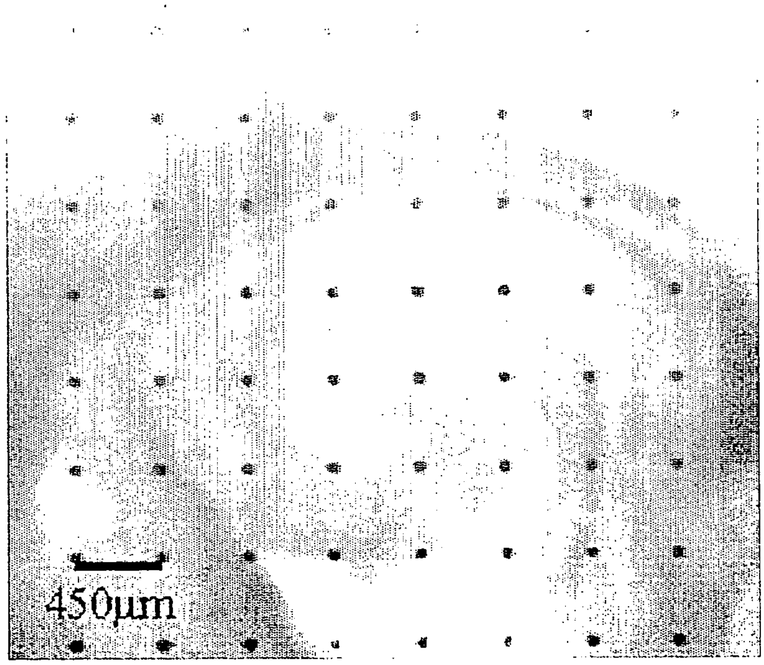


B

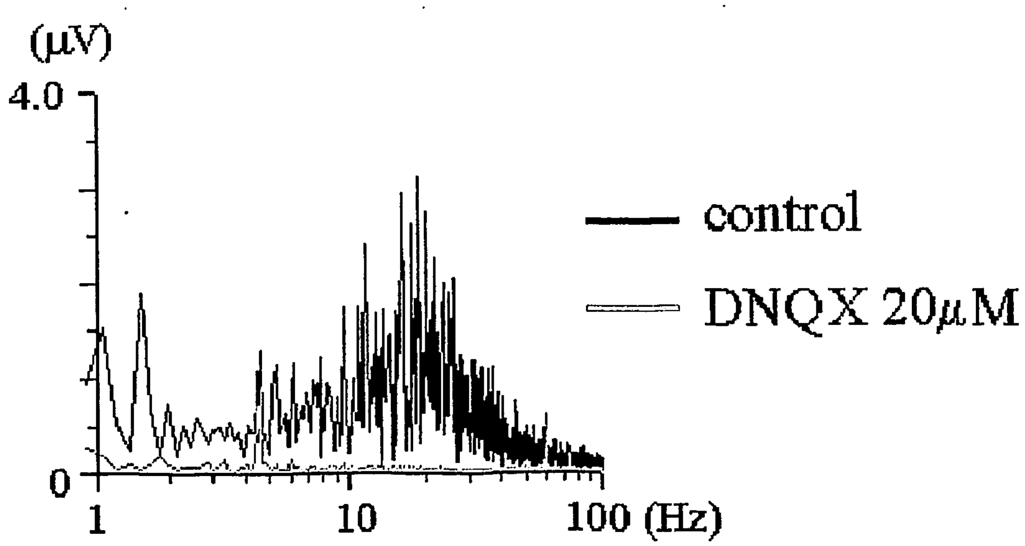

FIG. 37

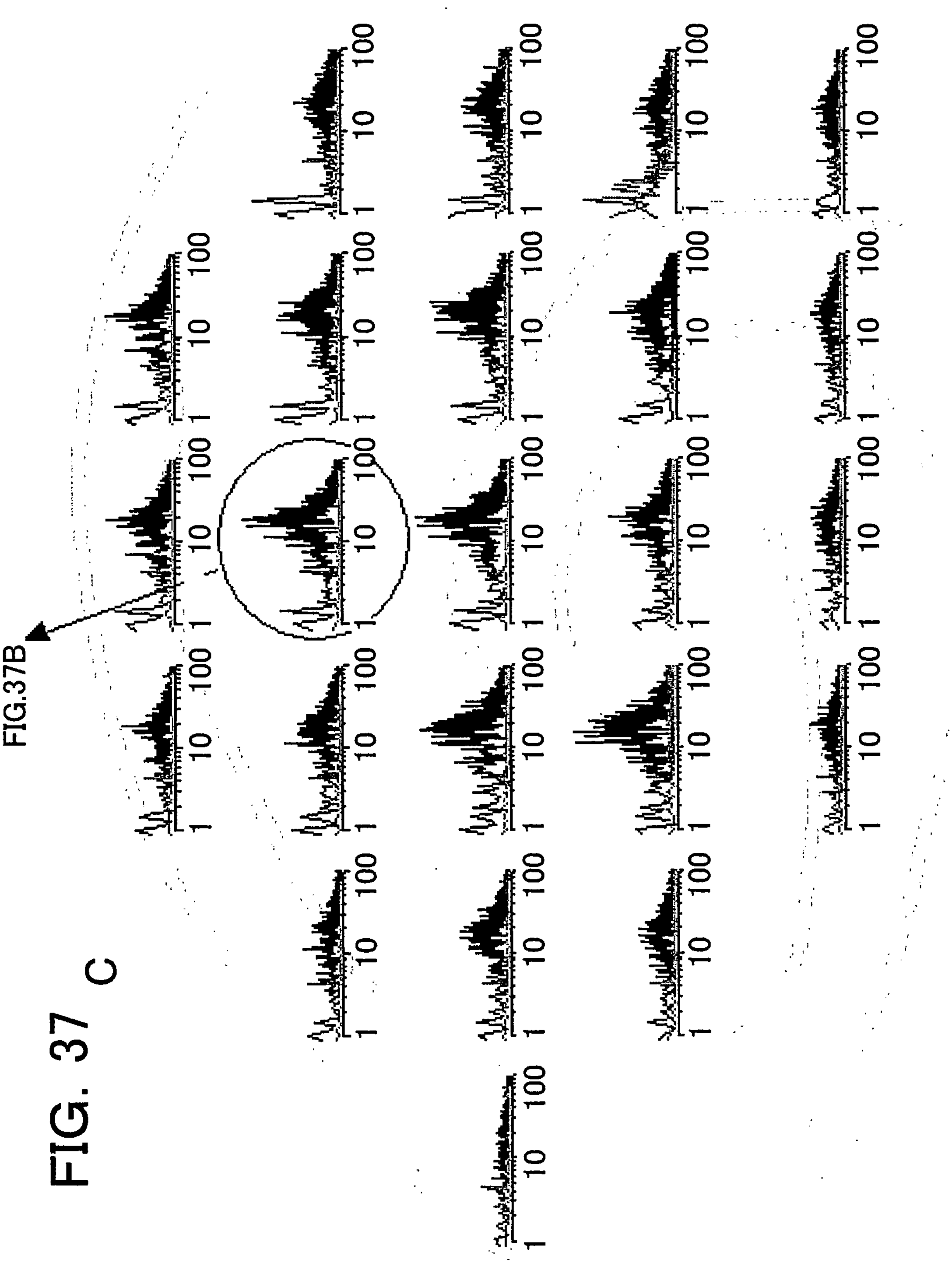

FIG. 38

A

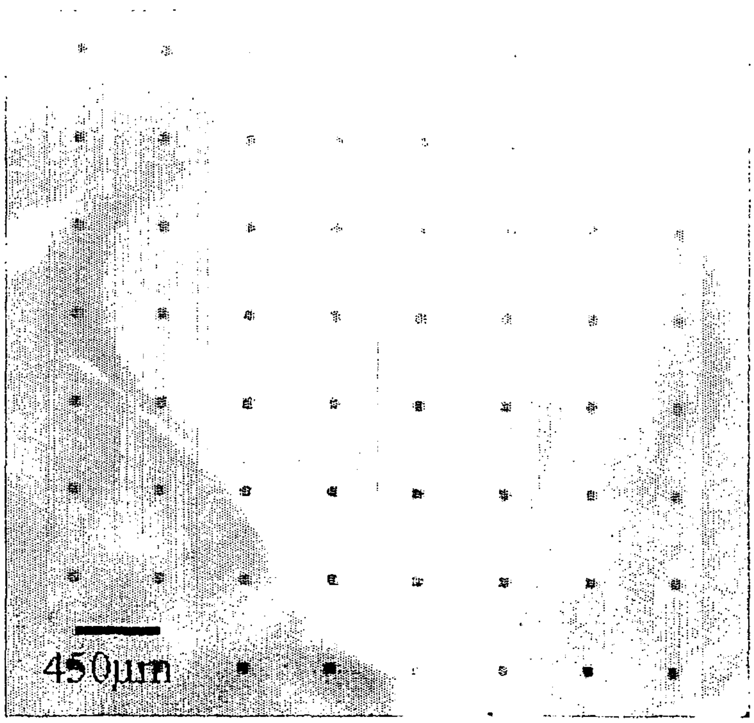


B

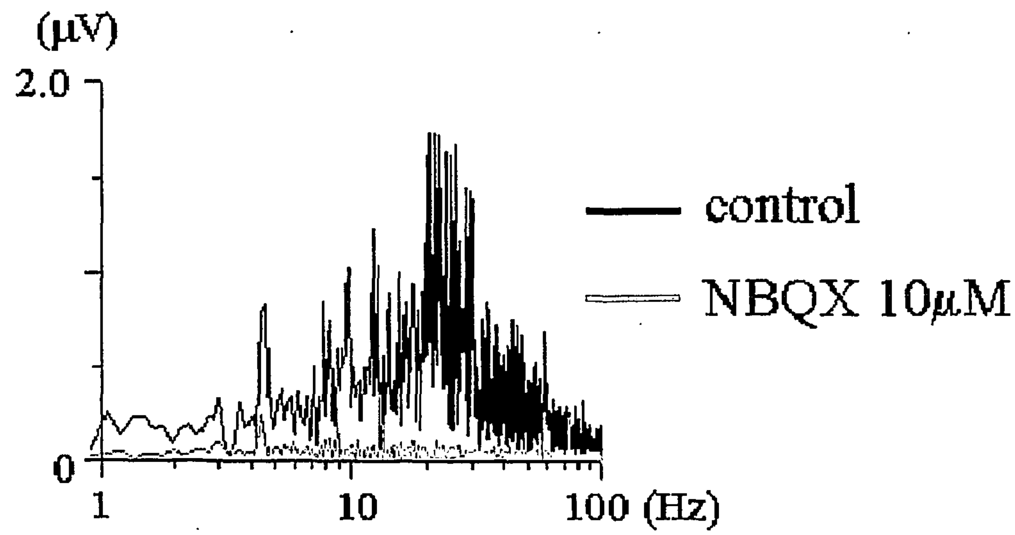

# FIG. 38 C

FIG.38B

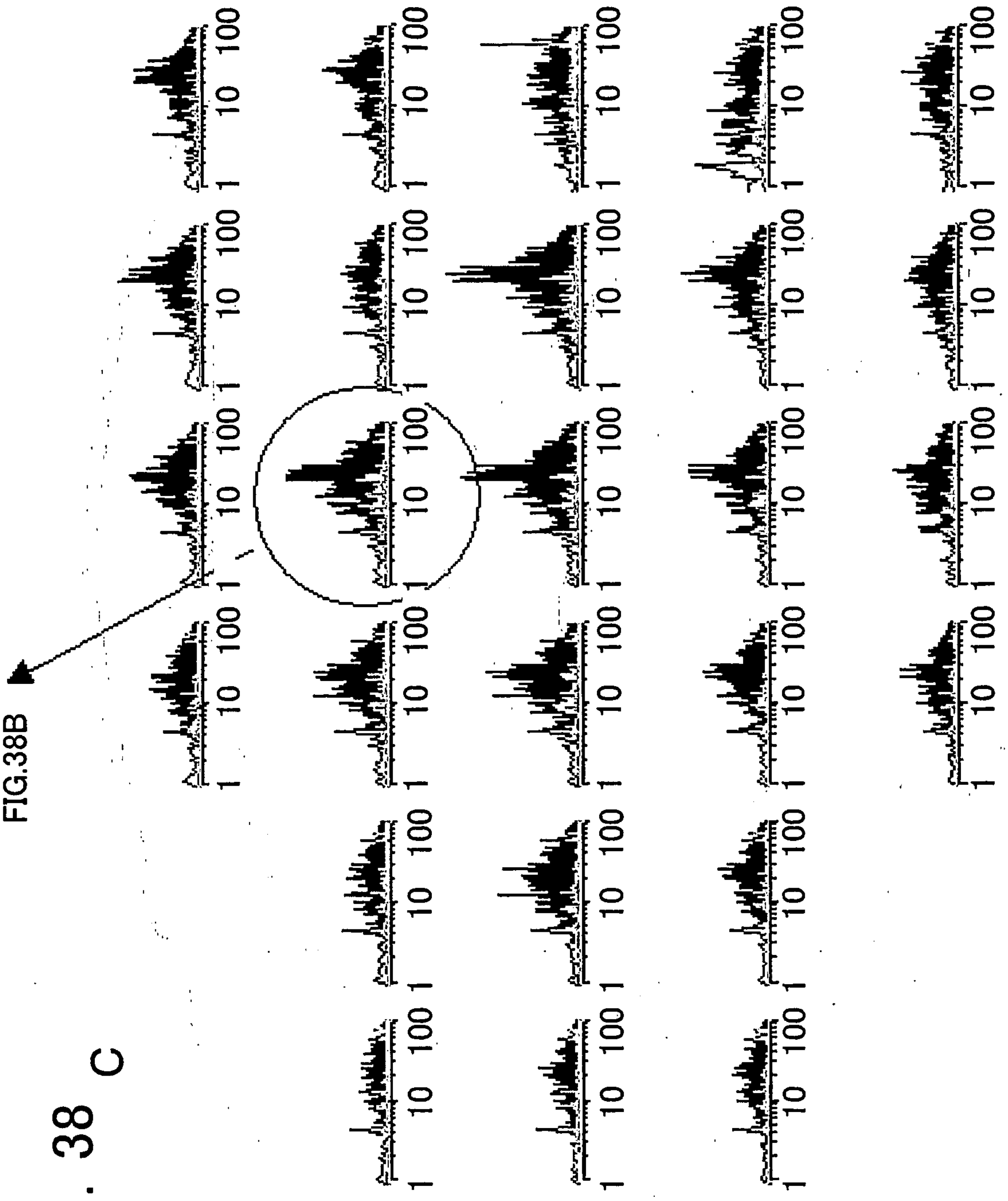

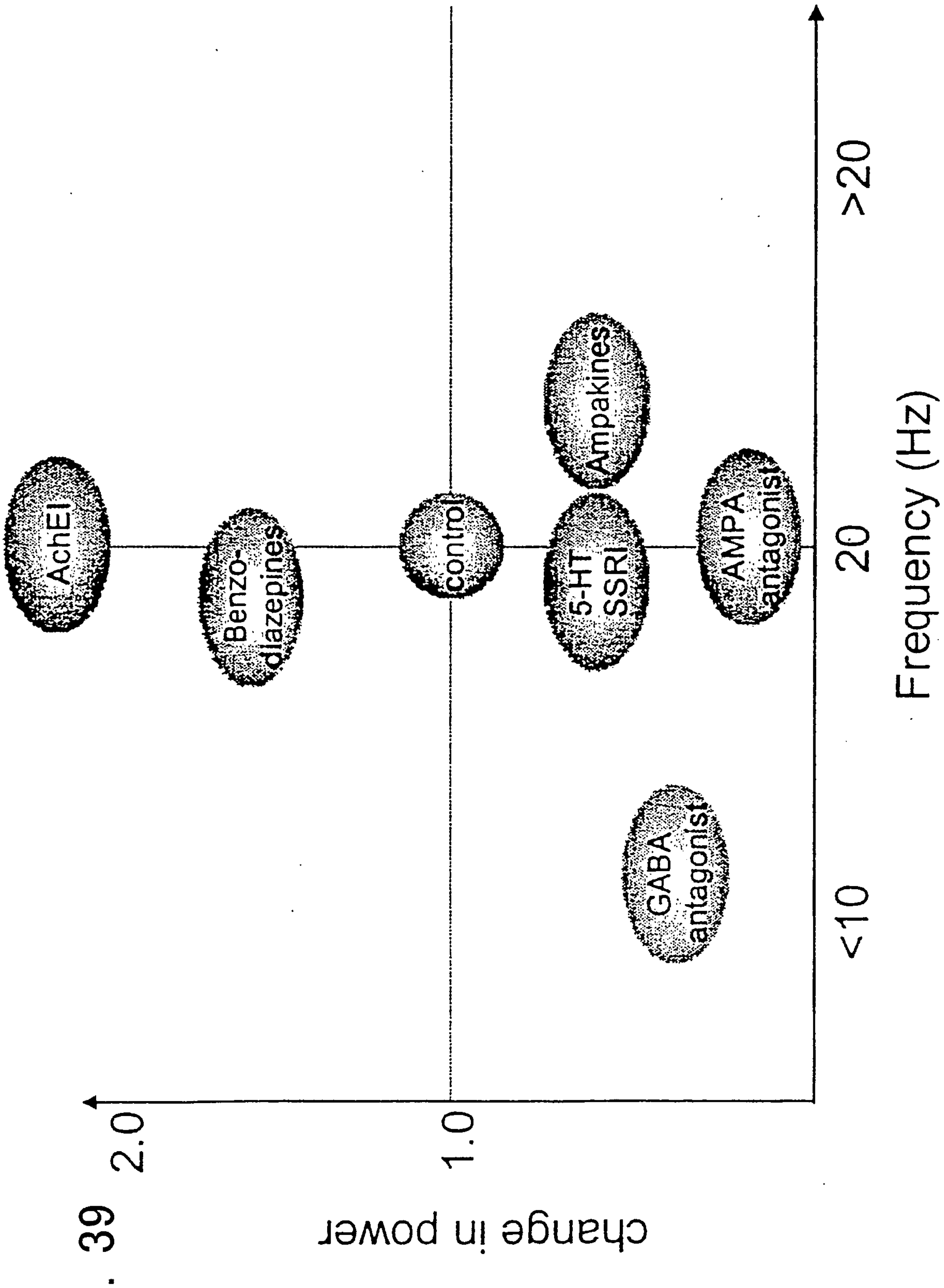
AchEI
Benzo-
diazepines
control
5-HT
SSRI
Ampakines
AMPA
antagonist
GABA
antagonist
2.0
1.0
change in power
<10
20
>20
Frequency (Hz)

FIG. 39